(19) Europäisches Patentamt / European Patent Office / Office européen des brevets

(11) EP 3 237 905 B1

(12) **FASCICULE DE BREVET EUROPEEN**

(45) Date de publication et mention de la délivrance du brevet:
**10.11.2021 Bulletin 2021/45**

(21) Numéro de dépôt: **15828359.8**

(22) Date de dépôt: **22.12.2015**

(51) Int Cl.:
***G01N 33/28*** $^{(2006.01)}$ ***G06Q 50/02*** $^{(2012.01)}$

(86) Numéro de dépôt international:
**PCT/FR2015/053710**

(87) Numéro de publication internationale:
**WO 2016/102883 (30.06.2016 Gazette 2016/26)**

(54) **PROCÉDÉ DE DÉTERMINATION DE LA COMPOSITION DE COUPES PÉTROLIÈRES ISSUES D'UN PROCÉDÉ DE RAFFINAGE POUR L'OPTIMISATION DE RAFFINERIES PÉTROLIÈRES**

VERFAHREN ZUR BESTIMMUNG DER ZUSAMMENSETZUNG VON ERDÖLFRAKTIONEN AUS EINEM RAFFINIERVERFAHREN ZUR OPTIMIERUNG VON ÖLRAFFINERIEN

METHOD FOR DETERMINING THE COMPOSITION OF PETROLEUM FRACTIONS RESULTING FROM A REFINING METHOD FOR OPTIMISING PETROLEUM REFINERIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2014 FR 1463036**

(43) Date de publication de la demande:
**01.11.2017 Bulletin 2017/44**

(73) Titulaire: **P Plus I**
**13100 Aix en Provence (FR)**

(72) Inventeur: **LENOEL, Michel**
**13100 Aix en Provence (FR)**

(74) Mandataire: **Germain Maureau**
**12, rue Boileau**
**69006 Lyon (FR)**

(56) Documents cités:

- **YONGWEN WU ET AL: "Molecular Characterization of Gasoline and Diesel Streams", INDUSTRIAL & ENGINEERING CHEMISTRY RESEARCH, vol. 49, no. 24, 15 décembre 2010 (2010-12-15), pages 12773-12782, XP055056671, ISSN: 0888-5885, DOI: 10.1021/ie101647d**
- **L. PEREIRA DE OLIVEIRA ET AL: "Development of a General Modelling Methodology for Vacuum Residue Hydroconversion", OIL & GAS SCIENCE AND TECHNOLOGY - REVUE DE L'INSTITUT FRANÇAIS DU PÉTROLE, vol. 68, no. 6, 13 novembre 2013 (2013-11-13), pages 1027-1038, XP055201616, ISSN: 1294-4475, DOI: 10.2516/ogst/2013135**
- **ALVAREZ ANTON ET AL: "On the application of petroleum feedstock modeling techniques for developing molecule-based models of hydrocarbon conversion processes", CATALYSIS TODAY, vol. 220, 13 mai 2013 (2013-05-13), pages 198-207, XP028793100, ISSN: 0920-5861, DOI: 10.1016/J.CATTOD.2013.04.009**
- **LOPEZ GARCIA C ET AL: "In-depth modeling of gas oil hydrotreating: From feedstock reconstruction to reactor stability analysis", CATALYSIS TODAY, ELSEVIER, AMSTERDAM, NL, vol. 150, no. 3-4, 30 March 2010 (2010-03-30), pages 279-299, XP026969481, ISSN: 0920-5861 [retrieved on 2009-09-19]**
- **L. Pereira De Oliveira ET AL: "Development of a General Modelling Methodology for Vacuum Residue Hydroconversion", Oil & Gas Science and Technology - Revue de l'Institut Fran?ais du P?trole, vol. 68, no. 6, 13 November 2013 (2013-11-13), pages 1027-1038, XP055201616, ISSN: 1294-4475, DOI: 10.2516/ogst/2013135**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- Yongwen Wu ET AL: "Molecular Characterization of Gasoline and Diesel Streams", Industrial & Engineering Chemistry Research, vol. 49, no. 24, 15 December 2010 (2010-12-15), pages 12773-12782, XP055056671, ISSN: 0888-5885, DOI: 10.1021/ie101647d
- D. Hudebine ET AL: "Statistical Reconstruction of Gas Oil Cuts", Oil & Gas Science and Technology - Revue de l'Institut Fran?ais du P?trole, vol. 66, no. 3, 24 November 2009 (2009-11-24), pages 461-477, XP055056805, ISSN: 1294-4475, DOI: 10.2516/ogst/2009047
- ALVAREZ ANTON ET AL: "On the application of petroleum feedstock modeling techniques for developing molecule-based models of hydrocarbon conversion processes", CATALYSIS TODAY, ELSEVIER, AMSTERDAM, NL, vol. 220, 13 May 2013 (2013-05-13), pages 198-207, XP028793100, ISSN: 0920-5861, DOI: 10.1016/J.CATTOD.2013.04.009

## Description

La présente invention concerne un procédé de détermination de propriétés de pétroles bruts et coupes pétrolières, permettant une modélisation des procédés de raffinage pour simuler le fonctionnement de raffineries pétrolières et d'usines pétrochimiques et en optimiser l'exploitation suivant des critères techniques et économiques.

Il est connu de réaliser une estimation de la composition chimique d'un pétrole brut à partir de données analytiques limitées pour des procédés de raffinage de pétrole.

Le document US 2009/0105966 décrit un procédé d'estimation des propriétés d'un pétrole brut à partir de données analytiques limitées pour des fins de modélisation et de prédiction des caractéristiques, et de rendements des procédés de raffinage de pétrole. La méthode d'estimation est constituée de deux étapes. La première étape utilise des données techniques d'analyse (telles que la spectroscopie, la viscosité et de la courbe d'ébullition) pour établir un modèle de référence de la composition d'un pétrole brut. Dans la seconde étape, ce modèle de référence est calculé au moyen d'un algorithme pour correspondre à un ensemble de données ou de propriétés analytiques supplémentaires appelés « cibles », de manière à fournir un modèle de composition du produit d'entrée. L'algorithme de la seconde étape modifie la composition de référence pour conserver la signature moléculaire, tout en atteignant les valeurs cibles.

Toutefois la pluralité des paramètres et des valeurs rendent le système complexe.

En effet, pour obtenir un niveau de précision satisfaisant, il est nécessaire de disposer d'un ensemble de données très important sur les produits d'entrée basé sur des mesures, et d'un ensemble de données très important de définition des réactions chimiques, ce qui limite l'utilisation de tel procédé.

Le document XP055056671 (Youngwen Wu and Nan Zhang, "Molecular Characterization of Gasoline and Diesel Streams", Ind. Eng. Chem. Res. 2010, 49, 12773-12782) propose en outre une méthode optimisée pour caractériser, à un niveau moléculaire, des processus de productions d'essences et de diesels, au moyen d'une matrice d'identification moléculaire par séries analogues - plus couramment désignée par l'acronyme anglo-saxon MTHS pour "molecular type homologous series". Pour améliorer la construction desdites matrices MTHS et des méthodes de transformation des propriétés générales en composition moléculaire, le document XP055056671 décrit la mise en oeuvre d'une distribution statistique et une application élargie de propriétés générales.

La présente invention a pour but de résoudre tout ou partie des inconvénients mentionnés ci-dessus.

À cet effet, la présente invention a pour objet un procédé selon la revendication 1.

Grâce aux dispositions selon l'invention, le nombre de paramètres nécessaire en entrée au modèle réactionnel est faible et provient de mesures rapides et abordables. Les performances de calcul en sont significativement améliorées par rapport à une solution connue dans laquelle le nombre de paramètres en entrée du modèle réactionnel serait considérable et proviendrait de mesures onéreuses, et / ou les paramètres eux même seraient non mesurables sur l'unité réactionnelle réelle à modéliser.

En effet, les calculs relatifs aux opérations de mélange et de fractionnement peuvent être réalisés sur la base d'une représentation précise en coupes étroites alors que les calculs relatifs aux opérations de conversions sont réalisées sur la base d'une représentation en coupe large, le lien entre les représentations entre les coupes étroites et les coupes larges étant notamment assuré par un fonction de distribution ou de répartition.

La présente invention a également pour objet un procédé de détermination de la composition d'au moins un produit pétrolier raffiné issu d'un procédé de raffinage selon la revendication 2.

Grâce aux dispositions selon l'invention, le nombre de paramètres nécessaire en entrée au modèle réactionnel est faible et provient de mesures rapides et abordables. Les performances de calcul en sont significativement améliorées par rapport à une solution connue dans laquelle le nombre de paramètres en entrée du modèle réactionnel serait considérable et proviendrait de mesures onéreuses, et / ou les paramètres eux même seraient non mesurables sur l'unité réactionnelle réelle à modéliser.

En effet, les calculs relatifs aux opérations de mélange et de fractionnement peuvent être réalisés sur la base d'une représentation en coupes étroites structurale.

Selon un mode de réalisation, la coupe étroite structurale d'entrée comprend au moins une des propriétés de la coupe étroite d'entrée parmi une propriété physique et une propriété chimique. Selon un mode de réalisation, une représentation en coupes larges d'entrée du produit pétrolier d'entrée comprend au moins deux intervalles de température de représentations en coupes étroites d'entrée. Selon un mode de réalisation, la coupe étroite structurale d'entrée comprenant au moins une des propriétés de la coupe étroite d'entrée parmi une propriété physique et une propriété chimique. Selon un mode de réalisation, un produit pétrolier d'entrée est caractérisé par au moins une coupe de produit pétrolier d'entrée qu'il contient. Selon un mode de réalisation, un produit pétrolier brut est caractérisé par au moins une coupe de produit pétrolier brut qu'il contient. Selon un mode de réalisation, la coupe d'un produit pétrolier brut est caractérisée par son pourcentage volumique. Selon un mode de réalisation, la coupe d'un produit pétrolier brut est caractérisée par son pourcentage massique. Selon un mode de réalisation, la coupe d'un produit pétrolier brut est caractérisée par son pourcentage molaire. Selon un mode de réalisation, la coupe d'un produit pétrolier d'entrée est caractérisée par sa valeur caractéristique volumique. Selon un mode de réalisation, la coupe d'un produit pétrolier

d'entrée est caractérisée par sa valeur caractéristique massique. Selon un mode de réalisation, la coupe d'un produit pétrolier d'entrée est caractérisée par sa valeur caractéristique molaire. Selon un mode de réalisation, la représentation en coupes étroites d'entrée est séparée d'au moins une composition chimique. Selon un mode de réalisation, la composition chimique comprend au moins un produit azoté. Selon un mode de réalisation, la composition chimique comprend au moins un produit soufré. Ainsi le produit à base d'azote, c'est-à-dire le produit azoté, et/ou le produit à base de soufre, c'est-à-dire le produit soufré, sont extraits de la représentations en coupes étroites d'entrée afin de leurs appliquer, plus tard, un modèle réactionnel d'hydrogénation à leurs décompositions cinétiques ou à déterminer une unité de fractionnement destinée à extraire ces produits. Cette composition détaillée d'après les éléments C, H, S, N permet de modéliser les réactions par bilan stœchiométrique rigoureux.

Selon un mode de réalisation, l'intervalle de température d'ébullition réelle est compris entre une température d'ébullition basse et une température d'ébullition haute. Selon un mode de réalisation, le produit pétrolier d'entrée comprend au moins un mélange de produit pétrolier brut. Selon un mode de réalisation, un mélange de produit pétrolier brut correspond à au moins une représentation en coupes étroites d'entrée d'un produit pétrolier d'entrée. Selon un mode de réalisation, le produit pétrolier d'entrée est caractérisé par les températures d'ébullitions réelles des produits pétroliers bruts qu'il contient. Selon un mode de réalisation, la représentation en coupes étroites d'entrée comprend un vecteur rendement. Selon un mode de réalisation, un vecteur rendement comprend au moins une coupe du produit pétrolier d'entrée. Selon un mode de réalisation, un vecteur rendement comprend au moins une coupe étroite d'entrée du produit pétrolier d'entrée. Selon un mode de réalisation, un fractionnement du vecteur rendement comprend une représentation en coupes larges d'entrée. Selon un mode de réalisation, le vecteur rendement est caractérisé par au moins une température d'ébullition réelle. Selon un mode de réalisation, le vecteur rendement du produit pétrolier d'entrée comprend au moins une somme pondérée des vecteurs rendements des produits pétroliers bruts. Selon un mode de réalisation, un vecteur rendement du produit pétrolier d'entrée comprend une valeur caractéristique volumique d'une coupe du produit pétrolier brut. A titre d'exemple, les valeurs caractéristiques volumiques peuvent être notamment : des compositions (pourcentage volumique de Naphtène), des densités ou masse volumique.

Selon un mode de réalisation, un vecteur rendement du produit pétrolier d'entrée comprend une valeur caractéristique massique d'une coupe du produit pétrolier brut. A titre d'exemple, les valeurs caractéristiques massiques peuvent être notamment : des compositions (pourcentage massique H ou S), des qualités (chaleur spécifique massique).

Selon un mode de réalisation, un vecteur rendement du produit pétrolier d'entrée comprend une valeur caractéristique molaire d'une coupe du produit pétrolier brut. A titre d'exemple, les valeurs caractéristiques molaires peuvent être notamment : des compositions (pourcentage molaires C1-C2, CN), des masses moléculaires.

Selon un mode de réalisation, un vecteur rendement du produit pétrolier d'entrée comprend un pourcentage volumique d'une coupe du produit pétrolier brut. Selon un mode de réalisation, un vecteur rendement du produit pétrolier d'entrée comprend un pourcentage massique d'une coupe du produit pétrolier brut. Selon un mode de réalisation, un vecteur rendement du produit pétrolier d'entrée comprend un pourcentage molaire d'une coupe du produit pétrolier brut. Selon un mode de réalisation, la valeur caractéristique volumique d'un vecteur rendement du produit pétrolier d'entrée est une somme de pourcentage volumique d'une coupe du produit pétrolier brut pondérée par un pourcentage volumique d'un produit pétrolier brut dans le produit pétrolier d'entrée. Selon un mode de réalisation, le pourcentage volumique d'un vecteur rendement du produit pétrolier d'entrée est une somme de pourcentage volumique d'une coupe du produit pétrolier brut pondérée par un pourcentage volumique d'un produit pétrolier brut dans le produit pétrolier d'entrée. Selon un mode de réalisation, un vecteur rendement du produit pétrolier d'entrée comprend une somme de pourcentage volumique d'une coupe du produit pétrolier brut pondérée par un pourcentage volumique d'un produit pétrolier brut dans un produit pétrolier d'entrée. Selon un mode de réalisation, le vecteur rendement du produit pétrolier d'entrée est définit par la somme suivante :

$$\%volC_{PPE}^{i} = \sum_{PPB=1}^{n} \%volC_{PPB}^{i} * \%volC_{PPE}^{PPB}$$

Avec :

- $\%volC_{PPE}^{i}$ : Pourcentage volumique d'une coupe « *i* » dans le produit pétrolier d'entrée
- $\%volC_{PPB}^{i}$ : Pourcentage volumique de la coupe « *i* » dans le produit pétrolier brut
- $\%volC_{PPE}^{PPB}$ : Pourcentage volumique du produit pétrolier brut dans le produit pétrolier d'entrée.

Ainsi, il peut être déterminé le pourcentage volumique d'une coupe « *i* », en d'autre termes une valeur caractéristique volumique dans le produit pétrolier d'entrée.

Selon un mode de réalisation, un vecteur rendement du produit pétrolier d'entrée comprend un pourcentage massique du produit pétrolier brut. Selon un mode de réalisation, un vecteur rendement du produit pétrolier d'entrée comprend un pourcentage massique d'une coupe du produit pétrolier brut. Selon un mode de réalisation, un vecteur rendement du produit pétrolier d'entrée comprend une somme de pourcentage massique du produit pétrolier brut pondérée par le pourcentage massique du produit pétrolier brut dans le produit pétrolier d'entrée. Selon un mode de réalisation, le pourcentage massique d'un vecteur rendement du produit pétrolier d'entrée est une somme de pourcentage massique d'une coupe du produit pétrolier brut pondérée par un pourcentage massique du produit pétrolier brut dans le produit pétrolier d'entrée. Selon un mode de réalisation, la valeur caractéristique massique d'un vecteur rendement du produit pétrolier d'entrée est une somme de pourcentage massique d'une coupe du produit pétrolier brut pondérée par un pourcentage massique du produit pétrolier brut dans le produit pétrolier d'entrée. Selon un mode de réalisation, un vecteur rendement du produit pétrolier d'entrée comprend une somme de pourcentage massique d'une coupe du produit pétrolier brut pondérée par un pourcentage massique du produit pétrolier brut dans le produit pétrolier d'entrée. Selon un mode de réalisation, le vecteur rendement est la somme :

$$\%wC_{PPE}^{i} = \sum_{PPB=1}^{n} \%wC_{PPB}^{i} * \%wC_{PPE}^{PPB}$$

Avec:

- $\%wC_{PPE}^{i}$ *:* Pourcentage massique d'une coupe *i* dans le produit pétrolier d'entrée ;
- $\%wC_{PPB}^{i}$ *:* Pourcentage massique de la coupe *i* dans le produit pétrolier brut ;
- $\%wC_{PPE}^{PPB}$ *:* Pourcentage massique du produit pétrolier brut dans le produit pétrolier d'entrée.

Ainsi, il peut être déterminé le pourcentage massique d'une coupe « *i* », en d'autre termes une valeur caractéristique massique dans le produit pétrolier d'entrée.

Selon un mode de réalisation, la valeur caractéristique molaire d'un vecteur rendement du produit pétrolier d'entrée est une somme de pourcentage molaire d'une coupe du produit pétrolier brut pondérée par un pourcentage molaire d'un produit pétrolier brut dans le produit pétrolier d'entrée. Selon un mode de réalisation, le pourcentage molaire d'un vecteur rendement du produit pétrolier d'entrée est une somme de pourcentage molaire d'une coupe du produit pétrolier brut pondérée par un pourcentage molaire d'un produit pétrolier brut dans le produit pétrolier d'entrée. Selon un mode de réalisation, un vecteur rendement du produit pétrolier d'entrée comprend une somme de pourcentage molaire d'une coupe du produit pétrolier brut pondérée par un pourcentage molaire d'un produit pétrolier brut dans un produit pétrolier d'entrée. Selon un mode de réalisation, le vecteur rendement du produit pétrolier d'entrée est définit par la somme suivante :

$$\%molC_{PPE}^{i} = \sum_{PPB=1}^{n} \%molC_{PPB}^{i} * \%molC_{PPE}^{PPB}$$

Avec :

- $\%molC_{PPE}^{i}$ : Pourcentage molaire d'une coupe « *i* » dans le produit pétrolier d'entrée
- $\%molC_{PPB}^{i}$ : Pourcentage molaire de la coupe « *i* » dans le produit pétrolier brut
- $\%molC_{PPE}^{PPB}$ : Pourcentage molaire du produit pétrolier brut dans le produit pétrolier d'entrée.

Ainsi, il peut être déterminé le pourcentage molaire d'une coupe « *i* », en d'autre termes une valeur caractéristique volumique dans le produit pétrolier d'entrée.

Selon un mode de réalisation, la représentation en coupes étroites comprend :

- un vecteur rendement ;
- une matrice compositionnelle-qualité ;

en fonction de leurs températures d'ébullitions réelles, de leurs pourcentages volumiques, de leurs pourcentages massiques, de leurs pourcentages molaires de leurs valeurs caractéristiques volumiques et/ou de leurs valeurs caractéristiques massiques et/ou de leurs valeurs caractéristiques molaires à partir de la représentation en coupes étroites d'entrée du produit pétrolier d'entrée. Selon un mode de réalisation, la matrice compositionnelle-qualité comprend au moins une somme de pourcentage volumique d'une coupe pondérée par une valeur caractéristique d'une coupe rapporté au pourcentage volumique de la coupe du vecteur rendement. Selon un mode de réalisation, la matrice compositionnelle-qualité comprend au moins une somme de pourcentage massique d'une coupe pondérée par une valeur caractéristique d'une coupe rapporté au pourcentage massique de la coupe du vecteur rendement. Selon un mode de réalisation, la matrice compositionnelle-qualité comprend au moins une somme de pourcentage molaire d'une coupe pondérée par une valeur caractéristique d'une coupe rapporté au pourcentage molaire de la coupe du vecteur rendement. Selon un mode de réalisation, une matrice compositionnelle-qualité comprend au moins une valeur caractéristique d'une coupe. Selon un mode de réalisation, un fractionnement du vecteur rendement comprend au moins une représentation en coupes larges de sortie. Selon un mode de réalisation, le procédé de détermination comprend une étape de constitution de la matrice compositionnelle-qualité en regroupant les représentations en coupes étroites d'entrée en représentation en coupes larges d'entrée. Selon un mode de réalisation, le procédé de détermination comprend une étape de constitution de la matrice compositionnelle-qualité en comprenant au moins une représentation en coupes larges d'entrée. Selon un mode de réalisation, le procédé de détermination comprend une étape de reconstitution de la matrice compositionnelle-qualité en déterminant la représentation en coupes larges de sortie en une représentation en coupes étroites de sortie. Selon un mode de réalisation, la représentation en coupes étroites comprend :

a) un vecteur rendement ;
b) une matrice compositionnelle-structure-qualité ;

en fonction de leurs températures d'ébullitions réelles, de leurs valeurs caractéristiques d'un élément chimique et/ou de leurs valeurs caractéristiques d'une structure chimique à partir de la représentation en coupes étroites d'entrée du produit pétrolier d'entrée. Selon un mode de réalisation, la matrice compositionnelle-structure-qualité comprend pour chaque coupe étroite d'entrée au moins une valeur caractéristique d'un élément chimique et/ou au moins une valeur caractéristique d'une structure chimique :

$$\%molC_{PPE}^{i} = \sum_{PPB=1}^{n} \%molC_{PPB}^{i} * \%molC_{PPE}^{PPB}$$

$$el\chi C_{PPE}^{i} = \frac{\sum_{PPB=1}^{n} \%molC_{PPB}^{i} * \%molC_{PPE}^{PPB} * el\chi C_{PPB}^{i}}{\sum_{PPB=1}^{n} \%molC_{PPB}^{i} * \%molC_{PPE}^{PPB}}$$

et

$$st\chi C_{PPE}^{i} = \frac{\sum_{PPB=1}^{n} \%molC_{PPB}^{i} * \%molC_{PPE}^{PPB} * st\chi C_{PPB}^{i}}{\sum_{PPB=1}^{n} \%molC_{PPB}^{i} * \%molC_{PPE}^{PPB}}$$

Avec:

- $el\chi C_{PPE}^{i}$ : Valeur caractéristique d'un élément chimique d'un produit pétrolier d'entrée PPE ;
- $el\chi C_{PPB}^{i}$ : Valeur caractéristique de l'élément chimique d'un pétrole brut PPB ;
- $st\chi C_{PPE}^{i}$ : Valeur caractéristique de la structure chimique d'un produit pétrolier d'entrée PPE ;
- $st\chi C_{PPB}^{i}$ : Valeur caractéristique de la structure chimique d'un pétrole brut PPB ;
- $\%molC_{PPE}^{i}$ : pourcentage molaire de la coupe étroite (i) d'un produit pétrolier d'entrée
- $\%molC_{PPB}^{i}$ : pourcentage molaire de la coupe étroite (i) d'un pétrole brut

- $\%molC_{PPE}^{PPB}$ : pourcentage molaire du pétrole brut PPB dans le produit pétrolier d'entrée PPE.

Ainsi, grâce à cette disposition, les valeurs caractéristiques des éléments et structures chimiques se pondèrent en moles, suivant la formule citée précédemment correspondant au mélange de plusieurs pétroles bruts PPB en produit pétrolier d'entrée PPE.

Selon un mode de réalisation, une matrice compositionnelle-structure-qualité comprend au moins une valeur caractéristique d'une coupe. Selon un mode de réalisation, un fractionnement du vecteur rendement comprend au moins une représentation en coupes étroites structurales de sortie. Selon un mode de réalisation, le procédé de détermination comprend une étape de constitution de la matrice compositionnelle-structure-qualité en regroupant les représentations en coupes étroites d'entrée en représentation en coupes structurale d'entrée. Selon un mode de réalisation, le procédé de détermination comprend une étape de constitution de la matrice compositionnelle-structure-qualité en comprenant au moins une représentation en coupes étroites structurale d'entrée. Selon un mode de réalisation, la valeur caractéristique de l'élément chimique comprend au moins un élément parmi le carbone, l'hydrogène, l'oxygène, le soufre et l'azote. Selon un mode de réalisation, l'élément chimique comprend au moins une caractérisation structurale parmi le cycle naphténique, le cycle aromatique, les chaines alkyles, le nombre de ponts entre cœur naphténique et cœur aromatique, l'indice de condensation, l'indice de branchement, et l'indice d'isomérisation. Selon un mode de réalisation, le modèle réactionnel comprend un modèle non-linéaire. Selon un mode de réalisation, le modèle réactionnel est caractérisé par des mesures. Selon un mode de réalisation, le modèle réactionnel est caractérisé par des mesures effectuées dans au moins un réacteur. Ainsi un modèle réactionnel non-linéaire permet une planification de raffineries pétrolières par programmation non-linéaire, ce qui permet d'éviter une optimisation linéaire de certains critères. En effet, l'optimisation linéaire de certains critères, qui est valide uniquement dans les hypothèses très limitatives ou le critère à optimiser est fonction linéaire des variables, et ou les contraintes prises en compte pour déterminer des solutions faisables sont elles mêmes fonctions linéaires des variables à optimiser, est très limitative par rapport à la complexité hautement non-linéaire des procédés pétroliers et du réseau récursif des produits transformés dans une raffinerie.

Selon un mode de réalisation, les mesures effectuées dans au moins un réacteur sont enregistrées et/ou archivées dans une base de données. Selon un mode de réalisation, les mesures effectuées dans au moins un réacteur sont comparées avec les mesures archivées d'une base de données. Selon un mode de réalisation, le modèle réactionnel est caractérisé par des constantes cinétiques. Selon un mode de réalisation, les constantes cinétiques du modèle réactionnel comprennent des mesures effectuées dans au moins un réacteur. Selon un mode de réalisation, les cinétiques du modèle réactionnel sont d'ordre 1, 2 ou fractionnaire par rapport aux produits réactifs. En effet, des mesures de réactions cinétiques sont effectuées dans un réacteur afin de mieux prendre en compte le déroulement exact des réactions cinétiques dans le réacteur. Ces mesures sont comparées avec des données d'une base de données, afin d'ajuster les constantes cinétiques. Ainsi une telle modélisation du modèle réactionnel permet une meilleure prise en compte des réactions chimiques qui peuvent se produire au sein d'un ou du réacteur.

Selon un mode de réalisation, le modèle réactionnel comprend un réseau cinétique configuré pour déterminer une représentation en coupes larges d'entrée en au moins une représentation en coupes larges de sortie. Selon un mode de réalisation, le modèle réactionnel comprend un réseau réactionnel configuré pour déterminer une représentation en coupe large d'entrée en au moins une représentation en coupe large de sortie ou une représentation en coupe étroite structurale d'entrée en au moins une représentation en coupe étroite structurale de sortie. Selon un mode de réalisation, le modèle réactionnel comprend un réseau réactionnel configuré pour déterminer une représentation en coupe étroite structurale d'entrée en au moins une représentation en coupe étroite structurale de sortie. Ainsi le modèle réactionnel permet à chaque paramètre de la représentation en coupes larges d'entrée une meilleure prise en compte des réactions chimiques et/ou réactions cinétiques qui peuvent se produire pour chaque représentation en coupes larges d'entrée. Selon un mode de réalisation, le réseau cinétique est configuré pour intégrer la décomposition cinétique des produits pétroliers d'entrée sur leurs temps de séjour et/ou sur leurs températures de réaction dans un réacteur ou plusieurs réacteurs en série et/ou parallèle. Selon un mode de réalisation, le réseau cinétique est configuré pour intégrer une décomposition cinétique pondérée des représentations en coupes larges d'entrée sur leurs temps de séjour et/ou sur leurs températures de réaction dans le réacteur. Selon un mode de réalisation, le modèle réactionnel est configuré pour déterminer la décomposition d'un produit correspondant à une représentation en coupes larges d'entrée en fonction de son temps de séjour et/ou de la température de réaction dans le réacteur en produits intermédiaires. Selon un mode de réalisation, le réseau réactionnel intègre la décomposition cinétique des représentations en coupes larges d'entrée sur leurs temps de séjour et sur leurs températures de réaction dans le réacteur. Selon un mode de réalisation, le réseau réactionnel intègre la décomposition cinétique des représentations en coupes étroite structurale d'entrée sur leurs temps de séjour et sur leurs températures de réaction dans le réacteur. Selon un mode de réalisation, le réseau réactionnel intègre la décomposition cinétique des représentations en coupes larges d'entrée sur leurs temps de séjour et sur leurs températures de réaction dans le réacteur ou la décomposition cinétique des représentations en coupes étroite structurale

d'entrée sur leurs temps de séjour et sur leurs températures de réaction dans le réacteur. Ainsi une telle disposition permet de déterminer la décomposition d'un produit correspondant à une représentation en coupes larges d'entrée en plusieurs produits intermédiaires dépendants du temps de séjour et/ou de la température de réaction. De plus, la décomposition est pondérée en fonction du temps de séjour et/ou de la température de réaction.

Selon un mode de réalisation, le modèle réactionnel est configuré pour déterminer la décomposition d'un produit correspondant à une représentation en coupes étroites structurales d'entrée en fonction de son temps de séjour et/ou de la température de réaction dans le réacteur en produits intermédiaires. Ainsi une telle disposition permet de déterminer la décomposition d'un produit correspondant à une représentation en coupes étroites structurales d'entrée en plusieurs produits intermédiaires dépendants du temps de séjour et/ou de la température de réaction. De plus, la décomposition est pondérée en fonction du temps de séjour et/ou de la température de réaction. Selon un mode de réalisation, la représentation en coupes étroites d'entrée est configurée pour être transformée en une représentation en coupes étroites de sortie résultant d'un équilibre thermodynamique. Selon un mode de réalisation, la représentation en coupes étroites d'entrée est configurée pour être transformée en une représentation en coupes étroites de sortie résultant d'un état transitoire, limité par la cinétique ou la thermodynamique de la réaction. Selon un mode de réalisation, un bilan élémentaire (C,H,S,N) entre les représentations en coupes étroites d'entrée et les coupes étroites de sortie est réalisé. Ainsi les bilans énergétiques et/ou les bilans de matière et/ou la stœchiométrie de la réaction cinétique et/ou des réactions cinétiques peuvent être vérifiés.

Selon un mode de réalisation, un fractionnement du vecteur rendement comprend au moins une coupe étroite de sortie. Selon un mode de réalisation, une fonction de distribution est configurée pour déterminer au moins une représentation en coupes étroites de sortie à partir d'au moins une représentation en coupes larges de sortie. Selon un mode de réalisation, la détermination d'une représentation en coupes étroites de sortie par application d'une fonction de distribution à la représentation en coupes larges de sortie est décrite par la fonction suivante :

$$p(x) = \frac{(x-\eta)^{\alpha-1} e^{-(x-\eta)/\beta}}{\beta^{\alpha}\Gamma(\alpha)}$$

Avec :

$\alpha$ = paramètre de forme
$\beta$ = paramètre d'échelle
$\eta$ = paramètre de position
$\Gamma(\alpha)$ = fonction Gamma

Selon un mode de réalisation, la fonction de distribution comprend une fonction gamma décrite par la fonction suivante :

$$\Gamma(\alpha) = \int_0^{\infty} t^{\alpha-1} e^{-t} dt$$

$\alpha$ = paramètre de forme
$t$ = paramètre temps

Selon un mode de réalisation, la détermination d'une représentation en coupes étroites de sortie comprend une étape d'application d'une fonction de distribution à au moins une représentation en coupes larges de sortie. Selon un mode de réalisation, la détermination d'une représentation en coupes étroites de sortie comprend une étape d'application d'une fonction de distribution à au moins une coupe large de sortie. Selon un mode de réalisation, au moins une représentation en coupes larges de sortie est répartie en une représentation en coupes étroites de sortie par utilisation d'une fonction de distribution. Ainsi à partir d'une représentation en coupe large de sortie, les coupes étroites de sortie peuvent être déterminées par répartition de la représentation en coupe large de sortie en plusieurs coupes étroites de sortie. Selon un mode de réalisation, la détermination d'une représentation en coupes étroites de sortie comprend une étape d'application d'une fonction de distribution à au moins une représentation en coupe large de sortie en fonction de l'intervalle de température de la représentation en coupe large de sortie. Selon un mode de réalisation, la détermination d'une représentation en coupes étroites de sortie comprend une étape d'application d'une fonction de distribution à au moins une représentation en coupe large de sortie en fonction de la composition des produits de sortie. Selon un mode de réalisation, la détermination d'une représentation en coupes étroites de sortie comprend une étape d'application d'une fonction de distribution à au moins une représentation en coupe large de sortie en fonction du

pourcentage massique des produits de sortie. Selon un mode de réalisation, les paramètres de la fonction de distribution sont ajustés par une méthode algorithmique de minimisation des écarts, par exemple par la méthode des moindre carrés, entre les rendements de coupes larges obtenus par le modèle réactionnel d'une part, et les rendements de coupes larges obtenus par la fonction de distribution. Selon un mode de réalisation, une détermination d'une représentation en coupes étroites de sortie corrigées comprend une étape d'intégration des produits azotés et/ou des produits soufrés dans au moins une coupe étroite de sortie. Selon un mode de réalisation, le modèle réactionnel comprend un modèle d'hydrogénation configuré pour intégrer la saturation des hydrocarbures oléfiniques en hydrocarbures saturés (paraffines et / ou naphtènes), et des hydrocarbures aromatiques polycycliques en aromatiques monocycliques et en naphtènes, sur le temps de séjour et/ou sur la température de réaction dans le réacteur. Selon un mode de réalisation, le modèle réactionnel comprend un modèle d'hydrogénation configuré pour intégrer la décomposition cinétique des produits azotés et/ou des produits soufrés sur le temps de séjour et/ou sur la température de réaction dans le réacteur. Selon un mode de réalisation, le modèle d'hydrogénation comprend un modèle d'hydrodésulfuration configuré pour intégrer la décomposition cinétique des produits soufrés sur le temps de séjour et/ou sur la température de réaction dans le réacteur. Selon un mode de réalisation, le modèle d'hydrogénation comprend un modèle d'hydrodéazotation configuré pour intégrer la décomposition cinétique des produits azotés sur le temps de séjour et/ou sur la température de réaction dans le réacteur. Ainsi les produits à base d'azote, c'est-à-dire les produits azotés, et les produits à base de soufre, c'est-à-dire les produits soufrés, sont extraits de la représentation en coupes larges d'entrée afin de leur appliquer un modèle d'hydrogénation, c'est-à-dire de transformer les produits à base d'azote (N) en ammoniac ($NH_3$) et/ou les produits à base de soufre en dihydrogène de soufre par exemple. De plus, les produits azotés et/ou les produits soufrés n'interagissent pas avec la représentation en coupes larges d'entrée lors de leurs décompositions cinétiques, ce qui permet de simplifier les calculs sans en altérer les résultats.

Selon un mode de réalisation, une détermination d'une représentation en coupes étroites de sortie corrigées comprend une étape d'intégration d'au moins un produit azoté et/ou d'au moins un produit soufré dans au moins une coupe étroite de sortie. Ainsi une prise en compte des produits azotés et des produits soufrés dans au moins une coupe étroite de sortie permet un résultat proche de la réalité. Selon un mode de réalisation, une représentation en coupe de produit fractionné correspond à l'application d'un point de coupe aux coupes étroites de sortie corrigées. Selon un mode de réalisation, on détermine une représentation de coupe de produit fractionné correspondant à l'application d'un point de coupe aux représentations en coupes étroites de sortie corrigées. Selon un mode de réalisation, on détermine une représentation de coupe de produit fractionné correspondant à l'application d'un point de coupe aux représentations en coupes étroites structurales de sortie. Selon un mode de réalisation, on détermine une représentation de coupe de produit fractionné correspondant à l'application d'un point de coupe aux représentations en coupes étroites de sortie corrigées ou aux représentations en coupes étroites structurales de sortie. Selon un mode de réalisation, la représentation en coupes étroites de sortie corrigées est fractionnée en au moins une coupe fractionnée en fonction d'un intervalle de température d'ébullition réelle. Selon un mode de réalisation, le procédé comprend au moins une étape de fractionnement dans lequel la représentation en coupes étroites de sortie corrigées est fractionnée en représentation en coupes fractionnées en fonction d'un intervalle de température. Selon un mode de réalisation, le procédé comprend au moins une étape de fractionnement dans lequel la représentation en coupe étroite structurale de sortie est fractionnée en représentation en coupes fractionnées en fonction d'un intervalle de température. Selon un mode de réalisation, le procédé comprend au moins une étape de fractionnement dans lequel la représentation en coupes étroites de sortie corrigées ou la représentation en coupe étroite structurale de sortie est fractionnée en représentation en coupes fractionnées en fonction d'un intervalle de température. Ainsi la coupe étroite de sortie corrigée peut être scindée ou peut être fractionné lors des étapes de fractionnement post-réacteur. Selon un mode de réalisation, on détermine la répartition des représentations en coupes étroites de sortie corrigées en représentations en coupes fractionnées par une fonction de répartition avec indice de fractionnement. Selon un mode de réalisation, on détermine la représentation en coupe étroite structurale de sortie en représentations en coupes fractionnées par une fonction de répartition avec indice de fractionnement. Selon un mode de réalisation, on détermine la répartition des représentations en coupes étroites de sortie corrigées ou la représentation en coupe étroite structurale de sortie en représentations en coupes fractionnées par une fonction de répartition avec indice de fractionnement. Selon un mode de réalisation, on détermine la répartition d'au moins une coupe étroite de sortie corrigée en au moins une coupe fractionnée par une fonction de répartition. Selon un mode de réalisation, la représentation en coupes étroites d'entrée du produit pétrolier d'entrée est obtenue à partir de données d'essai en laboratoire ou sur un réacteur par application d'une fonction de distribution. Selon un mode de réalisation, la représentation en coupes étroites de sortie corrigées est fractionnée en au moins une coupe légère, en au moins une coupe moyenne et/ou en au moins une coupe lourde par une fonction de répartition. Selon un mode de réalisation, la fonction de répartition comprend un indice de fractionnement, un point de coupe et/ou un point de fractionnement comprenant une température d'ébullition réelle. Selon un mode de réalisation, la représentation en coupes étroites de sortie corrigées est fractionnée en au moins une coupe légère, en au moins une coupe moyenne et/ou en au moins une coupe lourde par une fonction de répartition définie comme suit :

$$FR = \frac{1}{1 + e^{-FI(CP-TBP)}}$$

Avec :

*FR* = Fonction de répartition
*FI* = Indice de Fractionnement
*CP* = Point de coupe Légère/Moyenne, Moyenne /Lourde et/ou Légère/Lourde
*TBP* = Température d'ébullition réelle de la Fraction

Ainsi la représentation en coupes étroites de sortie corrigées est fractionnée en au moins une coupe par une fonction de répartition représentative de la qualité du fractionnement d'une unité de fractionnement et/ou de conversion.
Selon un mode de réalisation, les représentations en coupes étroites d'entrée sont extraites de données initiales du produit pétrolier brut par une fonction de distribution. Selon un mode de réalisation, la représentation en coupes étroites d'entrée du produit pétrolier d'entrée est obtenue à partir de donnée d'essai en laboratoire par application d'une fonction de fonction de distribution. Selon un mode de réalisation, un produit programme d'ordinateur comprenant des instructions de code agencées pour mettre en œuvre les étapes d'un Procédé de détermination de la composition d'au moins un produit pétrolier raffiné issu d'un procédé de raffinage comprenant au moins une étape de conversion d'un produit pétrolier d'entrée en un produit pétrolier de sortie dans un réacteur, lorsque ledit programme est exécuté sur un processeur d'une unité de contrôle.
L'invention sera mieux comprise à l'aide de la description détaillée qui est exposée ci-dessous en regard du dessin annexé dans lequel :

La figure 1 illustre schématiquement le procédé de raffinage.
La figure 2 illustre la représentation d'un produit d'entrée en représentation en coupes étroites d'entrée.
La figure 3 illustre la formation d'une représentation en coupes larges d'entrée à partir d'une représentation en coupes étroites d'entrée.
La figure 4 illustre une représentation en coupes larges d'entrée, un produit pétrolier intermédiaire PPI de sortie et une représentation en coupes larges de sortie dans un modèle réactionnel.
La figure 5 illustre la formation d'une représentation en coupes étroites de sortie à partir d'une représentation en coupes larges de sortie.
La figure 6 illustre le fractionnement d'une représentation en coupes étroite de sortie en coupes fractionnées.
La figure 7 schématise les différentes étapes de modélisation d'un procédé de raffinage avec des unités de conversions et d'hydrotraitements, telles que décrites dans la description détaillée qui va suivre.
La figure 8 illustre la parité représentant la masse volumique prédite en fonction de la masse volumique expérimentale.
La figure 9 illustre le graphique de parité représentant la Température d'ébullition prédite en fonction de la Température d'ébullition expérimentale
La figure 10 illustre la coupe essences dont la température d'ébullition réelle TBP est comprise entre 50-200°C.
La figure 11 illustre l'ensemble de la coupe dont la température d'ébullition réelle TBP s'étend jusqu'à 700 °C.
La figure 12 illustre un modèle cinétique.
La figure 13 illustre la décomposition d'une coupe Gasoil sous vide en fonction du temps de séjour dans le réacteur.
La figure 14 illustre une coupe Gasoil Sous Vide décomposée en différentes fractions.
La figure 15 illustre la répartition PIONA C5-C10.
La figure 16 illustre la caractéristique Densité ($D_{15/4}$) pour la coupe essence totale.
La figure 17 illustre une fonction de répartition en fonction du point de coupe et de l'indice de fractionnement.
La figure 18 illustre la répartition cumulée en coupes étroites structurale de sortie des produits en fonction de la température d'ébullition réelle TBP reconstituée de l'effluent réacteur
La figure 19 illustre l'évolution des rendements en fonction de l'avancement de la réaction.

Dans la description détaillée qui va suivre des figures définies ci-dessus, les mêmes éléments ou les éléments remplissant des fonctions identiques pourront conserver les mêmes références de manière à simplifier la compréhension de l'invention.
Comme cela est représenté sur la figure 1, une raffinerie comporte une unité de conversion comprenant en aval une étape de fractionnement, une étape réactionnel, et amont une étape de mélange .
Chaque unité de raffinerie *UR* comprend un ensemble d'unités de fractionnement $U_F$ , de conversion $U_C$ et/ou de mélange $U_B$ .

Un produit pétrolier PPB peut être composé de composés organiques, et essentiellement des hydrocarbures. Certaines des molécules d'hydrocarbures comprenant des hétéro atomes (essentiellement du soufre et/ou de l'azote, avec de l'oxygène et des métaux (nickel, vanadium et autres) dans les fractions lourdes.

Leurs compositions chimiques varient selon leurs origines : par exemple un produit pétrolier brut PPB de type ALASKA peut être différent d'un produit pétrolier brut PPB de type PAZFLOR.

La qualité du produit pétrolier brut PPB peut être déterminée à partir des teneurs en différents composés comme par exemple des chaînes carbonées linéaires et/ou ramifiés (normal paraffines ou iso paraffines), ainsi que des chaînes carbonées cycliques saturées et/ou insaturées comme les naphténiques et aromatiques.

Le produit pétrolier brut PPB ne peut pas être utilisé dans son état brut dans la plupart des cas, car il peut être composé d'hydrocarbures (composés d'atomes d'hydrogène et de carbone) dont la structure et/ou la masse moléculaire, et en conséquence les propriétés pétrolières et d'usage, diffèrent de celles recherchées et spécifiées dans les produits pétroliers finis (ainsi l'indice d'octane minimal des essences, l'indice de cétane minimal des gazoles, la viscosité des fiouls) . Afin d'obtenir un produit pétrolier raffiné PPR, un traitement approprié peut être appliqué au produit pétrolier brut PPB selon la qualité et/ou la nature des produits finis que l'on désire obtenir.

Comme il peut être observé sur la figure 1, un procédé de raffinage d'une unité de raffinerie *UR* peut permettre de traiter et de transformer différents produits pétroliers brut PPB à travers de longs et complexes procédés de raffinage pour donner des produits pétroliers raffinés PPR. La complexité des raffineries dépend des différents produits pétroliers bruts PPB mélangés en entrée pour donner un produit pétrolier d'entrée PPE, de la combinaison des unités de fractionnement par distillation, des unités de conversion et des unités de raffinage utilisées, ainsi que de la diversité des différents produits pétroliers raffinés PPR en sortie.

Les produits pétroliers brut PPB contiennent diverses composants comme par exemple des molécules d'hydrocarbures dites paraffiniques (alcanes), des naphténiques (molécules cycliques saturées), des aromatiques, des sulfures, thiols, benzothiophènes et des composants chimiques pouvant comprendre au moins un élément azote et/ou soufre.

Étant donné la complexité des données des produits pétroliers bruts PPB, le produit pétrolier d'entrée PPE peut être caractérisé par au moins une coupe d'un produit pétrolier brut PPB qu'il contient. Le terme de coupe, bien connu de l'homme du métier, signifie au moins un mélange d'hydrocarbures défini par un intervalle de température d'ébullition réelle $I_{TBP}$ ou par le nombre d'atomes de carbone de ses composants : par exemple une coupe d'essence dite lourde peut être définie par un intervalle de température d'ébullition réelle $I_{TBP}$ compris entre une température d'ébullition basse d'environ $T_{IBP}$=100°C et une température d'ébullition haute d'environ $T_{EBP}$=180°C par exemple ou par le nombre d'atomes de carbone de ses composants, c'est-à-dire, entre environ 7 et environ 10 atomes de carbone (C7-C10) par exemple.

En outre, une coupe peut aussi se définir par un étroit intervalle de température d'ébullition réelle $I_{TBP}^{E}$ ou par un nombre restreint d'atomes de carbone de ses composants : on parlera de coupe étroite. Par exemple une coupe étroite peut être définie par un étroit intervalle de température d'ébullition réelle $\mathrm{I_{TBP}^{E}}$ de 5°C, 10°C ou 20°C ou par un nombre d'atome de carbone (coupe C6), ou par un intervalle restreint de nombre d'atomes de carbone, c'est-à-dire, entre 18 et 20 atomes de carbone (C18-C20).

Une coupe peut se définir par un large intervalle de température d'ébullition réelle $I_{TBP}^{L}$ ou par un large intervalle de nombre d'atomes de carbone de ses composants : on parlera de coupe large. Par exemple une coupe large peut être définie par un large intervalle de température d'ébullition réelle $\mathrm{I_{TBP}^{L}}$ de 50°C ou par un grand nombre d'atomes de carbone, c'est-à-dire, entre 10 et 20 atomes de carbone (C10-C20). Une coupe large peut aussi correspondre à au moins deux coupes étroites en termes de température et/ou à au moins cinq coupes étroites en termes de nombre d'atomes de carbone. Par un large intervalle de température d'ébullition réelle $\mathrm{I_{TBP}^{L}}$ de 50°C peut comprendre au moins deux étroits intervalles de température d'ébullition réelle $\mathrm{I_{TBP}^{E}}$ de 20°C par exemple et un grand nombre d'atomes de carbone de C10-C30 peut comprendre au moins le nombre d'atomes d'une coupe étroite de C11-C17, C18-C23 et C24-30 par exemple.

Il est connu de l'homme du métier de répartir les coupes pétrolières en trois types de coupes :

- les coupes légères : naphta, essence légère, essence lourde
- les coupes moyennes ou distillats moyens : kérosène, diesel et fuel domestique
- et les coupes lourdes : distillats sous vides, fuel lourd ou résidu atmosphérique.

Comme il peut être observé sur la figure 1, le produit pétrolier d'entrée PPE est obtenu à partir de plusieurs produits pétroliers bruts PPB. Le produit pétrolier d'entrée PPE est admis en entrée d'une unité de traitement de fractionnement $U_F^1$ dans une première étape. Cette unité de traitement de fractionnement $U_F^1$ permet de séparer les coupes gaz des coupes essences par fractionnement par exemple. Par exemple, une coupe C1-C10, sur le graphique noté *PE,* en entrée peut être fractionnée en coupes C1/C2, C3, C4, C5-C6 (coupe essence légère) et C7-C10 (coupe essence lourde) de sortie, sur le graphique noté *PS*. Dans une deuxième étape, la coupe essence légère et la coupe essence lourde, sur le graphique noté $PE^1$ sont admises en entrée d'une unité de conversion $U_C^1$ , afin d'améliorer la qualité des coupes. Dans le cas de la coupe essence légère, la coupe essence sera isomérisée afin d'en augmenter l'indice d'octane. La coupe essence lourde subira un reformage catalytique et un mélange. En sortie d'une unité de mélange $U_B^1$ , les deux coupes d'essences $PS^3$ formeront un type de produit pétrolier raffiné PPR : carburant pour automobiles formulé en différentes variantes correspondant à des spécifications précises pour différents marchés.

Afin de prévoir les produits pétroliers raffinés PPR pouvant être obtenus, des analyses des différentes coupes des produits pétroliers bruts PPB sont mises à disposition par les fournisseurs de ces produits sous la forme de données provenant d'essais en laboratoire (« crude assay »).

Le niveau de précision des données d'essai peut être considéré comme insuffisant ou les données incomplètes pour obtenir une représentation utilisable du produit pétrolier brut qui permette de modéliser les procédés de raffinage pour simuler le fonctionnement et en optimiser l'exploitation suivant des critères techniques et économiques.

Ces données incomplètes peuvent être représentées sous la forme d'une matrice compositionnelle-qualité $M^{PPB}$ et/ou vecteur rendement $V^{PPB}$ spécifique du produit pétrolier brut PPB et caractérisée par au moins une coupe de produit pétrolier.

Ces données peuvent être ensuite normalement complétées selon les besoins et les données des matrices compositionnelle-qualité $M^{PPB}$ des produits pétroliers bruts PPB nécessaires à la raffinerie. Ceci a pour conséquence d'entraîner des coûts importants en termes d'analyses supplémentaires comme par exemple l'analyse spectroscopique infrarouge, la résonance magnétique nucléaire, la chromatographie en phase gazeuse ou liquide.

Le procède de détermination peut permettre de déterminer, à partir de données incomplètes, la composition du produit pétrolier d'entrée PPE à partir d'une fonction de distribution p(x). Cette fonction peut permettre de définir la composition globale des matrices compositionnelle-qualité $M^{PPB}$ des produits pétroliers bruts PPB.

La fonction de distribution p(x) peut permettre de compléter les données des matrices compositionnelle-qualité $M^{PPB}$ des produits pétroliers bruts PPB mises à disposition par certaines industries pétrolières et par la même occasion de déterminer une matrice compositionnelle-qualité $M^{PPE}$ du produit pétrolier d'entrée PPE. La fonction de distribution p(x) peut être définie de la façon suivante :

$$p(x) = \frac{(x-\eta)^{\alpha-1} e^{-(x-\eta)/\beta}}{\beta^{\alpha} \Gamma(\alpha)}$$

Avec :

$\alpha$ = paramètre de forme
$\beta$ = paramètre d'échelle
$\eta$ = paramètre de position
$\Gamma(\alpha)$ = fonction Gamma

La fonction gamma $\Gamma(\alpha)$ peut être décrite par la fonction suivante :

$$\Gamma(\alpha) = \int_0^{\infty} t^{\alpha-1} e^{-t} dt$$

$\alpha$ = paramètre de forme
$t$ = paramètre temps

La distribution peut être en fonction de la température d'ébullition de la coupe étroite d'entrée, ou du pourcentage massique cumulé correspondant à la position de cette coupe étroite d'entrée dans le produit pétrolier d'entrée. Les

paramètres de la fonction de distribution sont ajustés par une méthode algorithmique de minimisation des écarts, par exemple par la méthode des moindres carrés, entre les rendements de coupes larges obtenus par le modèle réactionnel d'une part, et les rendements de coupes larges obtenus par la fonction de distribution.

La matrice compositionnelle-qualité $M^{PPE}$ du produit pétrolier d'entrée PPE peut comprendre un pourcentage volumique d'une coupe « *i* » dans le produit pétrolier d'entrée $\%volC_{PPE}^{i}$ et/ou une valeur caractéristique volumique d'une coupe « *i* » dans le produit pétrolier d'entrée $volc_{PPE}^{i}$ .

Le pourcentage volumique d'une coupe « *i* » dans le produit pétrolier d'entrée $\%volC_{PPE}^{i}$ peut correspondre au pourcentage volumique d'une coupe « *i »* du produit pétrolier brut $\%volC_{PPB}^{i}$ , si le produit pétrolier d'entrée PPE et le produit pétrolier brut PPB peuvent être confondus.

Le pourcentage volumique d'une coupe « *i* » dans le produit pétrolier d'entrée $\%volC_{PPE}^{i}$ peut aussi correspondre à une somme de pourcentage volumique de la coupe d'au moins un produit pétrolier brut $\%volC_{PPB}^{i}$ pondérée par le pourcentage volumique de l'au moins un produit pétrolier brut PPB dans le produit pétrolier d'entrée $\%volC_{PPE}^{PPB}$ :

$$\%volC_{PPE}^{i} = \sum_{PPB=1}^{n} \%volC_{PPB}^{i} * \%volC_{PPE}^{PPB}$$

Avec:

- $\%volC_{PPE}^{i}$ : Pourcentage volumique d'une coupe « *i* » dans le produit pétrolier d'entrée ;
- $\%volC_{PPB}^{i}$ : Pourcentage volumique de la coupe dans le produit pétrolier brut PPB ;
- $\%volC_{PPE}^{PPB}$ : Pourcentage volumique du produit pétrolier brut PPB dans le produit pétrolier d'entrée ;

Ainsi, il peut être déterminé le pourcentage volumique d'une coupe dans le produit pétrolier d'entrée $\%volC_{PPE}^{i}$ .

Le pourcentage volumique du produit pétrolier brut $\%volC_{PPE}^{PPB}$ dans le produit pétrolier d'entrée peut être calculé à partir de la quantité volumique de produit pétrolier brut *volPPB* normé par la somme des quantités volumiques des produits pétroliers bruts formant le produit pétrolier d'entrée *volPPE:*

$$\%volC_{PPE}^{PPB_{\rho}} = \frac{volPPB_{\rho}}{volPPE}$$

$$volPPE = \sum_{\rho=1}^{n} volPPB_{\rho}$$

La valeur caractéristique volumique d'une coupe « *i* » dans le produit pétrolier d'entrée $volc_{PPE}^{i}$ peut se définir par la somme pondérée et normalisée des caractéristiques volumiques d'une coupe « *i* » dans les produits pétroliers bruts PPB :

$$volc_{PPE}^{i} = \frac{\sum_{PPB=1}^{n} volcC_{PPB}^{i} * \%volC_{PPB}^{i} * \%volC_{PPE}^{PPB}}{\sum_{PPB=1}^{n} \%volC_{PPB}^{i} * \%volC_{PPE}^{PPB}}$$

Or

$$\%volC_{PPE}^{i} = \sum_{PPB=1}^{n} \%volC_{PPB}^{i} * \%volC_{PPE}^{PPB}$$

Donc

$$volc_{PPE}^{i} = \frac{\sum_{PPB=1}^{n} volcC_{PPB}^{i} * \%volC_{PPB}^{i} * \%volC_{PPE}^{PPB}}{\%volC_{PPE}^{i}}$$

Avec:

- $volc_{PPE}^{i}$ : Valeur de la caractéristique volumique d'une coupe « *i* » dans le produit pétrolier d'entrée ;
- $volcC_{PPB}^{i}$ : Valeur de la caractéristique volumique d'une coupe « *i* » dans le produit pétrolier brut ;

La matrice compositionnelle-qualité $M^{PPE}$ du produit pétrolier d'entrée PPE peut aussi comprendre un pourcentage massique d'une coupe « *i* » dans le produit pétrolier d'entrée $\%wC_{PPE}^{i}$ et/ou une valeur caractéristique massique d'une coupe « *i* » dans le produit pétrolier d'entrée $wc_{PPE}^{i}$ .

Le pourcentage massique d'une coupe « *i* » dans le produit pétrolier d'entrée $\%wC_{PPE}^{i}$ peut correspondre au pourcentage massique d'une coupe « *i* » du produit pétrolier brut $\%wC_{PPB}^{i}$ , si le produit pétrolier d'entrée PPE et le produit pétrolier brut PPB peuvent être confondus.

Le pourcentage massique d'une coupe « *i* » dans le produit pétrolier d'entrée $\%wC_{PPE}^{i}$ peut aussi correspondre à une somme de pourcentage massique de la coupe dans au moins un produit pétrolier brut $\%wC_{PPB}^{i}$ pondérée par le pourcentage massique de l'au moins un produit pétrolier brut dans le produit pétrolier d'entrée $\%wC_{PPE}^{PPB}$ :

$$\%wC_{PPE}^{i} = \sum_{PPB=1}^{n} \%wC_{PPB}^{i} * \%wC_{PPE}^{PPB}$$

Avec:

- $\%wC_{PPE}^{i}$ : Pourcentage massique d'une coupe « *i* » dans le produit pétrolier d'entrée ;
- $\%wC_{PPB}^{i}$ : Pourcentage massique de la coupe *i* dans le produit pétrolier brut ;
- $\%wC_{PPE}^{PPB}$ : Pourcentage massique du produit pétrolier brut dans le produit pétrolier d'entrée ;

Ainsi, il peut être déterminé le pourcentage massique d'une coupe « *i* » dans le produit pétrolier d'entrée $\%wC_{PPE}^{i}$ .

Le pourcentage massique du produit pétrolier brut dans le produit pétrolier d'entrée $\%wC_{PPE}^{PPB}$ peut être calculé à partir de la quantité massique du produit pétrolier brut *wPPB* normée par la somme des quantités de produits pétroliers bruts formant le produit pétrolier d'entrée :

$$\%wC_{PPE}^{PPB_\rho} = \frac{wPPB_\rho}{wPPE}$$

avec

$$wPPE = \sum_{\rho=1}^{n} wPPB_\rho$$

La valeur caractéristique massique d'une coupe « *i* » dans le produit pétrolier d'entrée $wc_{PPE}^{i}$ peut se définir par la somme pondérée et normalisée des caractéristiques massiques d'une coupe « *i* » dans les produits pétroliers bruts :

$$wc_{PPE}^{i} = \frac{\sum_{PPB=1}^{n} wcC_{PPB}^{i} * \%wC_{PPB}^{i} * \%wC_{PPE}^{PPB}}{\sum_{PPB=1}^{n} \%wC_{PPB}^{i} * \%wC_{PPE}^{PPB}}$$

or

$$\%wC_{PPE}^{i} = \sum_{PPB=1}^{n} \%wC_{PPB}^{i} * \%wC_{PPE}^{PPB}$$

donc

$$wc_{PPE}^{i} = \frac{\sum_{PPB=1}^{n} wcC_{PPB}^{i} * \%wC_{PPB}^{i} * \%wC_{PPE}^{PPB}}{\%wC_{PPE}^{i}}$$

avec :

- $wc_{PPE}^{i}$ : Valeur de la caractéristique massique d'une coupe « *i* » dans le produit pétrolier d'entrée ;
- $wcC_{PPB}^{i}$ : Valeur de la caractéristique massique d'une coupe « *i* » dans le produit pétrolier brut ;

La matrice compositionnelle-qualité $M^{PPE}$ du produit pétrolier d'entrée PPE peut comprendre un pourcentage molaire d'une coupe « *i* » dans le produit pétrolier d'entrée $\%molC_{PPE}^{i}$ et/ou une valeur caractéristique molaire d'une coupe « *i* » dans le produit pétrolier d'entrée $molc_{PPE}^{i}$ .

Le pourcentage molaire d'une coupe « *i* » dans le produit pétrolier d'entrée $\%molC_{PPE}^{i}$ peut correspondre au pourcentage molaire d'une coupe « *i* » du produit pétrolier brut $\%molC_{PPB}^{i}$ , si le produit pétrolier d'entrée PPE et le produit pétrolier brut PPB peuvent être confondus.

Le pourcentage molaire d'une coupe « *i* » dans le produit pétrolier d'entrée $\%volC_{PPE}^{i}$ peut aussi correspondre à une somme de pourcentage molaire de la coupe d'au moins un produit pétrolier brut $\%molC_{PPB}^{i}$ pondérée par le pourcentage molaire d'au moins un produit pétrolier brut PPB dans le produit pétrolier d'entrée $\%molC_{PPE}^{PPB}$ :

$$\%molC_{PPE}^{i} = \sum_{PPB=1}^{n} \%molC_{PPB}^{i} * \%molC_{PPE}^{PPB}$$

Avec :

- $\%molC_{PPE}^{i}$ : Pourcentage molaire d'une coupe « *i* » dans le produit pétrolier d'entrée ;
- $\%molC_{PPB}^{i}$ : Pourcentage molaire de la coupe dans le produit pétrolier brut PPB ;
- $\%molC_{PPE}^{PPB}$ : Pourcentage molaire du produit pétrolier brut PPB dans le produit pétrolier d'entrée ;

Ainsi, il peut être déterminé le pourcentage molaire d'une coupe dans le produit pétrolier d'entrée $\%molC_{PPE}^{i}$.

Le pourcentage molaire du produit pétrolier brut $\%molC_{PPE}^{PPB}$ dans le produit pétrolier d'entrée peut être calculé à partir de la quantité molaire de produit pétrolier brut *molPPB* normé par la somme des quantités molaires des produits pétroliers bruts formant le produit pétrolier d'entrée *molPPE*:

$$\%molC_{PPE}^{PPB_{\rho}} = \frac{molPPB_{\rho}}{molPPE}$$

$$molPPE = \sum_{\rho=1}^{n} molPPB_{\rho}$$

La valeur caractéristique molaire d'une coupe « *i* » dans le produit pétrolier d'entrée $molc_{PPE}^{i}$ peut se définir par la somme pondérée et normalisée des caractéristiques molaires d'une coupe « *i* » dans les produits pétroliers bruts PPB :

$$mol_{PPE}^{i} = \frac{\sum_{PPB=1}^{n} molC_{PPB}^{i} * \%molC_{PPB}^{i} * \%molC_{PPE}^{PPB}}{\sum_{PPB=1}^{n} \%molC_{PPB}^{i} * \%molC_{PPE}^{PPB}}$$

Or

$$\%molC_{PPE}^{i} = \sum_{PPB=1}^{n} \%molC_{PPB}^{i} * \%molC_{PPE}^{PPB}$$

Donc

$$molc_{PPE}^{i} = \frac{\sum_{PPB=1}^{n} molC_{PPB}^{i} * \%molC_{PPB}^{i} * \%molC_{PPE}^{PPB}}{\%molC_{PPE}^{i}}$$

Avec:

- $mol_{PPE}^{i}$ : Valeur de la caractéristique molaire d'une coupe « *i* » dans le produit pétrolier d'entrée ;
- $molC_{PPB}^{i}$ : Valeur de la caractéristique molaire d'une coupe « *i* » dans le produit pétrolier brut ;

Ces coupes peuvent être organisées sous forme de vecteur rendement $V^{PPE}$ du produit pétrolier d'entrée PPE et peuvent comprendre un pourcentage volumique d'une coupe « *i* » dans le produit pétrolier d'entrée $\%volC_{PPE}^{i}$. Dans ce cas le pourcentage volumique d'une coupe « *i* » dans le produit pétrolier d'entrée $\%volC_{PPE}^{i}$ correspond au pourcentage volumique d'une coupe « *i* » du produit pétrolier brut $\%volC_{PPB}^{i}$, car le produit pétrolier d'entrée et le produit pétrolier brut peuvent être confondus.

Le pourcentage volumique d'une coupe « *i* » dans le produit pétrolier d'entrée $\%volC_{PPE}^{i}$ peut aussi correspondre à une somme de pourcentage volumique de la coupe « *i* » dans le produit pétrolier brut pondérée par le pourcentage volumique du produit pétrolier brut dans le produit pétrolier d'entrée $\%volC_{PPE}^{PPB}$ :

$$\%volC_{PPE}^{i} = \sum_{PPB=1}^{n} \%volC_{PPB}^{i} * \%volC_{PPE}^{PPB}$$

Avec :

- $\%volC_{PPE}^{i}$ : Pourcentage volumique d'une coupe « *i* » dans le produit pétrolier d'entrée ;
- $\%volC_{PPB}^{i}$ : Pourcentage volumique de la coupe *i* dans le produit pétrolier brut ;
- $\%volC_{PPE}^{PPB}$ : Pourcentage volumique du produit pétrolier brut dans le produit pétrolier d'entrée ;

Le pourcentage volumique du produit pétrolier brut dans le produit pétrolier d'entrée $\%volC_{PPE}^{PPB}$ étant calculé à partir de la quantité volumique de produit pétrolier brut *volPPB* normée par la somme des quantités de produits pétroliers bruts formant le produit pétrolier d'entrée :

$$\%volC_{PPE}^{PPB_\rho} = \frac{volPPB_\rho}{volPPE}$$

$$volPPE = \sum_{\rho=1}^{n} volPPB_\rho$$

Ainsi, il peut être déterminé le pourcentage volumique d'une coupe *i* dans le produit pétrolier d'entrée $\%volC_{PPE}^{i}$.

Par exemple, dans le cas d'un produit pétrolier d'entrée PPE issu du mélange d'un produit pétrolier brut de type ALASKA et d'un produit pétrolier brut de type PAZFLOR, une coupe d'essence $\%volC_{PPE}^{Ess}$ se définira de la façon suivante : $\%volC_{PPE}^{Ess} = \%volC_{\mathrm{ALASKA}}^{Ess} * \%volC_{PPE}^{\mathrm{ALASKA}} + \%volC_{\mathrm{PAZFLOR}}^{Ess} * \%volC_{PPE}^{\mathrm{PAZFLOR}}$ Avec :

- $\%volC_{PPE}^{Ess}$ : Pourcentage volumique d'essence lourde dans le produit pétrolier d'entrée ;
- $\%volC_{\mathrm{ALASKA}}^{Ess}$ : Pourcentage volumique d'essence lourde dans le produit pétrolier brut de type ALASKA ;
- $\%volC_{\mathrm{PAZFLOR}}^{Ess}$ : Pourcentage volumique d'essence lourde dans le produit pétrolier brut de type PAZFLOR ;
- $\%volC_{PPE}^{\mathrm{ALASKA}}$ : Pourcentage volumique du produit pétrolier brut de type ALASKA dans le produit pétrolier d'entrée ;
- $\%volC_{PPE}^{\mathrm{PAZFLOR}}$ : Pourcentage volumique du produit pétrolier brut de type PAZFLOR dans le produit pétrolier

d'entrée.

La matrice compositionnelle-qualité $M^{PPE}$ spécifique d'un produit pétrolier d'entrée PPE peut, dès lors, se présenter sous la forme représentée au tableau 1.

Tableau 1

| | $T_{IBP}$=220°C $T_{EBP}$=230°C | $T_{IBP}$=230°C $T_{EBP}$=240°C | $T_{IBP}$=240°C $T_{EBP}$=250°C | $T_{IBP}$=250°C $T_{EBP}$=260°C | $T_{IBP}$=260°C $T_{EBP}$=270°C |
|---|---|---|---|---|---|
| HGO | 3,0% | 2,5% | 1,4% | 0,2% | 0% |
| VGO1 | 2,4% | 1,9% | 1,6% | 0,4% | 0% |
| VGO2 | 0,2% | 1,2% | 0,9% | 0,7% | 0,5% |
| VGO3 | 0,1% | 0,5% | 0,6% | 0,4% | 0,2% |
| VGO4 | 0,6% | 1,1% | 1,8% | 2,9% | 2,6% |
| VGO5 | 0% | 0,1% | 0,5% | 1,2% | 1,6% |

Avec :

- $T_{IBP}$: température d'ébullition basse ;
- $T_{EBP}$: température d'ébullition haute ;
- HGO : Gasoil lourd ;
- VGO1: Gasoil sous vide 1 ;
- VGO2 : Gasoil sous vide 2 ;
- VGO3 : Gasoil sous vide 3 ;
- VGO4 : Gasoil sous vide 4 ;
- VGO5 : Gasoil sous vide 5 ;

Le vecteur rendement du produit pétrolier d'entrée $V^{PPE}$ peut aussi comprendre un pourcentage massique d'une coupe « *i* » dans le produit pétrolier d'entrée $\%wC_{PPE}^{i}$ . Dans ce cas le pourcentage massique d'une coupe « *i* » dans le produit pétrolier d'entrée $\%wC_{PPE}^{i}$ correspond au pourcentage massique d'une coupe « *i* » du produit pétrolier brut $\%wC_{PPB}^{i}$ , car le produit pétrolier d'entrée PPE et le produit pétrolier brut PPB sont confondus. Le pourcentage massique d'une coupe « *i* » dans le produit pétrolier d'entrée $\%wC_{PPE}^{i}$ peut aussi correspondre à une somme de pourcentage massique de la coupe « *i* » dans produit pétrolier brut $\%wC_{PPB}^{i}$ pondérée par le pourcentage massique du produit pétrolier brut dans le produit pétrolier d'entrée $\%wC_{PPE}^{PPB}$ :

$$\%wC_{PPE}^{i} = \sum_{PPB=1}^{n} \%wC_{PPB}^{i} * \%wC_{PPE}^{PPB}$$

Avec:

- $\%wC_{PPE}^{i}$ : Pourcentage massique d'une coupe « *i* » dans le produit pétrolier d'entrée
- $\%wC_{PPB}^{i}$ : Pourcentage massique de la coupe *i* dans le produit pétrolier brut
- $\%wC_{PPE}^{PPB}$ : Pourcentage massique du produit pétrolier brut dans le produit pétrolier d'entrée.

Ainsi, il peut être déterminé le pourcentage massique d'une coupe *i* dans le produit pétrolier d'entrée $\%wC_{PPE}^{i}$ .

Le pourcentage massique du produit pétrolier brut dans le produit pétrolier d'entrée $\%wC_{PPE}^{PPB}$ étant calculé à partir de la quantité massique de produit pétrolier brut *wPPB* normé par la somme des quantités de produits pétroliers bruts formant le produit pétrolier d'entrée :

$$\%wC_{PPE}^{PPB_\rho} = \frac{wPPB_\rho}{wPPE}$$

$$wPPE = \sum_{\rho=1}^{n} wPPB_\rho$$

Ainsi, il peut être déterminé le pourcentage massique d'un produit pétrolier brut dans le produit pétrolier d'entrée $\%wC_{PPE}^{PPB}$.

Par exemple, dans le cas d'un produit pétrolier d'entrée issu du mélange d'un produit pétrolier brut de type ALASKA et d'un produit pétrolier brut de type PAZFLOR, une coupe d'essence $\%wC_{PPE}^{Ess}$ se définira de la façon suivante : $\%wC_{PPE}^{Ess} = \%wC_{\text{ALASKA}}^{Ess} * \%wC_{PPE}^{\text{ALASKA}} + \%wC_{\text{PAZFLOR}}^{Ess} * \%wC_{PPE}^{\text{PAZFLOR}}$ Avec :

- $\%wC_{PPE}^{Ess}$ : Pourcentage massique d'essence lourde dans le produit pétrolier d'entrée ;
- $\%wC_{\text{ALASKA}}^{Ess}$ : Pourcentage massique d'essence lourde dans le produit pétrolier brut de type ALASKA ;
- $\%wC_{\text{PAZFLOR}}^{Ess}$ : Pourcentage massique d'essence lourde dans le produit pétrolier brut de type PAZFLOR ;
- $\%wC_{PPE}^{\text{ALASKA}}$ : Pourcentage massique du produit pétrolier brut de type ALASKA dans le produit pétrolier d'entrée ;
- $\%wC_{PPE}^{\text{PAZFLOR}}$ : Pourcentage massique du produit pétrolier brut de type PAZFLOR dans le produit pétrolier d'entrée.

Dans la suite de la description, le vecteur rendement *V* et la matrice compositionnelle-qualité M seront représentés sous forme de représentation en coupes étroites d'entrée $RCE_E$.

Comme illustre la figure 2, chaque représentation en coupes étroites d'entrée $RCE_E$ correspondant à au moins un pourcentage volumique $\%volC_{PPE}^{RCE_E}$ et/ou massique $\%wC_{PPE}^{RCE_E}$ et/ou molaire $\%moleC_{PPE}^{RCE_E}$ du produit pétrolier d'entrée dont la température d'ébullition réelle TBP peut être comprise dans un étroit intervalle de température d'ébullition $I_{TBP}^{E}$.

Lors de la formation des représentations en coupes étroites d'entrée $RCE_E$, certaines compositions chimiques, comme par exemple des produits à base d'azote, c'est-à-dire les produits azotés $P_N$, et/ou des produits à base de soufre, c'est-à-dire les produits soufrés $P_S$, peuvent être extraits des représentations en coupes étroites d'entrée$RCE_E$.

À partir de plusieurs représentations en coupes étroites d'entrée $RCE_E$, au moins une représentation en coupes larges d'entrée $RCL_E$ du produit pétrolier d'entrée peut être déterminée.

Comme il peut l'être observé sur la figure 3, chaque coupe large d'entrée $RCL_E$ correspondant à une somme de pourcentage volumique et/ou massique et/ou molaire du produit pétrolier d'entrée dont la température d'ébullition réelle TBP comprend au moins un étroit intervalle de température d'ébullition réelle $I_{TBP}^{E}$ de coupe étroite d'entrée $RCE_E$ compris dans un large intervalle de température d'ébullition réelle $I_{TBP}^{L}$ d'une coupe large d'entrée $RCL_E$.

À la représentation en coupe large d'entrée $RCL_E$ peut être appliqué un modèle réactionnel *R*. L'application du modèle réactionnel *R* à au moins une représentation en coupes larges d'entrée $RCL_E$ peut permettre de déterminer au moins une représentation en coupes larges de sortie $RCL_S$ d'un produit pétrolier intermédiaire PPI de sortie comme l'illustre la figure 4.

Le modèle réactionnel *R* peut être une représentation des différentes réactions que peut subir une représentation en coupes larges d'entrée $RCL_E$. Par exemple le Craquage Catalytique en lit Fluidisé FCC, l'Hydrocraquage $C_H$, le

vapocraquage Cv, la cokéfaction $C_K$, l'Hydrogénation H, le craquage thermique $C_{Th}$ et autre.

Le modèle réactionnel *R* peut être appliqué à la représentation en coupes larges d'entrée $RCL_E$ et peut simuler les réactions avec des constantes cinétiques $k_v$ qui peuvent avoir lieu au sein d'un réacteur quel qu'en soit le mode de mise en œuvre (réacteur homogène, réacteur avec catalyseur en lit fixe, lit mobile, lit fluide, catalyseur dispersé dit « slurry »).

Ces réactions peuvent être la plupart du temps non-linéaires. Ces réactions peuvent être modélisées par un réseau cinétique réactionnel entre coupes larges, avec des cinétiques de réaction d'ordre 1, 2 ou fractionnaire.

Le modèle réactionnel *R* est caractérisé par des mesures effectuées $\mu_{eff}$ dans au moins un réacteur. Ces mesures effectuées $\mu_{eff}$ dans au moins un réacteur sont enregistrées et archivées dans une base de données *DB* et peuvent être ensuite comparées avec les mesures archivées $\mu_{arch}$ de la base de données *DB.* De plus, la validité du modèle réactionnel *R* n'est pas limité au réacteur ou aux conditions opératoires utilisés pour mesurer ou estimer les constantes cinétiques, ce qui rend possible une interpolation et extrapolation à d'autres réacteurs ayant le même modèle réactionnel, avec des plages de fonctionnement plus larges que celles correspondant à l'historique sur lequel est basé l'analyse statistique.

Le modèle réactionnel *R* est caractérisé par des constantes cinétiques *k*. Les constantes cinétiques *k* du modèle réactionnel *R* comprennent les mesures effectuées $\mu_{eff}$ dans au moins un réacteur. Ainsi un tel modèle réactionnel *R*, comprennant des mesures effectuées $\mu_{eff}$ dans au moins un réacteur, permet une planification non-linéaire de raffineries pétrolières, ce qui permet d'éviter une optimisation linéaire de certains critères. En effet, l'optimisation linéaire de certains critères (qui est valide uniquement dans les hypothèses très limitatives ou le critère à optimiser est fonction linéaire des variables, et ou les contraintes prises en compte pour déterminer des solutions faisables sont elles mêmes fonctions linéaires des variables à optimiser) est très limitative et grossièrement simplificatrice par rapport à la complexité hautement non-linéaire des procédés pétroliers et du réseau récursif des produits transformés dans une raffinerie.

Pour estimer les constantes cinétiques et paramètres du modèle réactionnel, des mesures de réactions cinétiques sont effectuées dans un réacteur afin de mieux prendre en compte le déroulement exact des réactions cinétiques dans un réacteur.

Ces mesures sont comparées avec des données d'une base de données *DB,* afin d'ajuster les constantes cinétiques *k* et permettre de représenter au mieux les réactions chimiques qui peuvent se produire dans le réacteur à modéliser.

C'est pourquoi un modèle réactionnel *R* peut être configuré pour déterminer au moins une représentation en coupe large intermédiaire de sortie $RCL_S$ à partir de l'au moins une représentation en coupe large d'entrée $RCL_E$. Le modèle réactionnel *R* peut être un ensemble interconnecté de réactions cinétiques pouvant permettre de modéliser la réaction de transformation d'une représentation en coupe large d'entrée $RCL_E$ en au moins une représentation en coupe large intermédiaire $RCL_I$ ou en au moins une représentation en coupe large de sortie $RCL_S$ . Par exemple, lors du séjour $t_s$ d'une représentation en coupe large d'entrée $RCL_E$ dans un réacteur, la représentation en coupes larges d'entrée $RCL_E$ peut se décomposer et peut produire des produits intermédiaires PPI issus du réseau cinétique $R_k$ , avant de résulter en représentation en coupe large de sortie $RCL_S$ .

Un réseau cinétique $R_k$ peut comprendre au moins une réaction cinétique. Un réseau cinétique $R_k$ peut intégrer au moins une décomposition cinétique d'un produit pétrolier d'entrée PPE, représenté en coupe large d'entrée $RCL_E$, sur son temps de séjour $t_s^{RCL_E}$ et sur sa température de réaction $T°_{RCL_E}$ dans le réacteur. Le réseau cinétique $R_k$ peut être aussi configuré pour intégrer une décomposition cinétique pondérée des représentations en coupes larges d'entrée $RCL_E$ sur leurs temps de séjour $t_s^{RCL_E}$ et sur leurs températures de réaction $T°_{RCL_E}$ dans le réacteur.

La détermination d'une représentation en coupes étroites de sortie $RCE_S$ comprend une étape d'application d'une fonction de distribution *p*(*x*) à la représentation en coupes larges de sortie $RCL_S$ .

La figure 5 illustre cette étape de procédé.

Après avoir obtenu une représentation en coupes larges de sortie du produit pétrolier de sortie $RCL_S$, une fonction de distribution *p*(*x*) peut être appliquée à la représentation en coupe large de sortie du produit pétrolier de sortie $RCL_S$, afin d'en déterminer d'une représentation en coupes étroites de sortie d'un produit pétrolier de sortie $RCL_S$ .

Cette fonction de distribution *p*(*x*) peut être définie par la fonction suivante :

$$p(x) = \frac{(x-\eta)^{\alpha-1} e^{-(x-\eta)/\beta}}{\beta^{\alpha}\Gamma(\alpha)}$$

Avec :

$\alpha$ = paramètre de forme
$\beta$ = paramètre d'échelle
$\eta$ = paramètre de position
$\Gamma(\alpha)$ = fonction Gamma

La fonction gamma $\Gamma(\alpha)$ peut être décrite par la fonction suivante :

$$\Gamma(\alpha) = \int_0^{\infty} t^{\alpha-1} e^{-t} dt$$

$\alpha$ = paramètre de forme
$t$ = paramètre temps

Afin de s'assurer du bon déroulement de l'application de la fonction de distribution $p(x)$, un bilan de matière $B_M$ et/ou un bilan élémentaire $B_{ELEM}$ peut être appliqué entre la représentation en coupes étroites d'entrée $RCE_E$ et la représentation en coupes étroites de sortie $RCE_S$ . Cette vérification peut permettre d'établir que le bilan stœchiométrique $B_{ST}$ (C, H, S, N, métaux) est conservé.

De plus, l'établissement d'un bilan de matière $B_M$ et/ou un bilan élémentaire $B_{ELEM}$ après le modèle réactionnel $R$ peut permettre de vérifier que le bilan stœchiométrique $B_{ST}$ a bien été respecté.

Après obtention d'au moins une représentation en coupe étroite de sortie $RCE_S$ , le procédé peut comprendre une étape de reconstitution du vecteur rendement $V$ et/ou de la matrice compositionnelle-qualité $M$.

Lors de la formation des représentations en coupes étroites d'entrée $RCE_S$ , les produits à base d'azote, c'est-à-dire les produits azotés$P_N$ , et les produits à base de soufre, c'est-à-dire les produits soufrés $P_S$ , peuvent être extraits des représentations en coupes étroites d'entrée afin de leur appliquer un modèle réactionnel propre à leurs décompositions cinétiques. De plus, les produits azotés $P_N$ et des produits soufrés $P_S$ n'interagissent pas avec les représentations en coupes larges d'entrée $RCL_E$ lors de leurs décompositions cinétiques, ce qui peut permettre de simplifier les calculs sans en altérer les résultats.

Les produits azotés $P_N$ et des produits soufrés $P_S$ peuvent posséder un modèle réactionnel $R_{k_{SN}}$. Le modèle réactionnel $R$ peut comprendre au moins un modèle d'Hydrogénation$R_H$ . Un modèle d'Hydrogénation $R_H$ peut comprendre un modèle d'hydrodésulfuration $R_{HS}$ configuré pour intégrer la réaction cinétique des produits soufrés $P_S$ sur le temps de séjour $t_s$ et sur la température $T°$ de réaction dans le réacteur et/ou au moins une unité d'épuration en ajoutant aux produits soufrés $P_S$ de l'hydrogène.

Un modèle d'Hydrogénation $R_H$ peut aussi comprendre un modèle d'hydrodéazotation $R_{NH}$ configuré pour intégrer la réaction cinétique des produits azotés $P_N$ sur le temps de séjour $t_s$ et sur la température $T°$ de réaction dans le réacteur et/ou au moins une unité d'épuration en ajoutant aux produits azotés $P_N$ de l'hydrogène.

Par exemple les produits azotés $P_N$ et/ou les produits soufrés $P_S$ peuvent donner par hydrogénation de l'ammoniac $NH_3$ et/ou du sulfure d'hydrogène $H_2S$.

Un hydrotraitement des représentations en coupes étroites d'entrée peut être appliqué. L'hydrotraitement des représentations en coupes étroites d'entrée $RCE_E$ peut permettre de séparer les produits azotés $P_N$ et des produits soufrés $P_S$. Le modèle d'Hydrogénation $R_H$ peut comprendre un modèle de d'hydrodésulfuration et/ou d'hydrodéazotation .

Une hydrodésulfuration peut aussi permettre de séparer les produits azotés $P_N$ et/ou des produits soufrés $P_S$ des représentations en coupes étroites de sortie $RCE_S$. Le modèle d'Hydrogénation $R_H$ peut comprendre une hydrodésulfuration. Par exemple: une coupe gazole et/ou une coupe de fioul domestique peut être séparée ou peuvent être séparées par hydrodésulfuration $R_H$ des produit azotés $P_N$ et/ou des produits soufrés $P_S$ . Le modèle réactionnel peut correspondre à une hydrodésulfuration $R_H$ d'une représentation en coupe étroite d'entrée dans un réacteur en présence d'hydrogène, sur un lit catalytique entre environ 320°C et environ 390°C et sous une pression d'environ 18 et d'environ 51 bars par exemple. L'hydrodésulfuration peut correspondre à l'incorporation d'hydrogène au produit soufré $P_S$ pour donner le Sulfure d'hydrogène $H_2S$.

Après une reconstitution des représentations en coupes étroites de sortie $RCE_S$ , une étape d'intégration d'au moins un produit azoté $P_N$ et/ou d'au moins un produit soufré $P_S$ dans les représentations en coupes étroites de sortie $RCE_S$ peut permettre de déterminer au moins une représentation en coupe étroite de sortie corrigée $RCE_S^*$ .

Les représentations en coupes étroites de sortie corrigées $RCE_S^*$ peuvent être fractionnées, c'est-à-dire qu'elles peuvent être séparées afin de subir différents traitements selon les produits pétrolier raffines PPR désirés.

Comme illustre la figure 6, les représentations en coupes étroites de sortie corrigées $\mathrm{RCE}_{\mathrm{S}}^{*}$ peuvent être fractionnées lors des étapes de fractionnement post-réacteur. Les représentations en coupes de produit fractionné RCF peuvent correspondre à l'application d'une température d'ébullition réelle TBP comme point de coupe aux coupes étroites de sortie corrigées $\mathrm{RCE}_{\mathrm{S}}^{*}$ . La représentation en coupes étroites de sortie corrigées $\mathrm{RCE}_{\mathrm{S}}^{*}$ peut être fractionnée en représentation en coupes fractionnées RCF en fonction d'un intervalle de température d'ébullition réelle $I^{TBP}$. Les représentations en coupes fractionnées RCF peuvent être de type coupes légères, coupes moyennes et/ou coupes lourdes par exemple.

La fonction de répartition FR peut être appliquée en fonction du point de coupe. La fonction de répartition FR peut se définir comme suit :

$$FR = \frac{1}{1 + e^{-FI(CP-TBP)}}$$

Avec :

*FR* = Fonction de répartition
*FI* = Indice de Fractionnement
*CP* = Point de coupe Légère/Moyenne, Moyenne /Lourde et/ou Légère/Lourde
*TBP* = Fraction du Point d'ébullition réelle

L'indice de fractionnement *FI* peut être ajusté pour que le résultat de la modélisation représente la qualité de fractionnement réelle de l'unité à simuler.

La reconstitution en coupes étroites de la coupe large essence (20-185°C) permet ensuite de reconstituer la matrice compositionnelle qualité *M* pour cette coupe large essence (étape 790), suivant une même procédure que pour les autres coupes larges (distillats moyens et sous vide, résidus).

A titre d'exemple, la caractéristique Densité $D_{15/4}$ pour la coupe essence totale (50-185°C) calculée suivant cette procédure (répartition PIONA C5-C10, puis réaffectation des composants moléculaires C5-C10 aux coupes étroites d'après leur température d'ébullition) est présentée pour la coupe essence du brut ALASKA North $\left(\frac{\%wC_{\mathrm{ALASKA}}^{Ess}}{\%volC_{\mathrm{ALASKA}}^{Ess}}\right)$ dans la figure 15, et comparée à la courbe de densité du pétrole brut obtenue directement à partir des analyses $TBP\ \left(\frac{\%wC_{PPE}^{Ess}}{\%volC_{PPE}^{Ess}}\right)$ de ce pétrole brut. Il est montré que cette procédure permet de reconstituer avec une bonne approximation cette courbe de densité expérimentale (avec les pics de densité correspondants aux distillations des aromatiques benzènes (80°C), Toluène (110°C) et xylènes (140°C).

Une répartition type Paraffine Oléfine, Naphtènes et Aromatique PONA, c'est-à-dire une représentation en coupe C5-C10, spécifique à chaque procédé (par exemple le Craquage Catalytique en lit Fluidisé FCC, l'Hydrocraquage $C_H$, le vapocraquage Cv, la cokéfaction $C_K$, l'Hydrogénation H, le craquage thermique $C_{Th}$ et autre) est utilisée pour les coupes essences.

Les représentations en coupes étroites de sortie corrigées $RCE_S^*$ sont fractionnées, c'est-à-dire qu'elles sont séparées afin de subir différents fractionnements avec des points de coupes spécifiques à chaque modèle d'unité selon les produits pétrolier raffinés PPR désirés. Les représentations en coupes étroites de sortie corrigées $RCE_S^*$ sont fractionnées lors des étapes de fractionnement post-réacteur. Les représentations en coupes de produit fractionné *RCF* correspondent à l'application d'une température d'ébullition réelle TBP comme césure (aussi appelé point de coupe) aux coupes étroites de sortie corrigées $RCE_S^*$ . La représentation en coupes étroites de sortie corrigées $RCE_S^*$ est fractionnée en représentation en coupes fractionnées *RCF* en fonction d'un intervalle de température d'ébullition réelle $I^{TBP}$ (étape 795).

Le fractionnement du vecteur rendement est fait suivant les points coupes énoncés précédemment avec un fractionnement idéal ou fractionnement réel avec indice fractionnement.

Les fractionnements dans certaines unités, comme l'unité de distillation atmosphérique, l'unité de distillation sous vide et autres, sont basés sur la décomposition coupes étroites d'après les points de coupes de température d'ébullition réelle TBP.

Par rapport au fractionnement théorique, un indice de fractionnement est appliqué pour tenir compte du fractionnement réel, ce qui permet de calculer les rendements, compositions et qualités des produits corrigés avec un fractionnement représentatif. Il en résulte le graphique de la figure 17.

La fonction de répartition FR est appliquée en fonction du point de coupe. La fonction de répartition FR peut se définir comme suit :

$$FR = \frac{1}{1 + e^{-FI(CP-TBP)}}$$

Avec :

- $FR$ = Fonction de répartition
- $FI$ = Indice de Fractionnement
- $CP$ = Point de coupe Leger/Lourd
- $TBP$ = Fraction du Point d'ébullition réelle

La matrice compositionnelle-qualité $M^{PPE}$ du produit pétrolier d'entrée PPE peut comprendre, selon un deuxième mode de réalisation, une matrice compositionnelle-structure-qualité $m^{PPE}$. Cette matrice compositionnelle-structure-qualité $m^{PPE}$ peut comprendre une valeur caractéristique d'un élément chimique présent dans la coupe « *i* » dans le produit pétrolier d'entrée $el\chi C^{i}_{PPE}$ et/ou une valeur caractéristique d'une structure chimique présente dans la coupe « *i* » dans le produit pétrolier d'entrée $st\chi C^{i}_{PPE}$.

La valeur caractéristique d'un élément chimique d'une coupe « *i* » dans le produit pétrolier d'entrée $el\chi C^{i}_{PPE}$ peut correspondre à la représentation d'un élément chimique d'une coupe « *i* » du produit pétrolier brut $el\chi C^{i}_{PPB}$, si le produit pétrolier d'entrée PPE et le produit pétrolier brut PPB peuvent être confondus.

Il en va de même pour la valeur caractéristique d'une structure chimique, c'est-à-dire que la valeur caractéristique d'une structure chimique d'une coupe « *i* » dans le produit pétrolier d'entrée $st\chi C^{i}_{PPE}$ peut correspondre à la représentation d'une structure chimique d'une coupe « *i* » du produit pétrolier brut $st\chi C^{i}_{PPB}$, si le produit pétrolier d'entrée PPE et le produit pétrolier brut PPB peuvent être confondus.

La valeur caractéristique d'un élément chimique d'une coupe « *i* » dans le produit pétrolier d'entrée $el\chi C^{i}_{PPE}$ peut aussi correspondre à une somme de valeur caractéristique de l'élément chimique de la coupe « *i* » d'au moins un produit pétrolier brut $el\chi C^{i}_{PPB}$ pondérée par une valeur caractéristique d'une structure chimique d'une coupe « *i* » dans le produit pétrolier brut $st\chi C^{i}_{PPB}$ normée par la somme de valeur caractéristique de la structure chimique d'une coupe « *i* » dans le produit pétrolier brut $st\chi C^{i}_{PPB}$ :

La valeur caractéristique d'un élément chimique d'une coupe « *i* » dans le produit pétrolier d'entrée $el\chi C^{i}_{PPE}$ et la valeur caractéristique d'une structure chimique d'une coupe « *i* » dans le produit pétrolier d'entrée $st\chi C^{i}_{PPE}$ se pondèrent en moles, suivant la formule suivante correspondant au mélange de plusieurs pétroles bruts *PPB* en produit pétrolier d'entrée *PPE :*

$$\%molC^{i}_{PPE} = \sum_{PPB=1}^{n} \%molC^{i}_{PPB} * \%molC^{PPB}_{PPE}$$

$$el\chi C_{PPE}^{i} = \frac{\sum_{PPB=1}^{n} \%molC_{PPB}^{i} * \%molC_{PPE}^{PPB} * el\chi C_{PPB}^{i}}{\sum_{PPB=1}^{n} \%molC_{PPB}^{i} * \%molC_{PPE}^{PPB}}$$

et

$$st\chi C_{PPE}^{i} = \frac{\sum_{PPB=1}^{n} \%molC_{PPB}^{i} * \%molC_{PPE}^{PPB} * st\chi C_{PPB}^{i}}{\sum_{PPB=1}^{n} \%molC_{PPB}^{i} * \%molC_{PPE}^{PPB}}$$

Avec:

- $el\chi C_{PPE}^{i}$ : Valeur caractéristique d'un élément chimique d'un produit pétrolier d'entrée PPE ;
- $el\chi C_{PPB}^{i}$ : Valeur caractéristique de l'élément chimique d'un pétrole brut PPB ;
- $st\chi C_{PPE}^{i}$ : Valeur caractéristique de la structure chimique d'un produit pétrolier d'entrée PPE ;
- $st\chi C_{PPB}^{i}$ : Valeur caractéristique de la structure chimique d'un pétrole brut PPB ;
- $\%molC_{PPE}^{i}$ : pourcentage molaire de la coupe étroite « *i* » d'un produit pétrolier d'entrée
- $\%molC_{PPB}^{i}$ : pourcentage molaire de la coupe étroite « *i* » d'un pétrole brut
- $\%molC_{PPE}^{PPB}$ : pourcentage molaire du pétrole brut PPB dans le produit pétrolier d'entrée PPE.

Ainsi, il peut être déterminé une pseudo-molécule caractéristique de la coupe « *i* » avec une caractérisation moléculaire se présentant sous une forme de description stœchiométrique $A_{el\alpha C_{PPE}^{i}} B_{el\beta C_{PPE}^{i}} C_{el\gamma C_{PPE}^{i}}$ et une caractérisation structurale définissant la configuration de la pseudo-molécule caractéristique de la coupe étroite « *i* ».

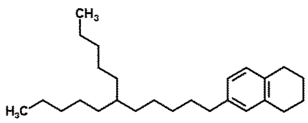

Par exemple, dans le cas où il est considéré qu'il n'y qu'une seule molécule 6-(6-Pentylundecyl)-1,2,3,4-tetrahydronaphthalene dans la coupe du produit pétrolier d'entrée, la valeur caractéristique de l'élément carbone de la coupe serait de $el_C C_{PPE}^{i} = 26$ et celle de l'hydrogène serait de $el_H C_{PPE}^{i} = 44$ . Toujours avec la même molécule, la valeur caractéristique d'élément carbone naphténique est de $Napht_C C_{PPB}^{i} = 4$ et la valeur caractéristique d'élément carbone aromatique est $Aro_C C_{PPB}^{i} = 6$ . Par ailleurs dans le 6-(6-Pentylundecyl)-1,2,3,4-tetrahydronaphthalene, la valeur caractéristique de nombre de cycle aromatique est de $CyArom_C C_{PPB}^{i} = 1$ et celle de nombre de cycle naphténique est de $CyNapht_C C_{PPB}^{i} = 1$ également. À cela s'ajoute $CyAro_C C_{PPB}^{i} = 2$ pour le nombre d'éléments carbones aromatiques formant la jonction entre cycles. À titre d'exemple non limitatif, une pseudo-molécule caractéristique de la coupe étroite « *i* » peut posséder près de 17 caractérisations structurales telles que : nombre de carbone paraffiniques ou présents dans des chaines alkyles substituées, nombre de carbone Naphténique, nombre de carbone aromatique, nombre de cycle aromatique, nombre de cycle naphténique, nombre de carbone aromatique (jonction entre cycles condensés (externe)), nombre de carbone aromatique (interne, cœur aromatique), nombre de carbone naphténique (jonction entre cycles condensés (externe)), nombre de carbone naphténique (interne, cœur naphténique), nombre de chaines alkyles reliées à un cœur naphténique/coeur aromatique, nombre de ponts entre cœur naphténique et cœur aromatique, nombre de carbone tertiaire et quaternaire, indice de condensation (cycles naphténiques/ cycles aromatiques), indice de branchement (nombre de chaines reliées à un cycle aromatique/cycle naphténique), indice d'isomérisation (carbone tertiaire, carbone quaternaire).

Il est à noter que dans ce mode de caractérisation des coupes étroites « *i* » en pseudo-molécules représentatives, les valeurs caractéristiques des éléments structuraux tels que définis ci-dessous sont des nombres réels positifs, sans être à priori des nombres entiers comme pour une représentation moléculaire réelle.

Ainsi, chaque coupe étroite d'entrée est ainsi caractérisée par une ensemble de paramètres structuraux correspondant aux valeurs d'éléments compositionnels et structuraux de cette « pseudo-molécule ».

La matrice compositionnelle-structure-qualité $m^{PPE}$ spécifique d'un produit pétrolier d'entrée PPE peut, dès lors, se présenter sous la forme représentée au tableau 2, pour une partie structurale de cette matrice, et pour les coupes étroites 220-230°C, 230-240°C, 240-250°C, 250-260°C, 260-270°C :

Tableau 2

| | $T_{IBP}$=220°C | $T_{IBP}$=230°C | $T_{IBP}$=240°C | $T_{IBP}$=250°C | $T_{IBP}$=260°C |
|---|---|---|---|---|---|
| | $T_{EBP}$=230°C | $T_{EBP}$=240°C | $T_{EBP}$=250°C | $T_{EBP}$=260°C | $T_{EBP}$=270°C |
| $C_c$ | 12,246 | 12,730 | 13,222 | 13,723 | 14,234 |
| $H_h$ | 23,058 | 23,864 | 24,674 | 25,491 | 26,317 |
| cycle naphthénique | 1,031 | 1,073 | 1,116 | 1,161 | 1,207 |
| cycle aromatique | 0,151 | 0,167 | 0,185 | 0,204 | 0,225 |
| carbone naphténique | 5,262 | 5,475 | 5,693 | 5,918 | 6,151 |
| carbone aromatique | 1,698 | 1,792 | 1,894 | 2,002 | 2,114 |

Il est à noter que cette représentation par pseudo-molécules sous forme de matrice compositionnelle-structure-qualité $m^{PPE}$ permet de reconstituer les principales propriétés physiques du produit pétrolier à simuler telles que la densité, de la viscosité, la température d'ébullition par exemple.

Cette représentation par pseudo-molécules est utilisée pour reconstituer les propriétés des coupes étroites structurales d'entrée $RCSE_E$ des produits pétroliers d'entrée *PPE* à simuler à partir de leur décomposition en vecteur rendement et en compositionnelle-structure-qualité $m^{PPE}$.

Cette reconstitution des propriétés physico-chimiques des coupes étroites structurales d'entrée $RCSE_E$ et des coupes larges structurales d'entrée $RCSL_E$ par intégration des coupes étroites structurales d'entrée $RCSE_E$ ) sont basées sur des corrélations structures-propriétés établies suivant le principe de contribution de groupes structuraux, sur une base de données d'un millier de molécules d'hydrocarbures identifiées de structures et composition très différentes, sur une plage de nombre d'atomes carbones par molécules de 4 à plus d'une centaine, et de températures d'ébullition de 0°C à plus de 800°C. Ces bases de données sont issues de sources telles que API (acronyme de « *American Petroleum Institute* ») et Chemspider de la Royal society of Chemistry.

En prenant comme exemples la reconstruction des propriétés physico-chimiques important comme la masse volumique et la température d'ébullition.

Pour la masse volumique, un coefficient de corrélation $r^2$ = 0,9921 sur un millier de molécules entre la masse volumique expérimentale et le masse volumique prédite par la corrélation est représenté sur le graphique à la figure 8. Cette corrélation est représentée sous la forme :

$$VM_i = \sum \chi\, A\, \chi * st\chi C^{i}_{PPB}$$

Avec:

- $VM_i$ : Volume Molaire coupe étroite « *i* »
- $A_X$ : coefficient pour valeur structural $S_X$
- $st\chi C^{i}_{PPB}$ : valeur structurale $_X$ de la coupe étroite « *i* »

Les $A_X$ sont issus de corrélations structures-propriétés établies suivant le principe de contribution de groupes structuraux, sur la base de données d'un millier de molécules d'hydrocarbures identifiées de structures et composition très différentes.

Le volume molaire permet alors de calculer la masse volumique. La qualité de prédiction - reconstitution de la masse volumique à partir de la décomposition en élément chimique $el_XC$ et valeur caractéristique d'une structure chimique $st_XC$ est démontrée dans le graphe de parité représentant la masse volumique prédite (contribution de groupes) en fonction de la masse volumique expérimentale (Chemspider) représentée sur la figure 8.

Pour la température d'ébullition, une corrélation avec un coefficient de corrélation $r^2$ = 0,9861 avec les éléments et valeurs structuraux a été établie sur un millier de molécules, sous la forme :

$$Teb_i = Tebnp_i + \sum\chi\ B\chi *\ st\chi C^i_{PPB}$$

Où :

- *$Tebnp_i$* : Température d'ébullition en Kelvin de l'hydrocarbure (normal paraffine) de même nombre d'atomes de carbone que la coupe étroite « *i* » :

  $el\chi C^i_{PPE}$

- $B_X$ *:* Coefficient pour groupe structural $S_X$
- $st\chi C^i_{PPB}$ : Indice structural coupe étroite « *i* »

Avec :

$$ln(Tebnp_i) = i_0 + i_1\ *\ ln\left(el\chi C^i_{PPE}\right) + i_2 * \ln\left(el\chi C^i_{PPE}\right)^2 + i_3 * ln(el\chi C^i_{PPE})^3$$

Avec $i_0$, $i_1$, $i_2$ et $i_3$ comme coefficients appartenant à $\mathbb{R}$

La qualité de prédiction-reconstitution de la température d'ébullition à partir de la décomposition en élément chimique $el_\chi C$ et valeur caractéristique d'une structure chimique $st_\chi C$ est démontrée dans le graphique de parité représentant la Température d'ébullition prédite (contribution de groupes) en fonction de la Température d'ébullition expérimentale (Chemspider) est représentée sur la figure 9.

Cette méthode permet de reconstituer les qualités (ici masse volumique) de coupes larges structurale d'entrée $RCSL_E$ *à* partir de la décomposition en coupes étroites structurale d'entrée $RCSE_E$ Cette même méthode permet de reconstituer les qualités des coupes larges structurales de sortie $RCSL_S$ à partir de la décomposition en coupes étroites structurale de sortie $RCSE_S$ .

Un exemple de reconstitution de qualité, en prenant les vecteurs rendements et matrice compositionnelle-structurale-qualité $m^{PPE}$, est fournie, à titre d'exemple, pour la coupe essences dont la température d'ébullition réelle TBP est comprise entre 50-200°C (représentée à la figure 10) et pour l'ensemble de la coupe dont la température d'ébullition réelle TBP s'étend jusqu'à 700 °C du brut Alaska North Slope à la figure 11. Le graphique représentée à la figure 10 représente une comparaison des courbes de densité expérimentales du crude assay et des courbes de densité reconstituées à partir de la décomposition en paramètres structuraux.

Dans ce mode de réalisation le vecteur rendement des produits pétrolier en entrée se calcule de manière similaire au premier mode de réalisation.

Dans la suite de la description, le vecteur rendement V et la matrice compositionnelle-structure-qualité *m* seront représentés sous forme de représentation en coupes étroites structurales d'entrée $RCSE_E$ .

Chaque représentation en coupes étroites structurales d'entrée $RCSE_E$ correspond à au moins une valeur caractéristique d'un élément chimique présent dans la coupe « *i* » dans le produit pétrolier d'entrée $el\chi C^i_{PPE}$ et/ou une valeur caractéristique d'une structure chimique présente dans la coupe « *i* » dans le produit pétrolier d'entrée $st\chi C^i_{PPE}$ dont la température d'ébullition réelle TBP peut être comprise dans un étroit intervalle de température d'ébullition $I^E_{TBP}$ .

À la représentation en coupes étroites structurales d'entrée $RCSE_E$ peut être appliqué un modèle réactionnel *R*. L'application du modèle réactionnel *R* à au moins une représentation en coupes étroites structurales d'entrée $RCSE_E$ peut permettre de déterminer au moins une représentation en coupes étroites structurales de sortie $RCSE_S$ d'un produit pétrolier intermédiaire PPI de sortie.

Le modèle réactionnel *R* peut être une représentation des différentes réactions que peut subir une représentation en coupes étroites structurales d'entrée $RCSE_E$ .

Le modèle réactionnel décrit par exemple les procédés de Craquage Catalytique en lit Fluidisé FCC, l'Hydrocraquage $C_H$, le vapocraquage $C_V$, la cokéfaction $C_K$, l'Hydrogénation H, le craquage thermique $C_{Th}$ et autre.

Le modèle réactionnel R peut être appliqué à la représentation en coupes étroites structurales d'entrée $RCSE_E$ et peut simuler les réactions avec des constantes cinétiques $k_v$ qui peuvent avoir lieu au sein d'un réacteur quel qu'en soit le mode de mise en œuvre (réacteur homogène, réacteur avec catalyseur en lit fixe, lit mobile, lit fluide, catalyseur dispersé dit « slurry »).

Ces réactions peuvent être la plupart du temps non-linéaires. Ces réactions peuvent être modélisées par un réseau cinétique réactionnel entre coupes étroites, avec des cinétiques de réaction d'ordre 1, 2 ou fractionnaire.

Le modèle réactionnel *R* est caractérisé par des mesures effectuées $\mu_{eff}$ dans au moins un réacteur. Ces mesures effectuées $\mu_{eff}$ dans au moins un réacteur sont enregistrées et archivées dans une base de données *DB* et peuvent être ensuite comparées avec les mesures archivées $\mu_{arch}$ de la base de données *DB.* De plus, la validité du modèle réactionnel *R* n'est pas limité au réacteur ou aux conditions opératoires utilisés pour mesurer ou estimer les constantes cinétiques, ce qui rend possible une interpolation et extrapolation à d'autres réacteurs ayant le même modèle réactionnel, avec des plages de fonctionnement différentes de celles correspondant à l'historique sur lequel est basé l'analyse statistique utilisée pour mesurer ou estimer les constantes cinétiques.

Le modèle réactionnel *R* est caractérisé par des constantes cinétiques *k*. Les constantes cinétiques *k* du modèle réactionnel *R* comprennent les mesures effectuées $\mu_{eff}$ dans au moins un réacteur. Ainsi un tel modèle réactionnel *R*, comprennant des mesures effectuées $\mu_{eff}$ dans au moins un réacteur, permet une planification non-linéaire de raffineries pétrolières, ce qui permet d'éviter une optimisation linéaire de certains critères. En effet, l'optimisation linéaire de certains critères (qui est valide uniquement dans les hypothèses très limitatives ou le critère à optimiser est fonction linéaire des variables, et ou les contraintes prises en compte pour déterminer des solutions faisables sont elles mêmes fonctions linéaires des variables à optimiser) est très limitative et grossièrement simplificatrice par rapport à la complexité hautement non-linéaire des procédés pétroliers et du réseau récursif des produits transformés dans une raffinerie.

Pour estimer les constantes cinétiques et paramètres du modèle réactionnel, des mesures de réactions cinétiques sont effectuées dans un réacteur afin de mieux prendre en compte le déroulement exact des réactions cinétiques dans un réacteur.

Ces mesures sont comparées avec des données d'une base de données *DB,* afin d'ajuster les constantes cinétiques *k* et permettre de représenter au mieux les réactions chimiques qui peuvent se produire dans le réacteur à modéliser.

C'est pourquoi un modèle réactionnel *R* peut être configuré pour déterminer au moins une représentation en coupe étroites structurales intermédiaire de sortie $RCSE_S$ à partir de l'au moins une représentation en coupe étroites structurales d'entrée $RCSE_E$ . Le modèle réactionnel *R* peut être un ensemble interconnecté de réactions cinétiques pouvant permettre de modéliser la réaction de transformation d'une représentation en coupe étroites structurales d'entrée $RCSE_E$ en au moins une représentation en coupe étroites structurales intermédiaire $RCSE_I$ ou en au moins une représentation en coupe étroites structurales de sortie $RCSE_S$ . Par exemple, lors du séjour $t_s$ d'une représentation en coupe étroites structurales d'entrée $RCSE_E$ dans un réacteur, la représentation en coupes étroites structurales d'entrée $RCSE_E$ peut se décomposer et peut produire des produits intermédiaires PPI issus du réseau cinétique $R_k$ , avant de résulter en représentation en coupe étroites structurales de sortie $RCSE_S$ .

Un réseau cinétique $R_k$ peut comprendre au moins une réaction cinétique. Un réseau cinétique $R_k$ peut intégrer au moins une décomposition cinétique d'un produit pétrolier d'entrée PPE, représenté en coupe étroites structurales d'entrée $RCSE_E$ , sur son temps de séjour $t_s^{RCSE_E}$ et sur sa température de réaction $T°_{RCSE_E}$ dans le réacteur. Le réseau cinétique $R_k$ peut être aussi configuré pour intégrer une décomposition cinétique pondérée des représentations en coupes étroites structurales d'entrée $RCSE_E$ sur leurs temps de séjour $t_s^{RCSE_E}$ et sur leurs températures de réaction $T°_{RCSEE}$ dans le réacteur.

La détermination d'une représentation en coupes étroites structurales de sortie $RCSE_S$ comprend une étape d'application d'une fonction de distribution *p*(*x*) à la représentation en coupes étroites structurales de sortie $RCSE_S$ .

Nous prenons comme exemple de réseau cinétique réactionnel le procédé d'hydrocraquage $C_H$ , d'autres réseaux cinétiques adaptés spécifiquement étant utilisés pour les autres procédés de Craquage Catalytique en lit Fluidisé FCC, l'Hydrotraitement $C_{Ht}$, le vapocraquage $C_V$, la cokéfaction $C_K$, l'Hydrogénation H, le craquage thermique $C_{Th}$ et autre.

Dans une réaction d'hydrocraquage, une pseudo-molécule de valeur caractéristique d'un élément carbone $el_CC$ peut comprendre un nombre d'atome de carbone égale à $N_j\ (N_j \in \mathbb{R}_*^+)$ est craquée en deux molécules de nombres de carbone $N_i$ *et* $N_k\ (N_i, N_k \in \mathbb{R}_*^{+,})$ *et* $N_i, N_k < N_j$

La réaction moléculaire est la suivante :

$$C_{N_j}H_x + H_2 \Rightarrow C_{N_i}H_y + C_{N_k}H_z$$

avec

$$N_k = N_j - N_i\,, \quad \text{et}\; x + 2 = y + z$$

Puisqu'il y a conservation du nombre d'atomes de carbone et du nombre d'atomes d'hydrogène pour une réaction d'hydrocraquage.

La cinétique réactionnelle cinétique d'hydrocraquage de la coupe étroite structurale d'entrée $RCSE_E^j$ est décrite par une équation cinétique, dans le cas présent d'ordre 1 par rapport aux réactifs hydrocarbures coupe étroite structurale d'entrée $RCSE_E$ et hydrogène.

$$dCj = -kj * Cj * ppH_2 * d(t_s)$$

Avec Cj : Concentration (moléculaire) en coupe étroites structurales d'entrée $RCSE_E^j$ de température d'ébullition Tj
$t_s$ : Temps de séjour dans le réacteur
kj : constante cinétique spécifique de la coupe étroite « *j* », dépendant de la température de réaction.
$ppH_2$ : pression partielle d'$H_2$

Les produits de cette réaction d'hydrocraquage sont réparties dans les coupes étroites de sorties $RCSE_S^i$

Cette répartition des produits de réactions de la coupe étroite d'entrée « *j* » en coupes étroites de sorties « *i* » se fait suivant une fonction de distribution *D(Ni,Nj).* Cette fonction de distribution est symétrique par rapport à $N_j/2$, ce qui se traduit par *D*(*Ni, Nj*) = *D*(*Nj* — *Ni, Nj*) .

Une molécule de nombre d'atomes de carbone $N_j$ se craque en 2 molécules de nombre d'atomes de carbone respectivement *Ni* et *Nj* — *Ni.* Il est à noter que ces nombres d'atomes de carbone *Nj* et *Ni,* valeur caractéristique de la structure chimique respectivement d'entrée $RCSE_E^j$ et de sortie $RCSE_S^i$, sont des nombres réels positifs, sans être à priori entiers. Il s'agit donc de réactions entre pseudo-molécules.

Un exemple de fonction de distribution des produits de la réaction en coupes étroites de sorties peut être une fonction Gamma symétrique par rapport à $^{Nj}/_2$, telle que :

$$D(Ni,Nj) = \frac{((Nj/Ni)^{\alpha-1}(1 - Nj/Ni)^{\beta-1} + (1 - Nj/Ni)^{\alpha-1}(Nj/Ni)^{\beta-1})\Gamma(\alpha+\beta)}{2\Gamma(\alpha)\Gamma(\beta)NORM(j)}$$

Dans cette fonction de distribution, $\alpha$ et $\beta$ sont des paramètres de formes qui permettent d'ajuster la distribution des produits de la réaction aux caractéristiques spécifiques du réacteur d'hydrocraquage à représenter (en particulier la sélectivité du catalyseur).

Avec *Ni* < *Nj.* Le coefficient de normalisation *NORM*(*j*) permet une normalisation à 2 du nombre de molécules en sortie produit d'une réaction d'hydrocraquage de coupe étroite $RCSE_E^j$ .

Dans ce mode de réalisation, les étapes de fractionnement post réacteur se calculent de manière similaire au premier mode de réalisation.

Dans la suite, le premier mode de réalisation de la présente invention est décrit plus en détail et plus spécifiquement à l'aide d'un exemple illustré par la figure 7, lequel n'entend toutefois pas limiter la présente invention.

Comme il a été décrit précédemment dans la description, les produits pétroliers brut PPB contiennent diverses composants comme par exemple des molécules d'hydrocarbures dites paraffiniques (alcanes), des naphténiques (molécules cycliques saturées), des aromatiques, des sulfures, thiols, benzothiophènes et des composants chimiques pouvant comprendre au moins un élément azote et/ou soufre.

Dans la suite de notre exemple, nous considèrerons les analyses de trois produits pétroliers bruts *PPB.*

Les données incomplètes, issues des analyses des trois produits pétroliers bruts *PPB,* sont représentées sous la forme d'une matrice compositionnelle-structure-qualité *M* et/ou vecteur rendement *V* spécifique du produit pétrolier

brut *PPB* (étape 700).

Il va de soi que si les données sont complètes, cette étape décrite précédemment est omise lors du procédé.

Le procédé de détermination détermine, à partir de données incomplètes, la composition du produit pétrolier brut *PPB* à partir de la fonction de distribution p(x). Une fois les produits pétroliers bruts complétés par la fonction de distribution p(x), le produit pétrolier d'entrée *PPE* est formé à partir des produits pétroliers bruts complétés.

Le produit pétrolier d'entrée *PPE* comprend, dans cet exemple, une représentation de dix coupes étroites d'entrée $RCE_E$ : D01, D02, D03, D04, D05, D06, V07, V08, V09 et R10 (étape 710).

Lors de la formation de la représentation en coupes étroites d'entrée $RCE_E$ , les produits azotés $P_N$ et les produits soufrés $P_S$ sont extraits des représentations en coupes étroites d'entrée $RCE_E$ .

La formation du produit pétrolier d'entrée *PPE* se fait donc de la manière suivante :

- pour la valeur caractéristique volumique :

$$volc_{PPE}^{i} = \frac{\sum_{PPB=1}^{n} volcC_{PPB}^{i} * \%volC_{PPB}^{i} * \%volC_{PPE}^{PPB}}{\sum_{PPB=1}^{n} \%volC_{PPB}^{i} * \%volC_{PPE}^{PPB}}$$

- pour la valeur caractéristique massique :

$$wc_{PPE}^{i} = \frac{\sum_{PPB=1}^{n} wcC_{PPB}^{i} * \%wC_{PPB}^{i} * \%wC_{PPE}^{PPB}}{\sum_{PPB=1}^{n} \%wC_{PPB}^{i} * \%wC_{PPE}^{PPB}}$$

Tableau 3

| *Coupes étroites* | D01 | D02 | D03 | D04 | D05 | D06 | V07 | V08 | V09 | R10 |
|---|---|---|---|---|---|---|---|---|---|---|
| $T_{IBP}$ | 200°C | 220°C | 260°C | 300°C | 340°C | 380°C | 420°C | 460°C | 500°C | 750°C |
| $T_{EBP}$ | 220°C | 260°C | 300°C | 340°C | 380°C | 420°C | 460°C | 500°C | 750°C | 1000°C |

Dans la suite de l'exemple, la matrice compositionnelle - qualité M est représentée sous forme de représentation en coupes étroites d'entrée $RCE_E$ . Chaque coupe étroite d'entrée $CE_E$ correspondant à une valeur caractéristique volumique $\%volC_{PPE}^{CE_E}$ et/ou massique $\%wC_{PPE}^{CE_E}$ et/ou molaire $\%mC_{PPE}^{CE_E}$ du produit pétrolier PPE d'entrée dont la température d'ébullition réelle TBP peut être comprise dans un étroit intervalle de température d'ébullition $I_{TBP}^{E}$ .

Par exemple, la coupe étroite d'entrée $D04_E$ a une valeur caractéristique volumique $\%volC_{PPE}^{D04}$ et/ou massique $\%wC_{PPE}^{D04}$ et/ou molaire $\%molC_{PPE}^{D04}$ du produit pétrolier d'entrée dont la température d'ébullition réelle TBP peut être comprise entre environ $T_{IBP}$=300°C et environ $T_{EBP}$=340°C.

À partir de la représentation en coupes étroites d'entrée $RCE_E$ , la représentation en coupes larges d'entrée $RCL_E$ du produit pétrolier d'entrée sont déterminées (étape 720). Chaque coupe large d'entrée $RCL_E$ correspondant à une somme de valeur caractéristique volumique $\%volC_{PPE}^{LE_E}$ et/ou massique $\%wC_{PPE}^{LE_E}$ et/ou molaire $\%molC_{PPE}^{LE_E}$ du produit pétrolier d'entrée PPE dont la température d'ébullition réelle TBP comprend plusieurs intervalles de température d'ébullition réelle $I_{TBP}^{E}$ de coupes étroites d'entrée $RCE_E$ compris dans un large intervalle de température d'ébullition réelle $I_{TBP}^{L}$ d'une coupe large d'entrée $RCL_E$ (étape 720). Par exemple :

- $(D02 - D04)_E = D02_E + D03_E + D04_E$
- $(D05 - R10)_E = D05_E + D06_E + V07_E + V08_E + V09_E + R10_E$

Tableau 4

| *Coupes larges* | $(D02 — D04)_E$ | $(D05 - R10)_E$ |
|---|---|---|
| $T_{IBP}$ | 220°C | 340°C |
| $T_{EBP}$ | 340°C | 1 000°C |
| Gasoil sous vide | 0,26% | 99,74% |
| $T_{IBP}$ | 220°C | 340°C |
| $T_{EBP}$ | 340°C | 1 000°C |

Dans cet exemple, la coupe $(D05 - R10)_E$ désignera une coupe Gasoil sous vide et est décomposée en :

- fraction hydrocarbure saturé (*Sat.*) à 41,51% ;
- fraction hydrocarbure aromatique (*Aro.*) à 49,64% ;
- fraction hydrocarbure résineux (*Rés.*) à 8,17% ; et,
- fraction asphaltène (*Asp.*) à 0,42%.

La représentation en coupe large d'entrée $RCL_E$ prend la forme du tableau 5.

Tableau 5

| *Coupes larges* | $(D02 — D04)_E$ | $(D05 — R10)_E$ | *Sat.* | *Aro.* | *Rés.* | *Asp.* |
|---|---|---|---|---|---|---|
| Gasoil sous vide (VGO) | 0,26% | 99,74% | 41,51% | 49,64% | 8,17% | 0,42% |

À la représentation en coupe large d'entrée $RCL_E$ est appliqué un modèle réactionnel *R* (étape 730). L'application du modèle réactionnel *R* à la représentation en coupes larges d'entrée $RCL_E$ permet de déterminer une représentation en coupes larges de sortie $RCL_S$ d'un produit pétrolier intermédiaire PPI de sortie. Le modèle réactionnel *R* est une représentation des différentes réactions que peut subir le produit pétrolier d'entrée, représenté en coupes larges d'entrée $RCL_E$.

Le modèle réactionnel R est un ensemble interconnecté de réactions cinétiques et se présente sous forme de réseau cinétique $R_k$. Le réseau cinétique $R_k$ simule les réactions cinétiques qui ont lieu au sein d'un réacteur. Ces réactions cinétiques sont la plupart du temps non-linéaires.

En effet, des mesures de réactions cinétiques sont effectuées $\mu_{eff}$ dans un réacteur afin de mieux prendre en compte le déroulement exact des réactions cinétiques et du réseau cinétique $R_k$ dans un réacteur.

Ces mesures $\mu_{eff}$ sont comparées avec des données d'une base de données DB. Ces mesures sont ensuite ajustées et intégrées au modèle réactionnel R, en l'occurrence au réseau cinétique $R_k$.

Le modèle réactionnel *R* est configuré pour déterminer à partir de la coupe Gasoil sous vide une représentation en coupes larges de sortie $RCL_S$ comme par exemple :

- Coupe Coke
- Coupe Gaz (Dihydrogène, , Méthane, Éthane, Ethylène)
- Coupe C3
- Coupe C4
- Coupe Gazoline (coupe C5 — 220°C)
- Coupe de fioul léger LCO (220°C-340°C)
- Coupe Gasoil sous vide (GSV - >340°C)

Ces réactions cinétiques peuvent se présenter sous la forme du tableau 6.

Tableau 6

| | Réactifs |
|---|---|

(suite)

| | R | Coke | Gaz | C3 | C4 | Essence | LCO | VGO |
|---|---|---|---|---|---|---|---|---|
| Produits | Coke | | | | | $k_{15}$ | $k_{11}$ | $k_6$ |
| | Gaz | | | $k_{18}$ | $k_{17}$ | $k_{14}$ | $k_{10}$ | $k_5$ |
| | C3 | | | | $k_{16}$ | $k_{13}$ | $k_9$ | $k_4$ |
| | C4 | | | | | $k_{12}$ | $k_8$ | $k_3$ |
| | Essence | | | | | | $k_7$ | $k_2$ |
| | LCO | | | | | | | $k_1$ |
| | VGO (HCO) | | | | | | | |

Les valeurs de constante cinétique du réseau cinétique ajustées aux mesures réalisées sur un réacteur sont représentées au tableau 7.

Tableau 7

| R | Coke | Gaz | C3 | C4 | Essence | LCO | VGO |
|---|---|---|---|---|---|---|---|
| Coke | | | | | 0,92 | 54,98 | 217,64 |
| Gaz | | | 205,27 | 8,94 | 25,74 | 19,29 | 76,35 |
| C3 | | | | 19,15 | 57,34 | 38,38 | 151,92 |
| C4 | | | | | 104,36 | 81,32 | 321,89 |
| Essence | | | | | | 802,96 | 3178,33 |
| LCO | | | | | | | 6022,20 |
| VGO | | | | | | | |

Les valeurs de constantes cinétiques sont également ajustées en fonction de la composition de chaque coupe, en utilisant les données extraites de la matrice réactionnelle. Ainsi, à titre d'exemple, les constantes cinétiques relatives à la décomposition du VGO, c'est-à-dire les constantes k1 à k6 dans l'exemple ci-dessus, sont ajustées en fonction des composantes selon le modèle SARA.

Le tableau 8 ci-dessous reprend les coefficients de correction des constantes cinétiques appliqués pour chaque composante SARA.

Tableau 8

| R | Feed | Sat. | Aro. | Res. | Asph |
|---|---|---|---|---|---|
| | $k$ | $\frac{k}{k_{Feed}}$ | $\frac{k}{k_{Feed}}$ | $\frac{k}{k_{Feed}}$ | $\frac{k}{k_{Feed}}$ |
| $k_6$ | 217,64 | 1 | 1 | 300 | 1000 |
| $k_5$ | 76,35 | 1 | 1 | 100 | 300 |
| $k_4$ | 151,92 | 1,5 | 1,5 | 5 | 10 |
| $k_3$ | 321,89 | 1,5 | 1,5 | 5 | 10 |
| $k_2$ | 3178,33 | 3 | 2 | 2 | 2 |
| $k_1$ | 6022,20 | 2 | 3 | 2 | 1 |

Ainsi, l'utilisation de la matrice permet d'adapter le modèle et de refléter plus précisément le comportement de la réaction en fonction de la décomposition en composantes SARA de la coupe VGO.

Dans l'exemple présenté ici, il est à noter que les coupes de sortie ne sont pas décomposées en un ensemble

de composantes en fonction de leur composition. Il est toutefois également possible d'utiliser une telle décomposition. Dans ce cas, il conviendrait également d'adapter chaque constante cinétique en fonction de la composante de destination. Bien entendu, une telle approche augmente le nombre de coefficients de correction à appliquer à chaque constante cinétique pour un couple donnée entre une composante de la coupe d'entrée et une composante de la coupe de sortie.

Le modèle réactionnel R intègre la décomposition cinétique du Gasoil sous vide VGO sur son temps de séjour $t_S^{VGO}$ et sur sa température de réaction $T°_{VGO}$ dans le réacteur. La figure 12 donne un exemple de cette décomposition.

Le calcul des rendements du Gasoil sous vide est effectué par intégration des équations différentielles représentant les valeurs caractéristiques massiques des représentations en coupes larges d'entrée. Ceci permet de modéliser l'influence des paramètres comme le temps de séjour et sur leurs températures de réaction dans le réacteur par son effet sur les constantes cinétiques k.

La coupe Gasoil Sous Vide (qui est transformée en coupe Fioul par la réaction de cracking catalytique) est décomposée en fractions avec des constantes de réaction spécifiques :

- les fractions Saturates et Aromatiques précurseurs d'Essence.
- les fractions Asphaltènes et partiellement les Résines précurseurs de coke.

Il en résulte le graphique de la figure 13.

Comme il peut être observé sur le graphique de la figure 13, la coupe Gasoil Sous Vide se décompose en coupe fioul léger LCO, en coupe Essence, en coupe Gaz et en coupe coke principalement.

Après avoir atteint son maximum de rendement à environ 30% du taux de conversion, la coupe fioul léger favorise la formation de coupe Essence. Cette même coupe essence atteint son maximum de rendement pour un taux de conversion d'environ 85%, puis est craquée en produits plus légers (point « d'overcracking »).

Le craquage catalytique en lit fluidisé FCC produit une coupe essence, une coupe de gaz de pétrole liquéfié GPL, une coupe de fioul léger LCO, une coupe de gaz légers Gaz et une coupe de Coke déposée sur le catalyseur en circulation dans le réacteur, et ultérieurement brulée dans le régénérateur.

À la sortie du réacteur (étape 740), la coupe Essence est majoritaire et la coupe de fioul léger minoritaire ainsi que les coupes Gaz et Coke. La coupe d'Essence, la coupe de fioul léger, ainsi que les coupes Gaz et Coke sont représentées sous forme de représentation en coupes larges de sortie.

Aux différentes coupes larges de sortie issues du modèle réactionnel, la fonction de distribution p(x) est appliquée afin de déterminer la représentation en coupes étroites de sortie (étape 750). Les coupes étroites de sortie en fonction de la température d'ébullition réelle sont représentées sur le graphique de la figure 14 et se définissent comme suit :

- Coupe Gaz + Coupe GPL + Coupe Coke ($T_{IBP}$=20°C et $T_{EBP}$=200°C)
- Coupe Gaz + Coupe GPL + Coupe Coke + Coupe Essence ($T_{IBP}$=200°C et $T_{EBP}$=350°C)
- Coupe Gaz + Coupe GPL + Coupe Coke + Coupe Essence + Coupe LCO ($T_{IBP}$=350°C).

La détermination d'une représentation en coupes étroites de sortie comprend une étape d'application d'une fonction de distribution p(x) à la représentation en coupes larges de sortie.

Afin de s'assurer du bon déroulement de l'application de la fonction de distribution p(x), un bilan de matière $B_M$ et/ou un bilan élémentaire $B_{ELEM}$ est appliqué entre la représentation en coupes étroites d'entrée $RCE_E$ et la représentation en coupes étroites de sortie $RCE_S$ (étape 760).

Les produits azotés $P_N$ et des produits soufrés $P_S$ possèdent un modèle d'hydrogénation $R_H$. Le modèle d'hydrogénation $R_H$ comprend un réseau cinétique configuré pour intégrer la décomposition cinétique des produits azotés $P_N$ et des produits soufrés $P_S$ sur le temps de séjour $t_s$ et sur la température T° de réaction dans le réacteur et/ou au moins une unité d'épuration.

Un procédé d'hydrodésulfuration sépare les produits soufrés $P_S$ des représentations en coupes étroites de sortie $RCE_S$ .

Par exemple: une coupe gazole et/ou une coupe de fioul domestique est séparé par hydrodésulfuration des produits soufrés $P_S$ .

Le modèle d'hydrogénation $R_H$ correspond à une hydrodésulfuration d'une représentation en coupe étroite d'entrée dans un réacteur en présence d'hydrogène, sur un lit catalytique entre environ 320°C et environ 390°C et sous une pression d'environ 18 et d'environ 51 bars par exemple. Le modèle d'hydrogénation $R_H$ peut correspondre à l'incorporation d'hydrogène au produit soufré $P_S$ pour donner le Sulfure d'hydrogène $H_2S$ comme dans une étape d'hydrodésulfuration.

Le Sulfure d'hydrogène $H_2S$ est mélangé à la représentation en coupes étroites de sortie $RCE_S$ pour donner

une représentation en coupes étroites de sortie corrigées $RCE_S^*$ , par exemple, une coupe de gazole moteur et/ou une coupe de fioul domestique.

Le modèle d'hydrogénation $R_H$ est aussi appliqué aux produits azotés $P_N$ pour donner par exemple de l'ammoniac NH3.

Les produits azotés $P_N$ et des produits soufrés $P_S$ hydrogénés, c'est-à-dire l'ammoniac $NH_3$ et le Sulfure d'hydrogène $H_2S$ sont répartis dans les Coupes Gazoline, fioul léger LCO et Gasoil sous vide GSV.

Après obtention de la représentation en coupes étroites de sortie corrigées $RCE_S$ , la matrice compositionnelle-structure-qualité M est reconstituée en respectant le bilan élémentaire $B_{ELEM}$ et la représentation en coupes étroites de sortie corrigées $RCE_S$ .

À partir de la représentation en coupes étroites de sortie corrigées $RCE_S$ , la densité $D_{4/15}$ des coupes étroites sont calculées.

Les qualités pétrolières, c'est-à-dire le point de trouble, le point de congélation, l'indice de cétane et la viscosité par exemple, sont calculées par corrélations à partir des compositions élémentaires C,H,S,N, moléculaires PONA (sur les coupes distillats moyens) et SARA (sur les coupes distillats sous vide et résidus).

La reconstitution de la représentation en coupes étroites de sortie à partir des coupes larges de sortie est différente pour les coupes essences (intervalle de distillation 20-185 °C), ce qui correspond aux composants moléculaires C5-C10 avec une décomposition PIONA (normal paraffines, isoparaffines, oléfines, naphtènes, aromatiques, où il est procédé à une étape de répartition du rendement 740 en coupe large essence (20-185°C) en composants moléculaires C5-C10, d'après une fonction de distribution p(x) dont les paramètres sont spécifiques à chaque procédé (par exemple le Craquage Catalytique en lit Fluidisé FCC, l'Hydrocraquage $C_H$, le vapocraquage $C_V$, la cokéfaction $C_K$, l'Hydrogénation H, le craquage thermique $C_{Th}$ et autre) et peuvent être ajustés aux réacteur et aux conditions opératoires à modéliser (étape 780). Suite à cette étape, l'étape 790 est réalisée.

Un exemple en figure 14 illustre la répartition PIONA C5-C10 pour les composants moléculaires de la coupe large essence, modélisée par une fonction de répartition gamma, dans le cas d'une unité de Craquage Catalytique en lit Fluidisé FCC.

Dans cet exemple, il peut être remarqué que la décomposition PIONA comprend les éléments suivant :

- les normales paraffines comprennent à peu près 1% d'éléments à 5 atomes de carbones, un peu moins de 2% d'éléments à 6 atomes de carbones, un peu moins de 2% d'éléments à 7 atomes de carbones, environ 2% d'éléments à 8 atomes de carbones, près de 1% d'éléments à 9 atomes de carbones et moins de 1% d'éléments à 10 atomes de carbones ;
- les isoparaffines comprennent un peu moins de 6%d'éléments à 5 atomes de carbones, un peu plus de 6% d'éléments à 6 atomes de carbones, moins de 4% d'éléments à 7 atomes de carbones, environ 2% d'éléments à 8 atomes de carbones, entre 1% et 2% d'éléments à 9 atomes de carbones et moins de 10% d'éléments à 10 atomes de carbones ;
- les oléfines comprennent près de 10% d'éléments à 5 atomes de carbones, un peu plus de 9% d'éléments à 6 atomes de carbones, entre 7% et 6% d'éléments à 7 atomes de carbones, un peu moins 4% d'éléments à 8 atomes de carbones, presque 2% d'éléments à 9 atomes de carbones et entre 2% et 1% d'éléments à 10 atomes de carbones ;
- les naphtènes comprennent environ 1% d'éléments à 5 atomes de carbones, environ 2% d'éléments à 6 atomes de carbones, un peu plus de 2% d'éléments à 7 atomes de carbones, environ 2% d'éléments à 8 atomes de carbones, un peu plus de 1,5% d'éléments à 9 atomes de carbones et moins de 1% d'éléments à 10 atomes de carbones ;
- les aromatiques comprend peu d'éléments à 6 atomes de carbones, un peu plus de 2% d'éléments à 7 atomes de carbones, environ 9% d'éléments à 8 atomes de carbones, presque 10% d'éléments à 9 atomes de carbones et moins de 6% d'éléments à 10 atomes de carbones.

Les composants moléculaires C5-C10 PIONA sont ensuite distribués dans la représentation en coupes étroites TBP d'après leur température d'ébullition par exemple nC6 (normal hexane), de température d'ébullition 68,73°C affecté à la coupe étroite 65-70°C, A6 (benzène), de température d'ébullition 80,1°C, affecté à la coupe étroite 80-85°C. Pour des composants moléculaires constitués de différents isomères de températures d'ébullition différentes (par exemple IP7 isoparaffines en C7), une fonction de répartition FR permet d'affecter le composant moléculaire à plusieurs coupes étroites adjacentes, d'après la température d'ébullition moyenne et les températures (basse, haute) de coupe étroites, avec un paramètre de distribution (indice de fractionnement *FI)* prenant en compte la plage de température d'ébullition des différents isomères.

$$FR = \frac{1}{1 + e^{-FI(CP-BP)}}$$

L'indice de fractionnement *FI* de la fonction de répartition FR est un paramètre de distribution des températures d'ébullition des isomères autour de la température d'ébullition moyenne *BP.*

La reconstitution en coupes étroites de la coupe large essence (20-185°C) permet ensuite de reconstituer la matrice compositionnelle qualité M pour cette coupe large essence (étape 790), suivant une même procédure que pour les autres coupes larges (distillats moyens et sous vide, résidus).

A titre d'exemple, la caractéristique Densité $D_{15/4}$ pour la coupe essence totale (50-185°C) calculée suivant cette procédure (répartition PIONA C5-C10, puis réaffectation des composants moléculaires C5-C10 aux coupes étroites d'après leur température d'ébullition) est présentée pour la coupe essence du brut ALASKA North Slope $\left(\frac{\%wC_{ALASKA}^{Ess}}{\%volC_{ALASKA}^{Ess}}\right)$, dans la figure 15, et comparée à la courbe de densité du pétrole brut obtenue directement à partir des analyses $TBP\ \left(\frac{\%wC_{PPE}^{Ess}}{\%volC_{PPE}^{Ess}}\right)$ de ce pétrole brut. Il est montré que cette procédure permet de reconstituer avec une bonne approximation cette courbe de densité expérimentale (avec les pics de densité correspondants aux distillations des aromatiques benzènes (80°C), Toluène (110°C) et xylènes (140°C).

Une répartition type Paraffine Oléfine, Naphtènes et Aromatique PONA, c'est-à-dire une représentation en coupe C5-C10, spécifique à chaque procédé (par exemple le Craquage Catalytique en lit Fluidisé FCC, l'Hydrocraquage CH, le vapocraquage CV, la cokéfaction CK, l'Hydrogénation H, le craquage thermique CTh et autre) est utilisée pour les coupes essences.

Les représentations en coupes étroites de sortie corrigées $RCE_S^*$ sont fractionnées, c'est-à-dire qu'elles sont séparées afin de subir différents fractionnements avec des points de coupes spécifiques à chaque modèle d'unité selon les produits pétrolier raffinés PPR désirés. Les représentations en coupes étroites de sortie corrigées $RCE_S^*$ sont fractionnées lors des étapes de fractionnement post-réacteur. Les représentations en coupes de produit fractionné RCF correspondent à l'application d'une température d'ébullition réelle TBP comme césure (aussi appelé point de coupe) aux coupes étroites de sortie corrigées $RCE_S^*$. La représentation en coupes étroites de sortie corrigées $RCE_S^*$ est fractionnée en représentation en coupes fractionnées RCF en fonction d'un intervalle de température d'ébullition réelle $I^{TBP}$ (étape 795).

Le fractionnement du vecteur rendement est fait suivant les points coupes énoncés précédemment avec un fractionnement idéal ou fractionnement réel avec indice fractionnement.

Les fractionnements dans certaines unités, comme l'unité de distillation atmosphérique, l'unité de distillation sous vide et autres, sont basés sur la décomposition coupes étroites d'après les points de coupes de température d'ébullition réelle TBP.

Par rapport au fractionnement théorique, un indice de fractionnement est appliqué pour tenir compte du fractionnement réel, ce qui permet de calculer les rendements, compositions et qualités des produits corrigés avec un fractionnement représentatif. Il en résulte le graphique de la figure 17.

La fonction de répartition FR est appliquée en fonction du point de coupe. La fonction de répartition FR peut se définir comme suit :

$$FR = \frac{1}{1 + e^{-FI(CP-TBP)}}$$

Avec :

*FR* = Fonction de répartition
*FI* = Indice de Fractionnement
*CP* = Point de coupe Leger/Lourd
*TBP* = Fraction du Point d'ébullition réelle

Dans la suite, le deuxième mode de réalisation de la présente invention est décrit plus en détail et plus spécifiquement à l'aide d'un exemple illustré par la figure 7, lequel n'entend toutefois pas limiter la présente invention.

Les données issues des analyses de résidus sous vides issus de trois produits pétroliers bruts *PPB,* sont représentées sous la forme d'une matrice compositionnelle-structure-qualité M et/ou vecteur rendement V spécifique du produit pétrolier brut *PPB* (étape 700).

Le procédé de détermination détermine, à partir de données incomplètes, la composition structurale du produit pétrolier brut PPB à partir de la fonction de distribution p(x). Une fois les produits pétroliers bruts complétés par la fonction de distribution p(x), le produit pétrolier d'entrée PPE est formé à partir des produits pétroliers bruts complétés.

Il va de soi que si les données sont complètes cette étapes décrite précédemment omise lors du procédé.

Dans la suite de l'exemple, la matrice compositionnelle-structure-qualité m est représentée sous forme de représentation en coupes étroites structurales d'entrée $RCSE_E$ . Chaque coupe étroite structurale d'entrée $CSE_E$ correspondant à une valeur caractéristique d'un élément chimique présent dans la coupe étroite structurale d'entrée $CSE_E$ dans le produit pétrolier d'entrée $el\chi C_{PPE}^{CSE_E}$ et/ou une valeur caractéristique d'une structure chimique présente dans la coupe $CSE_E$ dans le produit pétrolier d'entrée $st\chi C_{PPE}^{CSE_E}$ du produit pétrolier PPE d'entrée dont la température d'ébullition réelle TBP peut être comprise dans un étroit intervalle de température d'ébullition $I_{TBP}^{E}$ .

Par exemple, la coupe étroite structurale d'entrée $V58_E$ a une valeur caractéristique d'un élément chimique $el\chi C_{PPE}^{V58}$ et/ou valeur caractéristique d'une structure chimique $st\chi C_{PPE}^{V58}$ du produit pétrolier d'entrée dont la température d'ébullition réelle TBP peut être comprise entre $T_{IBP}$=520°C et $T_{EBP}$=540°C.

Un titre d'exemple non limitatif de cette matrice compositionnelle-structure-qualité est représenté ci-dessous, pour les coupes étroites de température d'ébullition compris entre 500°C et 580°C, utiles pour représenter un résidu sous vide.

Tableau 9

| *Coupes étroites* | V57 | V58 | V59 | V60 |
|---|---|---|---|---|
| $T_{IBP}$ | 500°C | 520°C | 540°C | 560°C |
| $T_{EBP}$ | 520°C | 540°C | 560°C | 580°C |
| $\%wC_{PPF}^{i}$ | 0,03 | 0,05 | 0,06 | 0,07 |
| $\%volC_{PPF}^{i}$ | 0,03 | 0,05 | 0,06 | 0,08 |
| $\%molC_{PPF}^{i}$ | 0,04 | 0,06 | 0,08 | 0,09 |
| D15 | 0,95 | 0,96 | 0,97 | 0,98 |
| %S | 2,03 | 2,28 | 2,59 | 2,81 |
| %N | 0,18 | 0,21 | 0,25 | 0,29 |
| %H | 11,59 | 11,48 | 11,37 | 11,27 |
| $el_C C$ | 30,171 | 31,605 | 33,019 | 34,495 |
| $el_H C$ | 52,340 | 54,095 | 55,633 | 57,282 |
| $el_S C$ | 0,255 | 0,299 | 0,354 | 0,402 |
| $T_{IBP}$ | 500°C | 520°C | 540°C | 560°C |
| $T_{EBP}$ | 520°C | 540°C | 560°C | 580°C |
| $el_N C$ | 0,054 | 0,067 | 0,080 | 0,095 |
| $Napht_C C$ | 9,953 | 9,966 | 9,887 | 9,849 |
| $Aro_C C$ | 5,537 | 6,297 | 7,200 | 8,092 |
| $CyArom_C C$ | 1,097 | 1,283 | 1,501 | 1,721 |
| $CyNapht_C C$ | 1,973 | 1,982 | 1,973 | 1,973 |
| $CyAro_C C$ | 0,078 | 0,096 | 0,117 | 0,140 |

Dans ce tableau qui caractérise la représentation en coupes étroites d'entrée $RCE_E$ de ce résidu sous vide, les premières lignes correspondent aux vecteurs rendements ( $\%wC^{i}_{PPE}, \%volC^{i}_{PPE}$ et $\%molC^{i}_{PPE}$ ) puis des éléments de la matrice composition (D15, %S, %N et %H), puis des éléments de la matrice structurelle (par exemple $el_CC$ $el_HC$, $el_SC$, $el_NC$, $Napht_CC$, $Aro_CC$, $CyArom_CC$, $CyNapht_CC$, et $CyAro_CC$)

À la représentation en coupe étroite structurale d'entrée $RCSE_E$ est appliqué un modèle réactionnel R (étape 810). L'application du modèle réactionnel R à la représentation en coupe étroite structurale d'entrée $RCSE^{j}_{E}$ , avec l'application d'une fonction de distribution D(Ni, Nj) en coupes étroites structurale de sorties $RCSE^{i}_{S}$ du produit de réaction d'une coupe étroite structurale d'entrée $RCSE^{j}_{E}$ (étape 820), permet de déterminer une représentation en coupes étroites structurales de sortie $RCSE_S$ d'un produit pétrolier intermédiaire PPI de sortie. Le modèle réactionnel R est une représentation des différentes réactions que peut subir le produit pétrolier d'entrée, représenté en coupes étroites structurales d'entrée $RCSE_E$ .

Le modèle réactionnel R est un ensemble interconnecté de réactions cinétiques et se présente sous forme de réseau cinétique $R_K$. Le réseau cinétique $R_K$ simule les réactions cinétiques qui ont lieu au sein d'un réacteur. Ces réactions cinétiques sont la plupart du temps non-linéaires.

En effet, des mesures de réactions cinétiques sont effectuées $\mu_{eff}$ dans un réacteur afin de mieux prendre en compte le déroulement exact des réactions cinétiques et du réseau cinétique $R_K$ dans un réacteur.

Ces mesures $\mu_{eff}$ sont comparées avec des données d'une base de données DB. Ces mesures sont ensuite ajustées et intégrées au modèle réactionnel R, en l'occurrence au réseau cinétique $R_K$.

A titre d'exemple, nous présentons, sur la figure 18, la répartition cumulée en coupes étroites structurale de sortie des produits en fonction de la température d'ébullition réelle TBP reconstituée de l'effluent réacteur pour la réaction d'hydrocraquage d'un résidu sous vide dont la température d'ébullition réelle TBP est supérieur à 500°C.

Par produits en coupes larges reconstituées, pour ne citer que le gas, le kérosène, et gasoil sous vide, le graphique 19 décrit l'évolution des rendements en fonction de l'avancement de la réaction.

Suivant ce deuxième mode de réalisation, les étapes 720 à 740 comprenant le regroupement des coupes étroites d'entrée $RCE_E$ en coupes larges d'entrée $RCL_E$, le modèle réactionnel R sur coupes larges d'entrée $RCL_E$, puis les étapes 750 à 770 comportant la redistribution de l'effluent de réacteur en coupes étroites de sortie $RCE_S$ , le bilan de matière $B_M$ et/ou un bilan élémentaire $B_{ELEM}$ qui correspondent spécifiquement au premier mode de réalisation, sont remplacées par les étapes intégrées 810 de réseau cinétique réactionnel sur coupes étroites et 820 de distribution de l'effluent de réaction des coupes étroites d'entrées en coupes étroites de sorties.

La suite du deuxième mode de réalisation pour les étapes de fractionnement en produits finaux de sortie, et de calcul de composition et de qualité de ces produits finaux, c'est-à-dire les étapes 795 et 799, est identique à celle du premier mode de réalisation.

## Revendications

**1.** Procédé de détermination de la composition d'au moins un produit pétrolier raffiné issu d'un procédé de raffinage comprenant au moins une étape de conversion d'un produit pétrolier d'entrée en un produit pétrolier de sortie dans un réacteur, ledit procédé de détermination comprenant les étapes suivantes :

- obtention d'une représentation en coupes étroites d'entrée *($RCE_E$ )* du produit pétrolier d'entrée ; chaque représentation en coupe étroite d'entrée *($RCE_E$ )* correspondant à un pourcentage volumique $(\%volC^{E}_{PPE})$ et/ou massique $(\%wC^{E}_{PPE})$ et/ou molaire $(\%molC^{E}_{PPE})$ du produit pétrolier d'entrée dont la température d'ébullition (TBP) est comprise dans un intervalle $(I^{E}_{TBP})$ ;
- constitution d'une matrice compositionnelle-structure-qualité comprenant au moins une représentation en coupes étroites structurale d'entrée ($RCSE_E$ ), la matrice compositionnelle-structure-qualité comprend pour chaque coupe étroite structurale d'entrée ($RCSE_E$ ):
  - au moins une valeur caractéristique d'un élément chimique $(el\chi C^{i}_{PPE})$ comprenant au moins un élément

parmi le carbone, l'hydrogène, l'oxygène, le soufre et l'azote ; et/ou

◦ au moins une valeur caractéristique d'une structure chimique $(st\chi C^{i}_{PPE})$ parmi la liste suivante : le cycle naphténique, le cycle aromatique, les chaines alkyles, le nombre de ponts entre cœur naphténique et cœur aromatique, l'indice de condensation, l'indice de branchement, et l'indice d'isomérisation,

ladite valeur caractéristique d'un élément chimique $(el\chi C^{i}_{PPE})$, et la valeur caractéristique d'une structure chimique $(st\chi C^{i}_{PPE})$ se pondérant en moles, suivant la formule suivante correspondant au mélange de plusieurs pétroles bruts *(PPB)* en produit pétrolier d'entrée (*PPE*) :

$$\%molC^{i}_{PPE} = \sum_{PPB=1}^{n} \%molC^{i}_{PPB} * \%molC^{PPB}_{PPE}$$

$$el\chi C^{i}_{PPE} = \frac{\sum_{PPB=1}^{n} \%molC^{i}_{PPB} * \%molC^{PPB}_{PPE} * el\chi C^{i}_{PPB}}{\sum_{PPB=1}^{n} \%molC^{i}_{PPB} * \%molC^{PPB}_{PPE}},$$

et

$$st\chi C^{i}_{PPE} = \frac{\sum_{PPB=1}^{n} \%molC^{i}_{PPB} * \%molC^{PPB}_{PPE} * st\chi C^{i}_{PPB}}{\sum_{PPB=1}^{n} \%molC^{i}_{PPB} * \%molC^{PPB}_{PPE}},$$

avec :

- $el\chi C^{i}_{PPE}$ : Valeur caractéristique d'un élément chimique d'un produit pétrolier d'entrée (PPE) ;
- $el\chi C^{i}_{PPB}$ : Valeur caractéristique de l'élément chimique d'un pétrole brut (PPB) ;
- $st\chi C^{i}_{PPE}$ : Valeur caractéristique de la structure chimique d'un produit pétrolier d'entrée (PPE) ;
- $st\chi C^{i}_{PPB}$ : Valeur caractéristique de la structure chimique d'un pétrole brut (PPB) ;
- $\%molC^{i}_{PPE}$ : pourcentage molaire de la coupe étroite « *i* » d'un produit pétrolier d'entrée
- $\%molC^{i}_{PPB}$ : pourcentage molaire de la coupe étroite « *i* » d'un pétrole brut
- $\%molC^{PPB}_{PPE}$ : pourcentage molaire du pétrole brut (PPB) dans le produit pétrolier d'entrée (PPE),

de sorte à déterminer une pseudo-molécule caractéristique de ladite coupe étroite structurale d'entrée ($RCSE_E$) avec une caractérisation moléculaire se présentant sous une forme de description stœchiométrique $A_{el\alpha C^{i}_{PPE}} B_{el\beta C^{i}_{PPE}} C_{el\gamma C^{i}_{PPE}}$, où $el\alpha C^{i}_{PPE}$, $el\beta C^{i}_{PPE}$, *et* $el\gamma C^{i}_{PPE}$ correspondent respectivement à une valeur caractéristique d'un élément chimique d'un produit pétrolier d'entrée (PPE), et une caractérisation structurale définissant la configuration de la pseudo-molécule caractéristique de la coupe étroite structurale d'entrée ($RCSE_E$).

• détermination d'une représentation en coupes larges d'entrée ($RCL_E$) du produit pétrolier d'entrée à partir de plusieurs représentations en coupes étroites d'entrée ($RCE_E$) ; chaque représentation en coupe large d'entrée ($RCL_E$) correspondant à un pourcentage volumique $\%volC^{L}_{PPE}$ et/ou massique $(\%wC^{L}_{PPE})$ et/ou molaire $(\%molC^{L}_{PPE})$ du produit pétrolier d'entrée dont la température d'ébullition (TBP) est comprise dans un intervalle $(I^{L}_{TBP})$ ; au moins un intervalle de température d'une représentation en coupe large d'entrée $(I^{L}_{TBP})$

comprenant au moins un intervalle de température de représentation en coupes étroites d'entrée $(I_{TBP}^{E})$ ;
• détermination d'une représentation en coupes larges de sortie (*$RCL_S$*) d'un produit pétrolier de sortie par application d'un modèle réactionnel ( *R*) à la représentation en coupes larges d'entrée (*$RCL_E$*) ;
• détermination d'une représentation en coupes étroites de sortie *($RCE_S$*) d'un produit pétrolier de sortie par application d'une fonction de distribution ( *p*(*x*)) à la représentation en coupes larges de sortie (*$RCL_S$*) du produit pétrolier de sortie ;
• détermination, à partir de la représentation en coupes étroites de sortie *($RCE_S$*), d'une matrice compositionnelle-structure-qualité comprenant au moins une représentation en coupes étroites structurale de sortie; la matrice compositionnelle-structure-qualité comportant, pour chaque coupe étroite structurale de sortie:

◦ au moins une valeur caractéristique d'un élément chimique comprenant au moins un élément parmi la liste suivante: carbone, hydrogène, oxygène, soufre, l'azote; et/ou,
◦ au moins une caractérisation structurale parmi la liste suivante: cycle naphténique, cycle aromatique, chaines alkyles, nombre de ponts entre cœur naphténique et cœur aromatique, indice de condensation, indice de branchement, indice d'isomérisation.

**2.** Procédé de détermination de la composition d'au moins un produit pétrolier raffiné issu d'un procédé de raffinage comprenant au moins une étape de conversion d'un produit pétrolier d'entrée en un produit pétrolier de sortie dans un réacteur, ledit procédé de détermination comprenant les étapes suivantes :

• obtention d'une représentation en coupes étroites d'entrée (*$RCE_E$*) du produit pétrolier d'entrée ; chaque représentation en coupe étroite d'entrée (*$RCE_E$*) correspondant à un pourcentage volumique $(\%volC_{PPE}^{E})$ et/ou massique $(\%wC_{PPE}^{E})$ ) et/ou molaire $(\%molC_{PPE}^{E})$ du produit pétrolier d'entrée dont la température d'ébullition (TBP) est comprise dans un intervalle $(I_{TBP}^{E})$ ; et
• obtention d'une représentation en coupe étroite structurale d'entrée (*$RCSE_E$*) de chaque coupe étroite d'entrée (*$RCE_E$*) du produit pétrolier d'entrée ; chaque coupe étroite structurale d'entrée comprenant au moins une valeur caractéristique d'un élément chimique $(el\chi C_{PPE}^{i})$ et/ou une valeur caractéristique d'une structure chimique $(st\chi C_{PPE}^{i})$, ladite valeur caractéristique d'un élément chimique $(el\chi C_{PPE}^{i})$, et la valeur caractéristique d'une structure chimique $(st\chi C_{PPE}^{i})$ se pondérant en moles, suivant la formule suivante correspondant au mélange de plusieurs pétroles bruts *(PPB)* en produit pétrolier d'entrée (*PPE*) :

$$\%molC_{PPE}^{i} = \sum_{PPB=1}^{n} \%molC_{PPB}^{i} * \%molC_{PPE}^{PPB}$$

$$el\chi C_{PPE}^{i} = \frac{\sum_{PPB=1}^{n} \%molC_{PPB}^{i} * \%molC_{PPE}^{PPB} * el\chi C_{PPB}^{i}}{\sum_{PPB=1}^{n} \%molC_{PPB}^{i} * \%molC_{PPE}^{PPB}},$$

et

$$st\chi C_{PPE}^{i} = \frac{\sum_{PPB=1}^{n} \%molC_{PPB}^{i} * \%molC_{PPE}^{PPB} * st\chi C_{PPB}^{i}}{\sum_{PPB=1}^{n} \%molC_{PPB}^{i} * \%molC_{PPE}^{PPB}},$$

avec :

• $el\chi C_{PPE}^{i}$ : Valeur caractéristique d'un élément chimique d'un produit pétrolier d'entrée (PPE) ;

- $el\chi C_{PPB}^{i}$ : Valeur caractéristique de l'élément chimique d'un pétrole brut (PPB) ;
- $st\chi C_{PPE}^{i}$ : Valeur caractéristique de la structure chimique d'un produit pétrolier d'entrée (PPE) ;
- $st\chi C_{PPB}^{i}$ : Valeur caractéristique de la structure chimique d'un pétrole brut (PPB) ;

- $\%molC_{PPE}^{i}$ : pourcentage molaire de la coupe étroite « *i* » d'un produit pétrolier d'entrée
- $\%molC_{PPB}^{i}$ : pourcentage molaire de la coupe étroite « *i* » d'un pétrole brut
- $\%molC_{PPE}^{PPB}$ : pourcentage molaire du pétrole brut (PPB) dans le produit pétrolier d'entrée (PPE), de sorte à déterminer une pseudo-molécule caractéristique de ladite coupe étroite structurale d'entrée ($RCSE_E$) avec une caractérisation moléculaire se présentant sous une forme de description stœchiométrique $A_{el\alpha C_{PPE}^{i}} B_{el\beta C_{PPE}^{i}} C_{el\gamma C_{PPE}^{i}}$, où $el\alpha C_{PPE}^{i}$, $el\beta C_{PPE}^{i}$, *et* $el\gamma C_{PPE}^{i}$ correspondent respectivement à une valeur caractéristique d'un élément chimique d'un produit pétrolier d'entrée (PPE) et une caractérisation structurale définissant la configuration de la pseudo-molécule caractéristique de la coupe étroite structurale d'entrée ($RCSE_E$) ;
- détermination d'au moins une représentation en coupes étroites structurales de sortie ($RCSE_S$) d'un produit pétrolier intermédiaire de sortie par application d'un modèle réactionnel *( R)* à la représentation en coupe étroite structurale d'entrée ($RCSE_E$) de chaque coupe étroite d'entrée ($RCE_E$) du produit pétrolier d'entrée;
- détermination, à partir de la représentation en coupes étroites structurales de sortie ($RCSE_S$) d'une matrice compositionnelle-structure-qualité comprenant au moins une représentation en coupes étroites structurale de sortie; la matrice compositionnelle-structure-qualité comportant, pour chaque coupe étroite structurale de sortie:

  ◦ au moins une valeur caractéristique d'un élément chimique comprenant au moins un élément parmi la liste suivante: carbone, hydrogène, oxygène, soufre, l'azote; et/ou,
  ◦ au moins une caractérisation structurale parmi la liste suivante: cycle naphténique, cycle aromatique, chaines alkyles, nombre de ponts entre cœur naphténique et cœur aromatique, indice de condensation, indice de branchement, indice d'isomérisation.

**3.** Procédé selon la revendication 2, dans lequel la coupe étroite structurale d'entrée comprenant au moins une des propriétés de la coupe étroite d'entrée ($RCE_E$) parmi une propriété physique et une propriété chimique.

**4.** Procédé selon l'une quelconque des revendications 1 à 3, dans lequel le modèle réactionnel (R) comprend un modèle non-linéaire.

**5.** Procédé selon l'une des revendications précédentes, dans lequel le modèle réactionnel (R) comprend un réseau réactionnel ($R_K$) configuré pour déterminer une représentation en coupe large d'entrée ($RCL_E$) en au moins une représentation en coupe large de sortie ($RCL_S$) ou une représentation en coupe étroite structurale d'entrée ($RCSE_E$) en au moins une représentation en coupe étroite structurale de sortie ($RCSE_S$).

**6.** Procédé selon l'une des revendications précédentes, dans lequel le réseau réactionnel ($R_K$) intègre la décomposition cinétique des représentations en coupes larges d'entrée ($RCL_E$) sur leurs temps de séjour ( $t_s^{RCL_E}$ ) et sur leurs températures de réaction ( $T°_{RCL_E}$ ) dans le réacteur ou la décomposition cinétique des représentations en coupes étroite structurale d'entrée ($RCSE_E$) sur leurs temps de séjour ( $t_s^{RCSE_E}$ ) et sur leurs températures de réaction ( $T°_{RCSE_E}$) dans le réacteur.

**7.** Procédé selon l'une des revendications précédentes, dans lequel un bilan élémentaire ( $B_{ELEM}$) entre la représentation en coupes étroites d'entrée ($RCE_E$) et la représentation en coupes étroites de sortie ($RCE_S$) est réalisé.

**8.** Procédé selon l'une des revendications précédentes, dans lequel la fonction de distribution (p(x)) comprend une loi gamma ($\Gamma(x)$).

**9.** Procédé selon l'une des revendications précédentes, dans lequel une détermination d'une représentation en coupes étroites de sortie corrigées $(RCE_S^*)$ comprend une étape d'intégration des produits azotés ($P_N$) et/ou des produits soufrés ($P_S$) dans les représentations en coupes étroites de sortie *($RCE_S$* ).

**10.** Procédé selon l'une des revendications précédentes, dans lequel le modèle réactionnel ( *R*) comprend un réseau réactionnel $(R_k^{SN})$ configuré pour intégrer la décomposition cinétique des produits azotés ($P_N$) et/ou des produits soufrés ($P_S$) sur le temps de séjour ( $t_S$) et sur la température de réaction (T°) dans le réacteur.

**11.** Procédé selon l'une des revendications 9 ou 10, dans lequel on détermine une représentation de coupe de produit fractionné *(RCF)* correspondant à l'application d'un point de coupe aux représentations en coupes étroites de sortie corrigées (ou aux représentations en coupes étroites structurales de sortie *($RCSE_S$* ).

**12.** Procédé selon l'une des revendications 9 à 11, comprenant au moins une étape de fractionnement dans lequel la représentation en coupes étroites de sortie corrigées (ou la représentation en coupe étroite structurale de sortie *($RCSE_S$* ) est fractionnée en représentation en coupes fractionnées *(RCF)* en fonction d'un intervalle de température ($f(I_F)$).

**13.** Procédé selon l'une des revendications 9 à 12, dans lequel on détermine la répartition des représentations en coupes étroites de sortie corrigées (ou la représentation en coupe étroite structurale de sortie *($RCSE_S$* ) en représentations en coupes fractionnées (*RCF* ) par une fonction de répartition (FR) avec indice de fractionnement *(FI)*

**14.** Procédé selon l'une des revendications précédentes, dans lequel la représentation en coupes étroites d'entrée ($RCE_E$ ) du produit pétrolier d'entrée est obtenue à partir de donnée d'essai en laboratoire par application d'une fonction de fonction de distribution (p(x)).

**15.** Produit programme d'ordinateur comprenant des instructions de code agencées pour mettre en œuvre les étapes d'un procédé de détermination de la composition d'au moins un produit pétrolier raffiné issu d'un procédé de raffinage selon l'une des revendications précédentes, lorsque ledit programme est exécuté sur un processeur d'une unité de contrôle.

## Patentansprüche

**1.** Verfahren zur Bestimmung der Zusammensetzung von mindestens einem raffinierten Erdölprodukt, das aus einem Raffinationsverfahren hervorgeht, umfassend mindestens einen Schritt eines Umwandelns eines Eingangserdölprodukts in ein Ausgangserdölprodukt in einem Reaktor, wobei das Bestimmungsverfahren die folgenden Schritte umfasst:

- Erlangen einer Darstellung des Eingangserdölprodukts in engen Eingangsfraktionen ($RCE_E$); wobei jede Darstellung in einer engen Eingangsfraktion *(RCEE)* einem Volumen- $( \%volC_{PPE}^{E} )$ und/oder Massen- $( \%wC_{PPE}^{E} )$ und/oder Molprozentanteil $( \%molC_{PPE}^{E} )$ des Eingangserdölprodukts entspricht, dessen Siedetemperatur (TBP) von einem Intervall $( I_{TBP}^{E} )$ umfasst ist;
- Bilden einer Zusammensetzungs-Struktur-Qualitätsmatrix, umfassend mindestens eine Darstellung in engen strukturellen Eingangsfraktionen ($RCSE_E$), wobei die Zusammensetzungs-Struktur-Qualitätsmatrix für jede enge strukturelle Eingangsfraktion ($RCSE_E$) umfasst:
  - mindestens einen charakteristischen Wert eines chemischen Elements $( elXC_{PPE}^{i} )$ , umfassend mindestens ein Element aus Kohlenstoff, Wasserstoff, Sauerstoff, Schwefel und Stickstoff; und/oder
  - mindestens einen charakteristischen Wert einer chemischen Struktur $( stXC_{PPE}^{i} )$ aus der folgenden Liste: Naphthenring, aromatischer Ring, Alkylketten, Anzahl von Brücken zwischen Naphthenkern und aromatischem Kern, Kondensationsindex, Verzweigungsindex und Isomerisierungsindex,

wobei der charakteristische Wert eines chemischen Elements ($elXC^{i}_{PPE}$) und der charakteristische Wert einer chemischen Struktur ($stXC^{i}_{PPE}$) in Mol gemäß der folgenden Formel gewichtet sind, die dem Gemisch von mehreren Rohölen (*PPB*) in einem Eingangserdölprodukt *(PPE)* entspricht:

$$\%molC^{i}_{PPE} = \sum_{PPB=1}^{n} \%molC^{i}_{PPB} * \%molC^{PPB}_{PPE}$$

$$elXC^{i}_{PPE} = \frac{\sum_{PPB=1}^{n} \%molC^{i}_{PPB} * \%molC^{PPB}_{PPE} * elXC^{i}_{PPB}}{\sum_{PPB=1}^{n} \%molC^{i}_{PPB} * \%molC^{PPB}_{PPE}}$$

und

$$stXC^{i}_{PPE} = \frac{\sum_{PPB=1}^{n} \%molC^{i}_{PPB} * \%molC^{PPB}_{PPE} * stXC^{i}_{PPB}}{\sum_{PPB=1}^{n} \%molC^{i}_{PPB} * \%molC^{PPB}_{PPE}},$$

wobei:

- $elXC^{i}_{PPE}$ = charakteristischer Wert eines chemischen Elements eines Eingangserdölprodukts (PPE);
- $elXC^{i}_{PPB}$ = charakteristischer Wert eines chemischen Elements eines Rohöls (PPB);
- $stXC^{i}_{PPE}$ = charakteristischer Wert der chemischen Struktur eines Eingangserdölprodukts (PPE);
- $stXC^{i}_{PPB}$ = charakteristischer Wert der chemischen Struktur eines Rohöls (PPB);
- $\%molC^{i}_{PPE}$ = Molprozentanteil der engen Fraktion "*i*" eines Eingangserdölprodukts
- $\%molC^{i}_{PPB}$ = Molprozentanteil der engen Fraktion "*i*" eines Rohöls
- $\%molC^{PPB}_{PPE}$ = Molprozentanteil des Rohöls (PPB) in dem Eingangserdölprodukt (PPE),

um ein charakteristisches Pseudomolekül der engen strukturellen Eingangsfraktion (*RCSE$_E$*) mit einer Molekülcharakterisierung, die sich in einer stöchiometrischen Beschreibungsform $A_{el\alpha C^{i}_{PPE}} B_{el\beta C^{i}_{PPE}} C_{el\gamma C^{i}_{PPE}}$ oder $el\alpha C^{i}_{PPE}$, $el\beta C^{i}_{PPE}$ und $el\gamma C^{i}_{PPE}$ präsentiert, die jeweils einem charakteristischen Wert eines chemischen Elements eines Eingangserdölprodukts (PPE) entspricht, und einer Strukturcharakterisierung, die die Konfiguration des charakteristischen Pseudomoleküls der engen strukturellen Eingangsfraktion (*RCSE$_E$*) definiert, zu bestimmen;

- Bestimmen einer Darstellung des Eingangserdölprodukts in breiten Eingangsfraktionen (*RCL$_E$*) aus mehreren Darstellungen in engen Eingangsfraktionen (*RCE$_E$*); wobei jede Darstellung in einer breiten Eingangsfraktion (*RCL$_E$*) einem Volumen- ($\%volC^{L}_{PPE}$) und/oder Massen- ($\%wC^{L}_{PPE}$) und/oder Molprozentanteil ($\%molC^{L}_{PPE}$) des Eingangserdölprodukts entspricht, dessen Siedetemperatur (TBP) von einem Intervall ($I^{L}_{TBP}$) umfasst ist; wobei mindestens ein Temperaturintervall einer Darstellung in einer breiten Eingangsfraktion ($I^{L}_{TBP}$) mindestens ein Temperaturintervall der Darstellung in engen Eingangsfraktionen ($I^{E}_{TBP}$) umfasst;
- Bestimmen einer Darstellung eines Ausgangserdölprodukts in breiten Ausgangsfraktionen *(RCL$_S$)* durch Anwendung eines Reaktionsmodells (R) auf die Darstellung in breiten Eingangsfraktionen *(RCL$_E$);*
- Bestimmen einer Darstellung eines Ausgangserdölprodukts in engen Ausgangsfraktionen *(RCEs)* durch Anwendung einer Verteilungsfunktion (*p*(*x*)) auf die Darstellung des Ausgangserdölprodukts in breiten Ausgangsfraktionen (*RCLs*);

• Bestimmen einer Zusammensetzungs-Struktur-Qualitätsmatrix aus der Darstellung in engen Ausgangsfraktionen *(RCEs),* umfassend mindestens eine Darstellung in engen strukturellen Ausgangsfraktionen; wobei die Zusammensetzungs-Struktur-Qualitätsmatrix für jede enge strukturelle Ausgangsfraktion umfasst:

◦ mindestens einen charakteristischen Wert eines chemischen Elements, umfassend mindestens ein Element aus der folgenden Liste: Kohlenstoff, Wasserstoff, Sauerstoff, Schwefel, Stickstoff; und/oder
◦ mindestens eine Strukturcharakterisierung aus der folgenden Liste: Naphthenring, aromatischer Ring, Alkylketten, Anzahl von Brücken zwischen Naphthenkern und aromatischem Kern, Kondensationsindex, Verzweigungsindex, Isomerisierungsindex.

**2.** Verfahren zur Bestimmung der Zusammensetzung von mindestens einem raffinierten Erdölprodukt, das aus einem Raffinationsverfahren hervorgeht, umfassend mindestens einen Schritt eines Umwandelns eines Eingangserdölprodukts in ein Ausgangserdölprodukt in einem Reaktor, wobei das Bestimmungsverfahren die folgenden Schritte umfasst:

• Erlangen einer Darstellung des Eingangserdölprodukts in engen Eingangsfraktionen (*RCE$_E$*); wobei jede Darstellung in einer engen Eingangsfraktion (*RCE$_E$*) einem Volumen- ( $\%volC_{PPE}^{E}$ ) und/oder Massen- ( $\%wC_{PPE}^{E}$ ) und/oder Molprozentanteil ( $\%molC_{PPE}^{E}$ ) des Eingangserdölprodukts entspricht, dessen Siedetemperatur (TBP) von einem Intervall ( $I_{TBP}^{E}$ ) umfasst ist; und

• Erlangen einer Darstellung des Eingangserdölprodukts in einer engen strukturellen Eingangsfraktion (*RCSE$_E$*) jeder engen Eingangsfraktion (*RCE$_E$*); wobei jede enge strukturelle Eingangsfraktion mindestens einen charakteristischen Wert eines chemischen Elements ( $elXC_{PPE}^{i}$ ) und/oder einen charakteristischen Wert einer chemischen Struktur ( $stXC_{PPE}^{i}$ ) umfasst, wobei der charakteristische Wert eines chemischen Elements ( $elXC_{PPE}^{i}$ ) und der charakteristische Wert einer chemischen Struktur ( $stXC_{PPE}^{i}$ ) in Mol gemäß der folgenden Formel gewichtet sind, die dem Gemisch von mehreren Rohölen (*PPB*) in einem Eingangserdölprodukt (PPE) entspricht:

$$\%molC_{PPE}^{i} = \sum_{PPB=1}^{n} \%molC_{PPB}^{i} * \%molC_{PPE}^{PPB}$$

und

$$elXC_{PPE}^{i} = \frac{\sum_{PPB=1}^{n} \%molC_{PPB}^{i} * \%molC_{PPE}^{PPB} * elXC_{PPB}^{i}}{\sum_{PPB=1}^{n} \%molC_{PPB}^{i} * \%molC_{PPE}^{PPB}}$$

$$stXC_{PPE}^{i} = \frac{\sum_{PPB=1}^{n} \%molC_{PPB}^{i} * \%molC_{PPE}^{PPB} * stXC_{PPB}^{i}}{\sum_{PPB=1}^{n} \%molC_{PPB}^{i} * \%molC_{PPE}^{PPB}},$$

wobei:

- $elXC_{PPE}^{i}$ = charakteristischer Wert eines chemischen Elements eines Eingangserdölprodukts (PPE);
- $elXC_{PPB}^{i}$ = charakteristischer Wert eines chemischen Elements eines Rohöls (PPB);
- $stXC_{PPE}^{i}$ = charakteristischer Wert der chemischen Struktur eines Eingangserdölprodukts (PPE);

- $stXC^{i}_{PPB}$ = charakteristischer Wert der chemischen Struktur eines Rohöls (PPB);
- $\%molC^{i}_{PPE}$ = Molprozentanteil der engen Fraktion "*i*" eines Eingangserdölprodukts
- $\%molC^{i}_{PPB}$ = Molprozentanteil der engen Fraktion "*i*" eines Rohöls
- $\%molC^{PPB}_{PPE}$ = Molprozentanteil des Rohöls (PPB) in dem Eingangserdölprodukt (PPE),

um ein charakteristisches Pseudomolekül der engen strukturellen Eingangsfraktion (*$RCSE_E$*) mit einer Molekülcharakterisierung, die sich in einer stöchiometrischen Beschreibungsform $A_{el\alpha C^{i}_{PPE}}B_{el\beta C^{i}_{PPE}}C_{el\gamma C^{i}_{PPE}}$ oder $el\alpha C^{i}_{PPE}$, $el\beta C^{i}_{PPE}$ und $el\gamma C^{i}_{PPE}$ präsentiert, die jeweils einem charakteristischen Wert eines chemischen Elements eines Eingangserdölprodukts (PPE) entspricht, und einer Strukturcharakterisierung, die die Konfiguration des charakteristischen Pseudomoleküls der engen strukturellen Eingangsfraktion (*$RCSE_E$*) definiert, zu bestimmen;

• Bestimmen mindestens einer Darstellung eines Zwischenausgangserdölprodukts in engen strukturellen Ausgangsfraktionen *($RCSE_S$)* durch Anwendung eines Reaktionsmodells (R) auf die Darstellung des Eingangserdölprodukts in einer engen strukturellen Eingangsfraktion (*$RCSE_E$*) jeder engen Eingangsfraktion (*$RCE_E$*);

• Bestimmen einer Zusammensetzungs-Struktur-Qualitätsmatrix aus der Darstellung in engen strukturellen Ausgangsfraktionen *(RCSEs),* umfassend mindestens eine Darstellung in engen strukturellen Ausgangsfraktionen; wobei die Zusammensetzungs-Struktur-Qualitätsmatrix für jede enge strukturelle Ausgangsfraktion umfasst:

 ◦ mindestens einen charakteristischen Wert eines chemischen Elements, umfassend mindestens ein Element aus der folgenden Liste: Kohlenstoff, Wasserstoff, Sauerstoff, Schwefel, Stickstoff; und/oder
 ◦ mindestens eine Strukturcharakterisierung aus der folgenden Liste: Naphthenring, aromatischer Ring, Alkylketten, Anzahl von Brücken zwischen Naphthenkern und aromatischem Kern, Kondensationsindex, Verzweigungsindex, Isomerisierungsindex.

**3.** Verfahren nach Anspruch 2, wobei die enge strukturelle Eingangsfraktion mindestens eine der Eigenschaften der engen Eingangsfraktion (*$RCE_E$*) aus einer physikalischen Eigenschaft und einer chemischen Eigenschaft umfasst.

**4.** Verfahren nach einem der Ansprüche 1 bis 3, wobei das Reaktionsmodell (*R*) ein nichtlineares Modell umfasst.

**5.** Verfahren nach einem der vorhergehenden Ansprüche, wobei das Reaktionsmodell (*R*) ein Reaktionsnetzwerk ($R_K$) umfasst, das zum Bestimmen einer Darstellung in einer breiten Eingangsfraktion (*$RCL_E$*) in mindestens einer Darstellung in einer breiten Ausgangsfraktion *($RCL_S$)* oder einer Darstellung in einer engen Eingangsfraktion (*$RCSE_E$*) in mindestens einer Darstellung in einer engen Ausgangsfraktion ($RCSE_S$) konfiguriert ist.

**6.** Verfahren nach einem der vorhergehenden Ansprüche, wobei das Reaktionsnetzwerk ($R_K$) den kinetischen Abbau von Darstellungen in breiten Eingangsfraktionen (*$RCL_E$*) bei ihren Haltezeiten ( $t_s^{RCL_E}$ ) und ihren Reaktionstemperaturen ($T^o_{RCL_E}$) in dem Reaktor oder den kinetischen Abbau von Darstellungen in engen strukturellen Eingangsfraktionen (*$RCSE_E$*) bei ihren Haltezeiten ($t_s^{RCSE_E}$) und ihren Reaktionstemperaturen ($T^o_{RCSE_E}$) in dem Reaktor integriert.

**7.** Verfahren nach einem der vorhergehenden Ansprüche, wobei eine elementare Bilanz (*$B_{ELEM}$*) zwischen der Darstellung in engen Eingangsfraktionen (*$RCE_E$*) und der Darstellung in engen Ausgangsfraktionen *(RCEs)* erstellt wird.

**8.** Verfahren nach einem der vorhergehenden Ansprüche, wobei die Verteilungsfunktion (*p(x)*) eine Gamma-Verteilung (*Γ(x)*) umfasst.

**9.** Verfahren nach einem der vorhergehenden Ansprüche, wobei eine Bestimmung einer Darstellung in korrigierten engen Ausgangsfraktionen ($RCE_S$*) einen Schritt eines Integrierens von Stickstoffprodukten ($P_N$) und/oder Schwefelprodukten (Ps) in den Darstellungen in engen Ausgangsfraktionen *(RCEs)* umfasst.

**10.** Verfahren nach einem der vorhergehenden Ansprüche, wobei das Reaktionsmodell (R) ein Reaktionsnetzwerk ($R_k^{SN}$) umfasst, das zum Integrieren des kinetischen Abbaus von Stickstoffprodukten ($P_N$) und/oder Schwefelprodukten (Ps) bei der Haltezeit (ts) und der Reaktionstemperatur (*T*°) in dem Reaktor konfiguriert ist.

**11.** Verfahren nach einem der Ansprüche 9 oder 10, wobei eine Darstellung einer Fraktion eines fraktionierten Produkts *(RCF)* bestimmt wird, das der Anwendung eines Fraktionspunkts auf die Darstellungen in korrigierten engen Ausgangsfraktionen ($RCE_S$*) oder auf die Darstellungen in engen strukturellen Ausgangsfraktionen (RCSEs) entspricht.

**12.** Verfahren nach einem der Ansprüche 9 bis 11, umfassend mindestens einen Schritt eines Fraktionierens, wobei die Darstellung in korrigierten engen Ausgangsfraktionen ($RCE_S$*) oder die Darstellung in einer engen strukturellen Ausgangsfraktion (*RCSEs*) in einer Darstellung in fraktionierten Fraktionen (*RCF*) in Abhängigkeit von einem Temperaturintervall ($f(I_F)$) fraktioniert wird.

**13.** Verfahren nach einem der Ansprüche 9 bis 12, wobei die Aufteilung von Darstellungen in korrigierten engen Ausgangsfraktionen ($RCE_S$*) oder der Darstellung in einer engen strukturellen Ausgangsfraktion (*RCSEs*) in Darstellungen in fraktionierten Fraktionen (RCF) durch eine Aufteilungsfunktion (*FR*) mit einem Fraktionierungsindex (*FI*) bestimmt wird.

**14.** Verfahren nach einem der vorhergehenden Ansprüche, wobei die Darstellung des Eingangserdölprodukts in engen Eingangsfraktionen ($RCE_E$) aus Labortestdaten durch Anwendung einer Funktion einer Verteilungsfunktion (*p(x)*) erlangt wird.

**15.** Computerprogrammprodukt, umfassend Codeanweisungen, die zum Durchführen der Schritte eines Verfahrens zur Bestimmung der Zusammensetzung von mindestens einem raffinierten Erdölprodukt, das aus einem Raffinationsverfahren hervorgeht, nach einem der vorhergehenden Ansprüche eingerichtet sind, wenn das Programm auf einem Prozessor einer Steuereinheit ausgeführt wird.

## Claims

**1.** A method for determining the composition of at least one refined petroleum product derived from a refining process comprising at least one step of converting an input petroleum product into an output petroleum product in a reactor, said determination method comprising the following steps:

- obtainment of a representation in narrow input sections ($RCE_E$) of the input petroleum product; each representation in narrow input sections ($RCE_E$) corresponding to a volume $(\%volC_{PPE}^E)$ and/or mass $(\%wC_{PPE}^E)$ and/or molar $(\%molC_{PPE}^E)$ percentage of the input petroleum product whose boiling point (TBP) is comprised within an interval $(I_{TBP}^E)$;
- constitution of a composition-structure-quality matrix comprising at least one representation in structural narrow input sections ($RCSE_E$), the composition-structure-quality matrix comprises for each structural narrow input section ($RCSE_E$):
  - ◦ at least one characteristic value of a chemical element $(el_\chi C_{PPE}^i)$ comprising at least one element amongst carbon, hydrogen, oxygen, sulfur and nitrogen; and/or
  - ◦ at least one characteristic value of a chemical structure $(st_\chi C_{PPE}^i)$ from the following list: naphthenic cycle, aromatic cycle, alkyl chains, the number of bridges between the naphthenic core and the aromatic core, the condensation index, the branching index, and the isomerization index,

said characteristic value of a chemical element $(el_\chi C^i_{PPE})$, and the characteristic value of a chemical structure $(st_\chi C^i_{PPE})$ being weighted in moles, according to the following formula corresponding to the mixture of several crude oils *(PPB)* into an input petroleum product *(PPE):*

$$\%molC^i_{PPE} = \sum_{PPB=1}^{n} \%molC^i_{PPB} * \%molC^{PPB}_{PPE}$$

$$el_\chi C^i_{PPE} = \frac{\sum_{PPB=1}^{n} \%molC^i_{PPB} * \%molC^{PPB}_{PPE} * el_\chi C^i_{PPB}}{\sum_{PPB=1}^{n} \%molC^i_{PPB} * \%molC^{PPB}_{PPE}};$$

and

$$st_\chi C^i_{PPE} = \frac{\sum_{PPB=1}^{n} \%molC^i_{PPB} * \%molC^{PPB}_{PPE} * st_\chi C^i_{PPB}}{\sum_{PPB=1}^{n} \%molC^i_{PPB} * \%molC^{PPB}_{PPE}},$$

with:

- $el_\chi C^i_{PPE}$ : Characteristic value of a chemical element of an input petroleum product (PPE);
- $el_\chi C^i_{PPB}$ : Characteristic value of the chemical element of a crude oil (PPB);
- $st_\chi C^i_{PPE}$ : Characteristic value of the chemical structure of an input petroleum product (PPE);
- $st_\chi C^i_{PPB}$ : Characteristic value of the chemical structure of a crude oil (PPB);
- $\%molC^i_{PPE}$ : molar percentage of the narrow section « i » of an input petroleum product;
- $\%molC^i_{PPB}$ : molar percentage of the narrow section « i » of a crude oil;
- $\%molC^{PPB}_{PPE}$ : molar percentage of the crude oil (PPB) in the input petroleum product (PPE),

so as to determine a characteristic pseudo-molecule of said structural narrow input section (*$RCSE_E$*) with a molecular characterization in a stoichiometric description form $A_{el\alpha C^i_{PPE}}$ $B_{el\beta C^i_{PPE}}$ $C_{el\gamma C^i_{PPE}}$, where $el\alpha C^i_{PPE}$, $el\beta C^i_{PPE}$, and $el\gamma C^i_{PPE}$ respectively correspond to a characteristic value of a chemical element of an input petroleum product (PPE), and a structural characterization defining the configuration of the characteristic pseudo-molecule of the structural narrow input section (*$RCSE_E$*);

• determination of a representation in wide input sections (*$RCL_E$*) of the input petroleum product from several representations in narrow input sections (*$RCE_E$*); each representation in wide input sections (*$RCL_E$*) corresponding to a volume $(\%volC^L_{PPE})$ and/or mass $(\%wC^L_{PPE})$ and/or molar $(\%molC^L_{PPE})$ percentage of the input petroleum product whose boiling point (TBP) is comprised within an interval $(I^L_{TBP})$; at least one temperature interval of a representation in wide input sections $(I^L_{TBP})$ comprising at least one temperature interval of a representation in narrow input sections $(I^E_{TBP})$;

• determination of a representation in wide output sections *($RCL_S$)* of an output petroleum product by application of a reaction model (R) to the representation in wide input sections (*$RCL_E$*);

• determination of a representation in narrow output sections (*$RCE_S$*) of an output petroleum product by application of a distribution function (*p(x)*) to the representation in wide output sections *($RCL_S$)* of the output petroleum product;

• determination, from the representation in narrow output sections ($RCE_S$), of a composition-structure-quality matrix comprising at least one representation in structural narrow output sections; the composition-structure-quality matrix including, for each structural narrow output section:

◦ at least one characteristic value of a chemical element comprising at least one element from the following list: carbon, hydrogen, oxygen, sulfur, nitrogen; and/or,
◦ at least one structural characterization from the following list: naphthenic cycle, aromatic cycle, alkyl chains, number of bridges between the naphthenic core and the aromatic core, condensation index, branching index, isomerization index.

**2.** A method for determining the composition of at least one refined petroleum product derived from a refining process comprising at least one step of converting an input petroleum product into an output petroleum product in a reactor, said determination method comprising the following steps:

• obtainment of a representation in narrow input sections ($RCE_E$) of the input petroleum product; each representation in narrow input sections ($RCE_E$) corresponding to a volume $(\%volC_{PPE}^{E})$ and/or mass $(\%wC_{PPE}^{E})$ and/or molar $(\%molC_{PPE}^{E})$ percentage of the input petroleum product whose boiling point (TBP) is comprised within an interval $(I_{TBP}^{E})$ and
• obtainment of a representation in structural narrow input sections ($RCSE_E$) of each narrow input section ($RCE_E$) of the input petroleum product; each structural narrow input section comprising at least one characteristic value of a chemical element $(el_{\chi}C_{PPE}^{i})$ and/or at least one characteristic value of a chemical structure $(st_{\chi}C_{PPE}^{i})$, said characteristic value of a chemical element $(el_{\chi}C_{PPE}^{i})$, and the characteristic value of a chemical structure $(st_{\chi}C_{PPE}^{i})$ being weighted in moles, according to the following formula corresponding to the mixture of several crude oils *(PPB)* into an input petroleum product *(PPE):*

$$\%molC_{PPE}^{i} = \sum_{PPB=1}^{n} \%molC_{PPB}^{i} * \%molC_{PPE}^{PPB}$$

$$el_{\chi}C_{PPE}^{i} = \frac{\sum_{PPB=1}^{n} \%molC_{PPB}^{i} * \%molC_{PPE}^{PPB} * el_{\chi}C_{PPB}^{i}}{\sum_{PPB=1}^{n} \%molC_{PPB}^{i} * \%molC_{PPE}^{PPB}},$$

and

$$st_{\chi}C_{PPE}^{i} = \frac{\sum_{PPB=1}^{n} \%molC_{PPB}^{i} * \%molC_{PPE}^{PPB} * st_{\chi}C_{PPB}^{i}}{\sum_{PPB=1}^{n} \%molC_{PPB}^{i} * \%molC_{PPE}^{PPB}},$$

with:

- $el_{\chi}C_{PPE}^{i}$ : Characteristic value of a chemical element of an input petroleum product (PPE);
- $el_{\chi}C_{PPB}^{i}$ : Characteristic value of the chemical element of a crude oil (PPB);
- $st_{\chi}C_{PPE}^{i}$ : Characteristic value of the chemical structure of an input petroleum product (PPE);
- $st_{\chi}C_{PPB}^{i}$ : Characteristic value of the chemical structure of a crude oil (PPB);

• $\%molC_{PPE}^{i}$ : molar percentage of the narrow section « i » of an input petroleum product;

- $\%molC_{PPB}^{i}$ : molar percentage of the narrow section « i » of a crude oil;
  - $\%molC_{PPE}^{PPB}$ : molar percentage of the crude oil (PPB) in the input petroleum product (PPE),

so as to determine a characteristic pseudo-molecule of said structural narrow input section ($RCSE_E$) with a molecular characterization in a stoichiometric description form $A_{el\alpha C_{PPE}^{i}} B_{el\beta C_{PPE}^{i}} C_{el\gamma C_{PPE}^{i}}$ , where $el\alpha C_{PPE}^{i}$ , $el\beta C_{PPE}^{i}$ , and $el\gamma C_{PPE}^{i}$ respectively correspond to a characteristic value of a chemical element of an input petroleum product (PPE), and a structural characterization defining the configuration of the characteristic pseudo-molecule of the structural narrow input section ($RCSE_E$);

• determination of at least one representation in structural narrow output sections ($RCSE_S$) of an intermediate petroleum product by application of a reaction model (R) to the representation in structural narrow input sections ($RCSE_E$) of each narrow input section ($RCE_E$) of the input petroleum product;

• determination, from the representation in structural narrow output sections ($RCSE_S$) of a composition-structure-quality matrix comprising at least one representation in structural narrow output sections; the composition-structure-quality matrix including, for each structural narrow output section:

◦ at least one characteristic value of a chemical element comprising at least one element from the following list: carbon, hydrogen, oxygen, sulfur, nitrogen; and/or,

◦ at least one structural characterization from the following list: naphthenic cycle, aromatic cycle, alkyl chains, number of bridges between the naphthenic core and the aromatic core, condensation index, branching index, isomerization index.

**3.** The method according to claim 2, wherein the structural narrow input section comprising at least one of the properties of the narrow input section ($RCE_E$) amongst a physical property and a chemical property.

**4.** The method according to any one of claims 1 to 3, wherein the reaction model (*R*) comprises a nonlinear model.

**5.** The method according to any of the preceding claims, wherein the reaction model (*R*) comprises a reaction network ($R_K$) configured to determine a representation in wide input sections ($RCL_E$) in at least one representation in wide output sections ($RCL_S$) or a representation in structural narrow input sections ($RCSE_E$) in at least one representation in structural narrow output sections ($RCSE_S$).

**6.** The method according to any of the preceding claims, wherein the reaction network ($R_K$) integrates the kinetic decompositions of the representations in wide input sections ($RCL_E$) over their stay-times $(t_s^{RCL_E})$ and over their reaction temperatures ($T°_{RCL_E}$) in the reactor or the kinetic decomposition of the representations in structural narrow input sections ($RCSE_E$) over their stay-times $(t_s^{RCSE_E})$ and over their reaction temperatures ($T°_{RCSEE}$) in the reactor.

**7.** The method according to any of the preceding claims, wherein an elementary balance ($B_{ELEM}$) between the representation in narrow input sections ($RCE_E$) and the representation in narrow output sections ($RCE_S$) is performed.

**8.** The method according to any of the preceding claims, wherein the distribution function (*p(x)*) comprises a gamma law ($\Gamma(x)$).

**9.** The method according to any of the preceding claims, wherein a determination of a representation in corrected narrow output sections $(RCE_S^*)$ comprises a step of integrating the nitrogenous products ($P_N$) and/or the sulfurous products ($P_S$) in the representations in narrow output sections ($RCE_S$).

**10.** The method according to any of the preceding claims, wherein the reaction model (R) comprises a reaction network $(R_k^{SN})$ configured to integrate the kinetic decomposition of the nitrogenous products ($P_N$) and/or the sulfurous

products ($P_S$) over the stay-time ($t_s$) and over the reaction temperature (T°) in the reactor.

**11.** The method according to any of claims 9 or 10, wherein a representation in fractionated product sections *(RCF),* corresponding to the application of a cutting point to the representations in corrected narrow output sections $(RCE_S^*)$ or to the representations in structural narrow output sections ($RCSE_S$), is determined.

**12.** The method according to any of claims 9 to 11, comprising at least one fractionation step wherein the representation in corrected narrow output sections $(RCE_S^*)$ or the representation in structural narrow output sections ($RCSE_S$) is fractionated into a representation in fractionated sections *(RCF)* according to a temperature interval ($f(I_F)$).

**13.** The method according to any of claims 9 to 12, wherein the cumulative distribution of the representations in corrected narrow output sections $(RCE_S^*)$ or the representation in structural narrow output sections ($RCSE_S$) in representations in fractionated sections *(RCF)* is determined by a cumulative distribution function (FR) with the fractionation index ($FI$).

**14.** The method according to any of the preceding claims, wherein the representation in narrow input sections ($RCE_E$) of the input petroleum product is obtained from laboratory test data by application of a distribution function ($p(x)$).

**15.** A computer program product comprising code instructions arranged so as to implement the steps of a method for determining the composition of at least one refined petroleum product derived from a refining process according to any of the preceding claims, when said program is executed on a processor of a control unit.

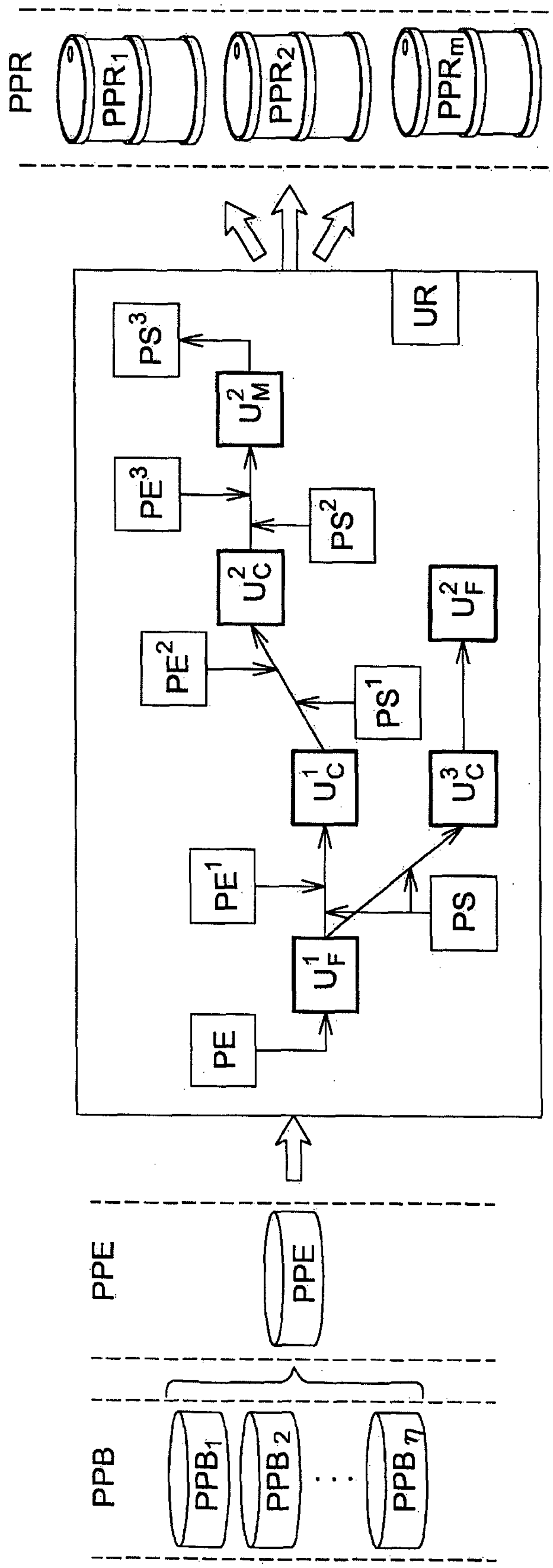
PPR
$PPR_1$
$PPR_2$
$PPR_m$
UR
$PS^3$
$U^2_M$
$PE^3$
$PS^2$
$U^2_C$
$PE^2$
$PS^1$
$U^1_C$
$U^2_F$
$U^3_C$
$PE^1$
PS
$U^1_F$
PE
PPE
PPE
PPB
$PPB_1$
$PPB_2$
$PPB_\eta$

Fig. 1

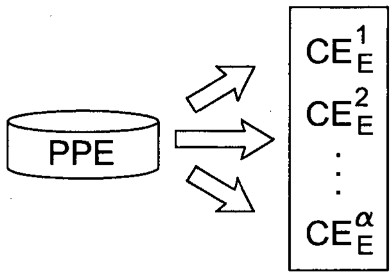


**Fig. 2**

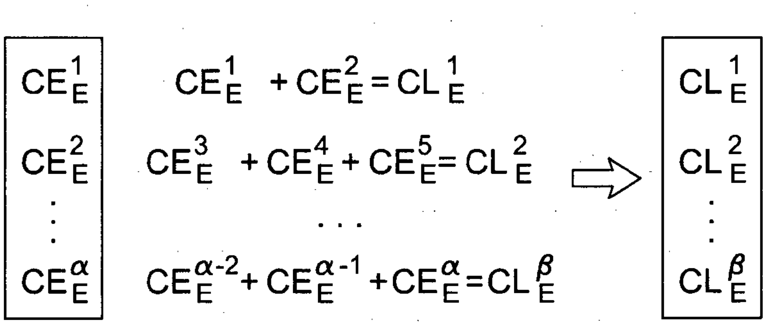


**Fig. 3**

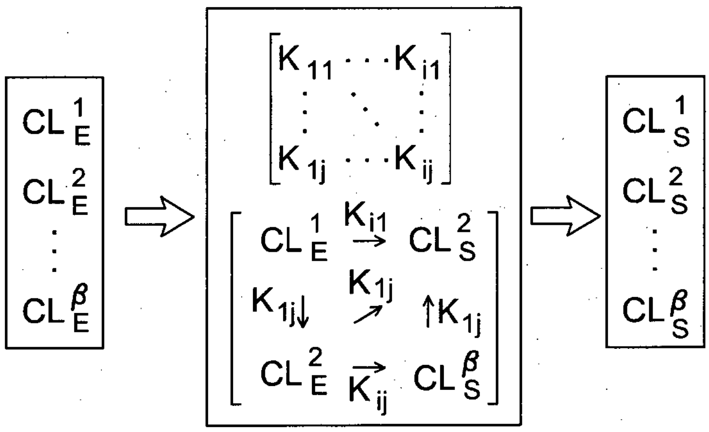


**Fig. 4**

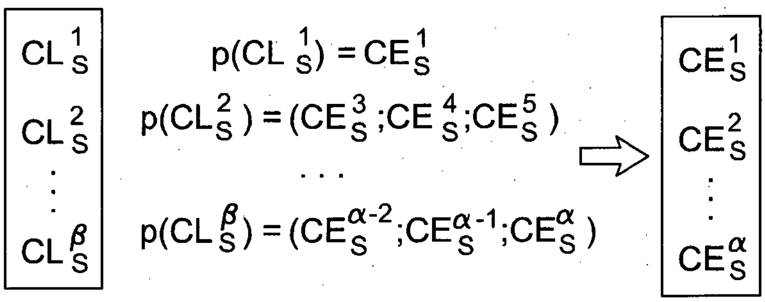


**Fig. 5**

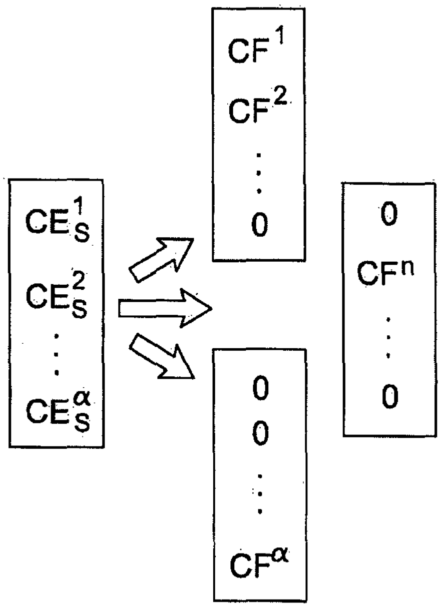
$CE_S^1$
$CE_S^2$
$CE_S^\alpha$
$CF^1$
$CF^2$
0
0
$CF^n$
0
0
0
$CF^\alpha$

Fig. 6

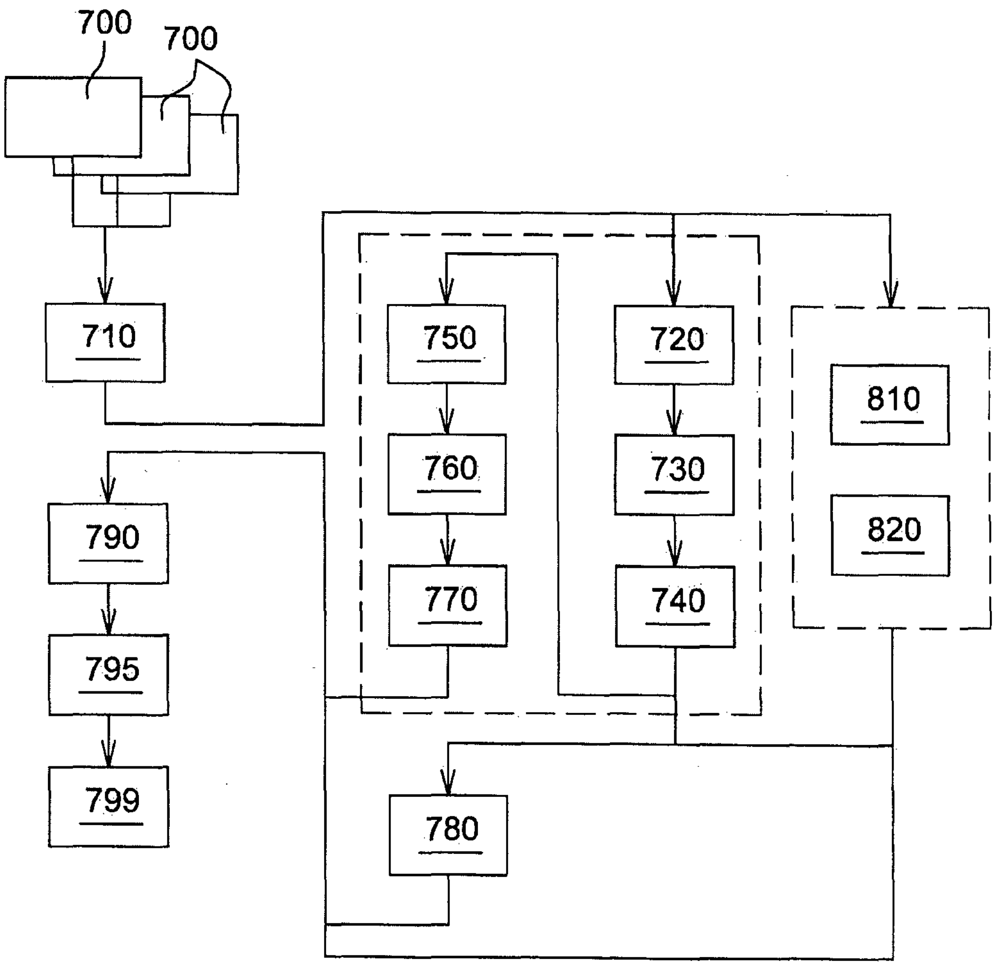
700
700
710
750
760
770
720
730
740
810
820
790
795
799
780

Fig. 7

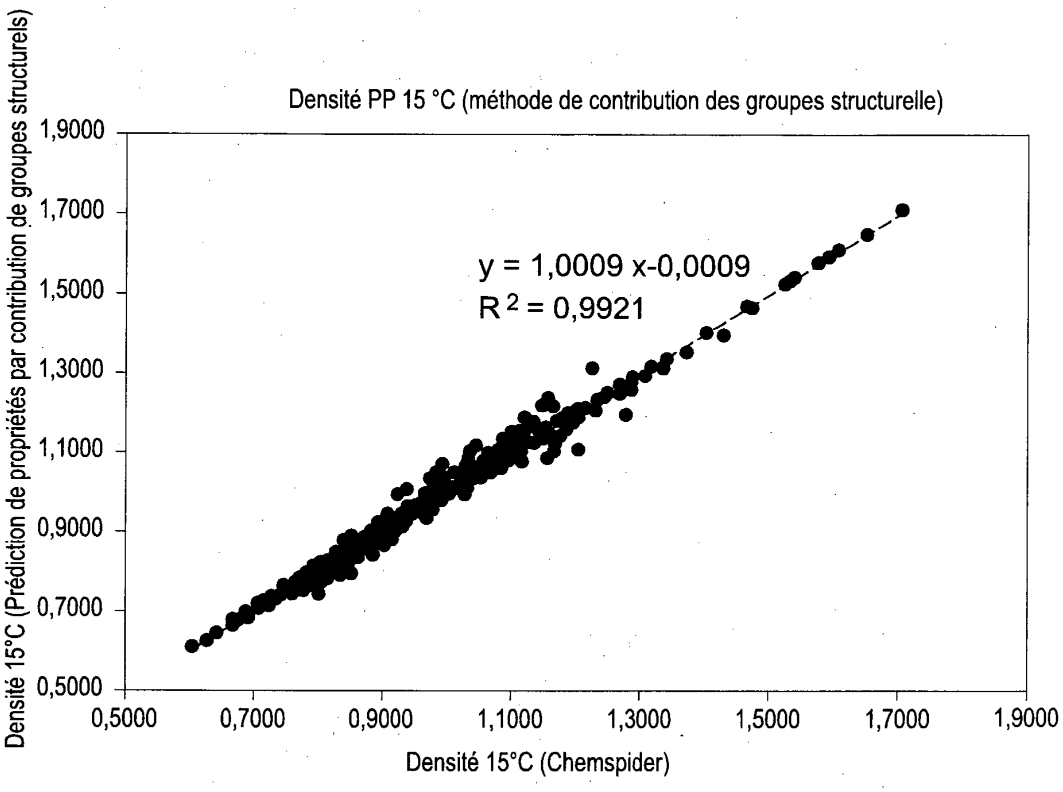
Densité PP 15 °C (méthode de contribution des groupes structurelle)
Densité 15°C (Prédiction de propriétés par contribution de groupes structurels)
1,9000
1,7000
1,5000
1,3000
1,1000
0,9000
0,7000
0,5000
0,5000
0,7000
0,9000
1,1000
1,3000
1,5000
1,7000
1,9000
Densité 15°C (Chemspider)
y = 1,0009 x-0,0009
$R^2$ = 0,9921

**Fig. 8**

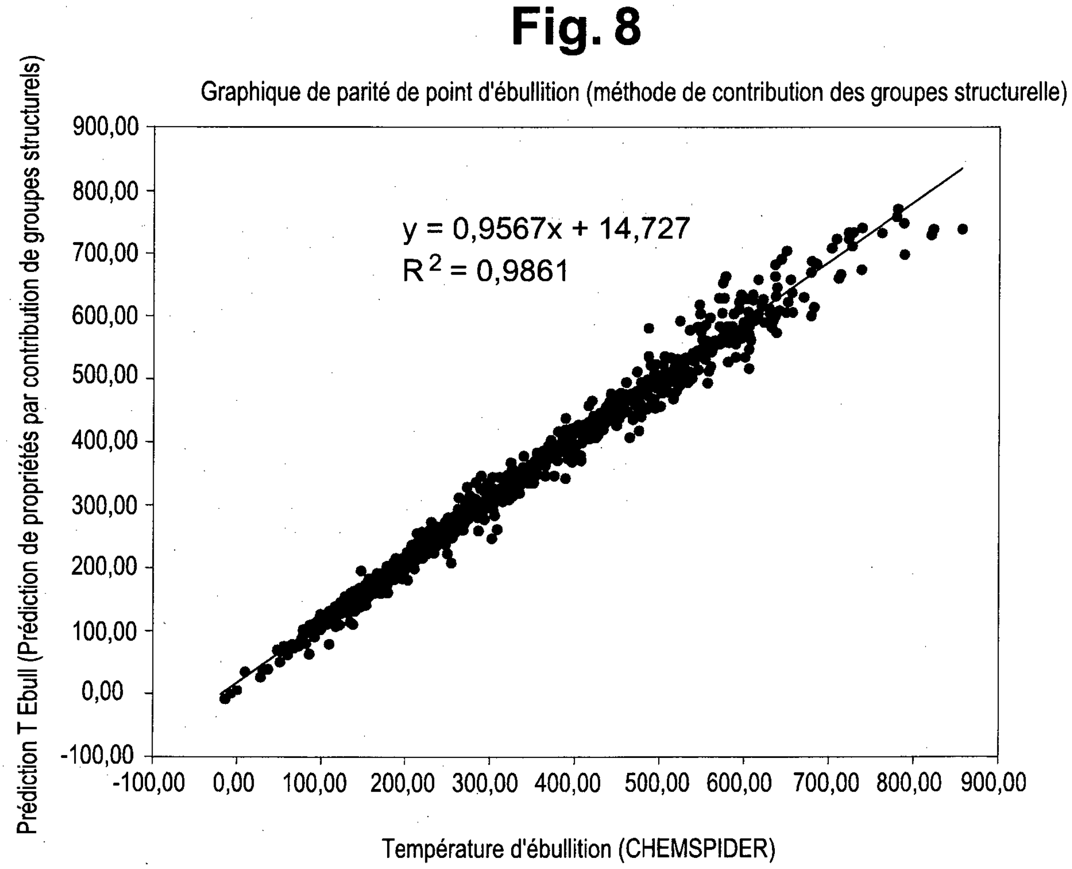
Graphique de parité de point d'ébullition (méthode de contribution des groupes structurelle)
Prédiction T Ebull (Prédiction de propriétés par contribution de groupes structurels)
900,00
800,00
700,00
600,00
500,00
400,00
300,00
200,00
100,00
0,00
-100,00
-100,00
0,00
100,00
200,00
300,00
400,00
500,00
600,00
700,00
800,00
900,00
Température d'ébullition (CHEMSPIDER)
y = 0,9567x + 14,727
$R^2$ = 0,9861

**Fig. 9**

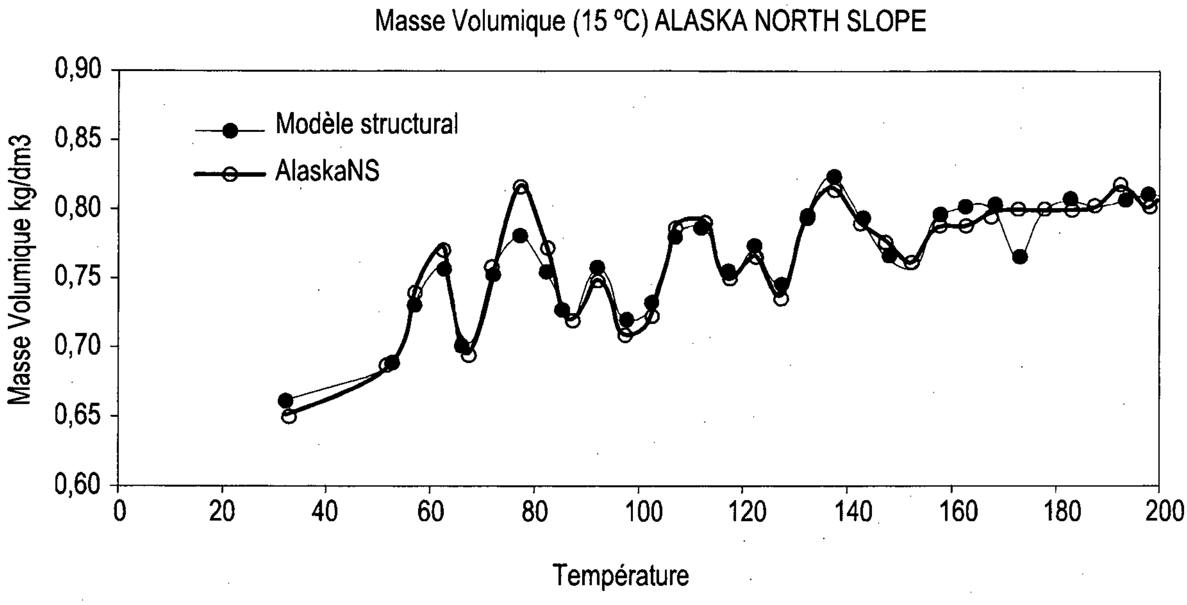
Masse Volumique (15 °C) ALASKA NORTH SLOPE
Modèle structural
AlaskaNS
Masse Volumique kg/dm3
0,90
0,85
0,80
0,75
0,70
0,65
0,60
0
20
40
60
80
100
120
140
160
180
200
Température

**Fig. 10**

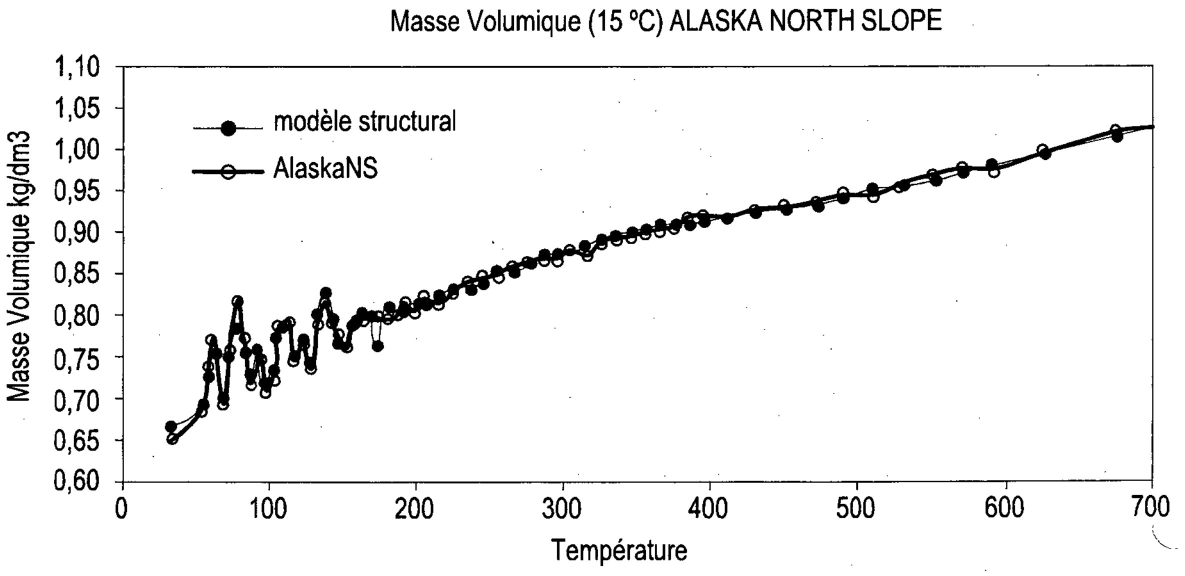
Masse Volumique (15 °C) ALASKA NORTH SLOPE
modèle structural
AlaskaNS
Masse Volumique kg/dm3
1,10
1,05
1,00
0,95
0,90
0,85
0,80
0,75
0,70
0,65
0,60
0
100
200
300
400
500
600
700
Température

**Fig. 11**

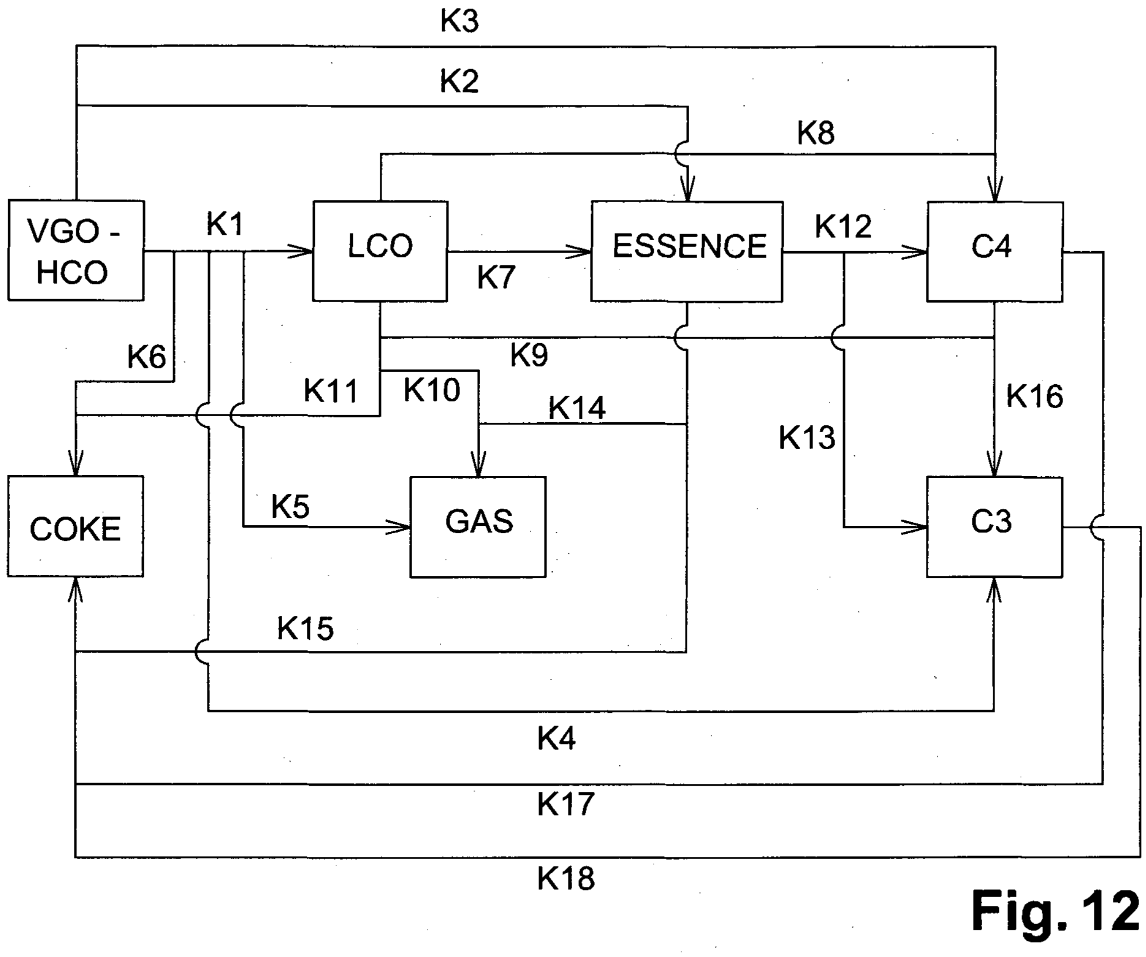
K3
K2
K8
VGO - HCO
K1
LCO
K7
ESSENCE
K12
C4
K6
K9
K11
K10
K14
K16
K13
COKE
K5
GAS
C3
K15
K4
K17
K18

Fig. 12

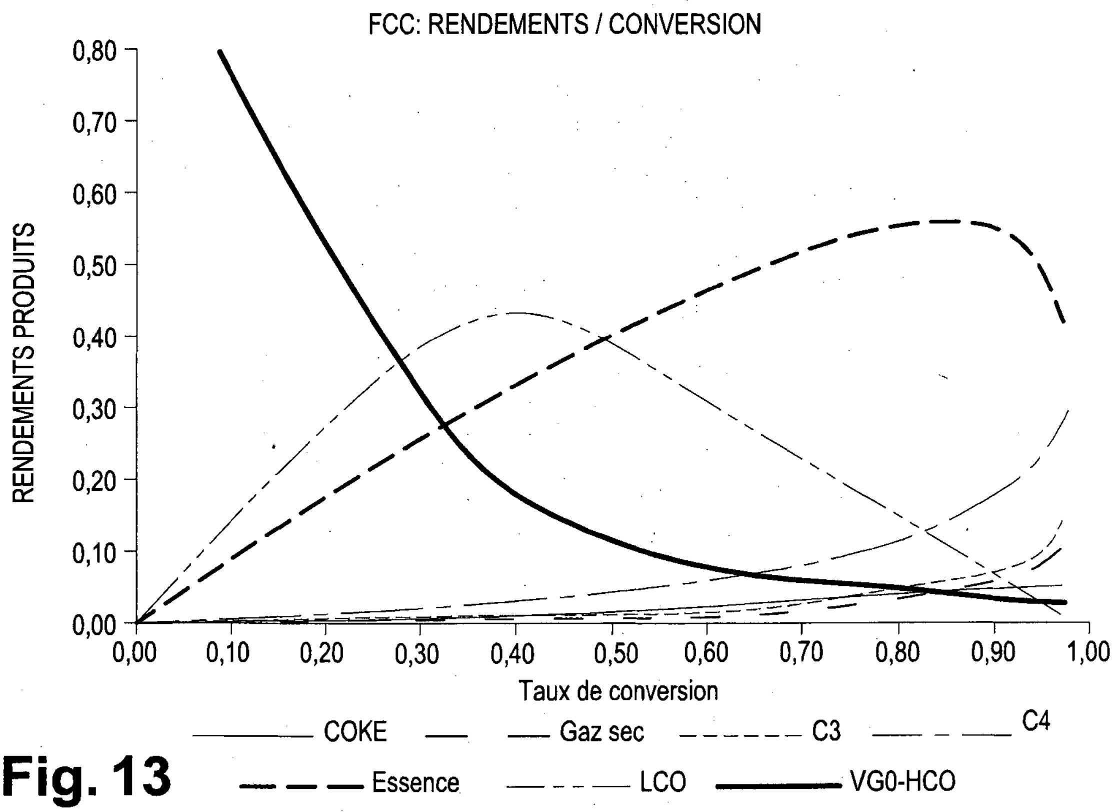
FCC: RENDEMENTS / CONVERSION
RENDEMENTS PRODUITS
0,80
0,70
0,60
0,50
0,40
0,30
0,20
0,10
0,00
0,00
0,10
0,20
0,30
0,40
0,50
0,60
0,70
0,80
0,90
1,00
Taux de conversion
COKE
Gaz sec
C3
C4
Essence
LCO
VG0-HCO

Fig. 13

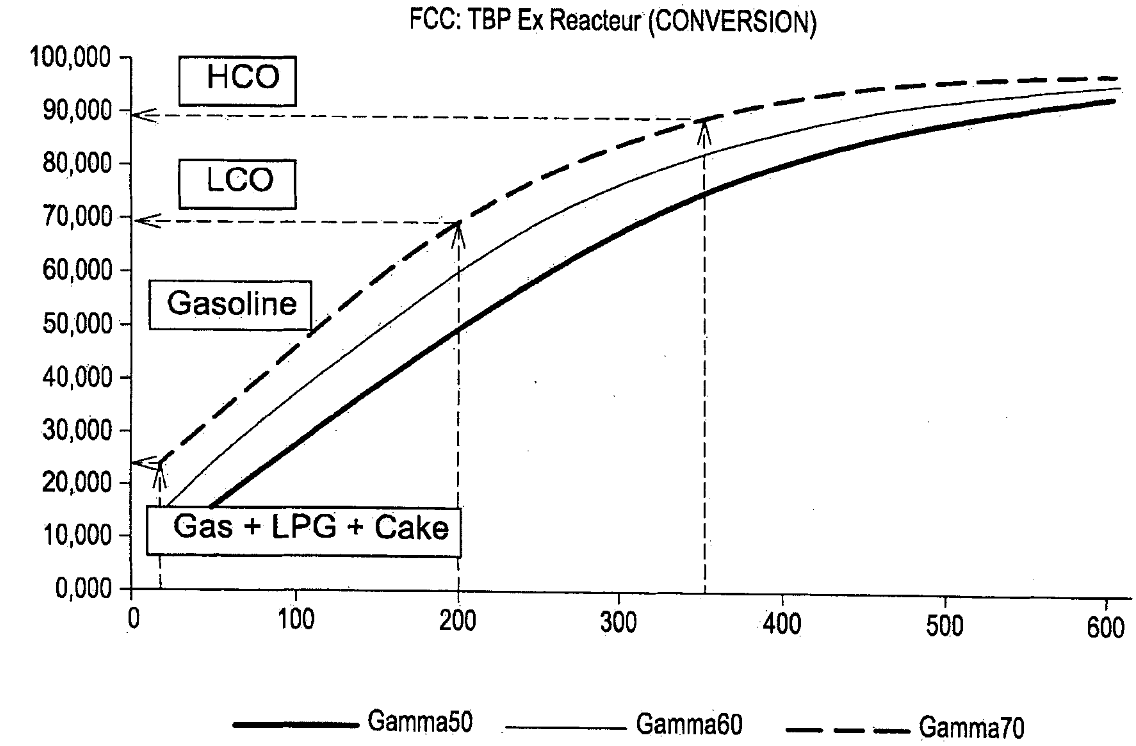
FCC: TBP Ex Reacteur (CONVERSION)
100,000
90,000
80,000
70,000
60,000
50,000
40,000
30,000
20,000
10,000
0,000
0
100
200
300
400
500
600
HCO
LCO
Gasoline
Gas + LPG + Cake
Gamma50
Gamma60
Gamma70

**Fig. 14**

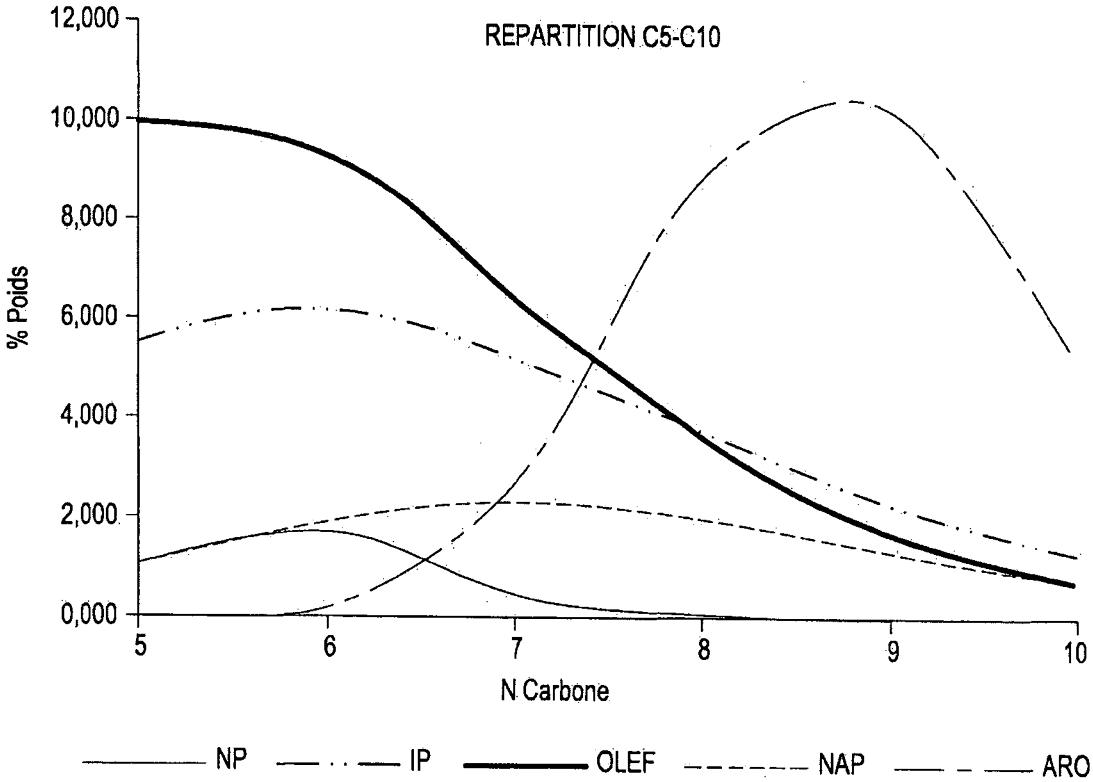
REPARTITION C5-C10
12,000
10,000
8,000
6,000
4,000
2,000
0,000
% Poids
5
6
7
8
9
10
N Carbone
NP
IP
OLEF
NAP
ARO

**Fig. 15**

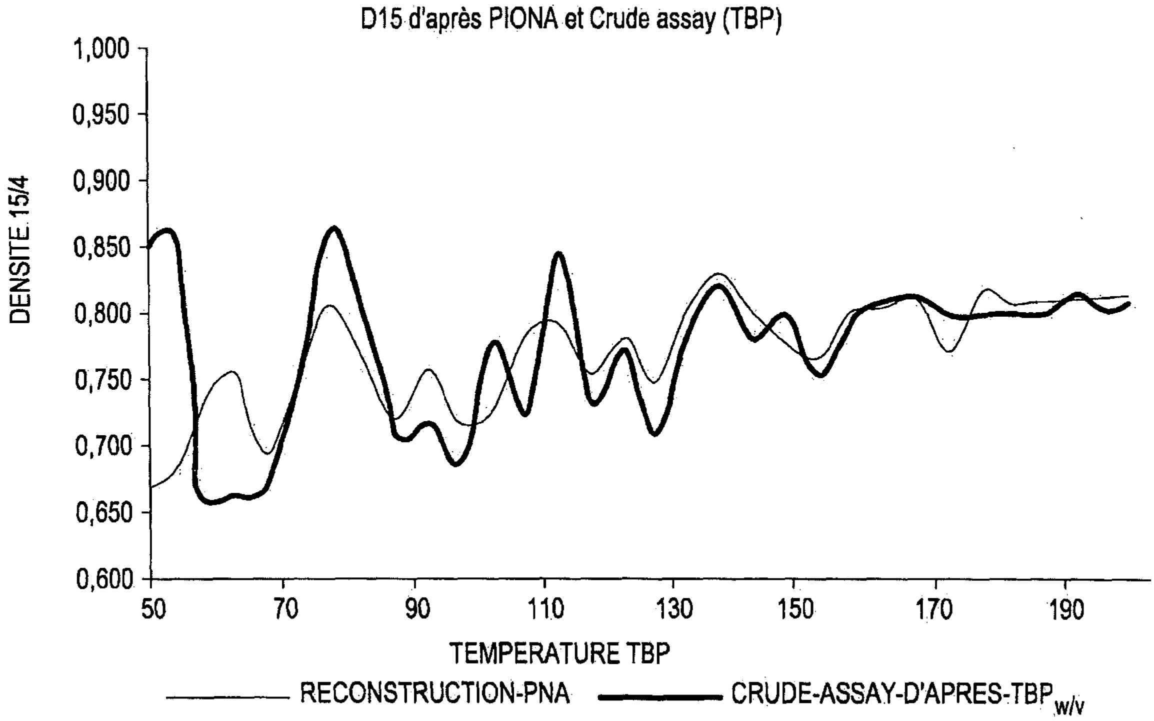
D15 d'après PIONA et Crude assay (TBP)
DENSITE 15/4
1,000
0,950
0,900
0,850
0,800
0,750
0,700
0,650
0,600
50
70
90
110
130
150
170
190
TEMPERATURE TBP
RECONSTRUCTION-PNA
CRUDE-ASSAY-D'APRES-TBP w/v

**Fig. 16**

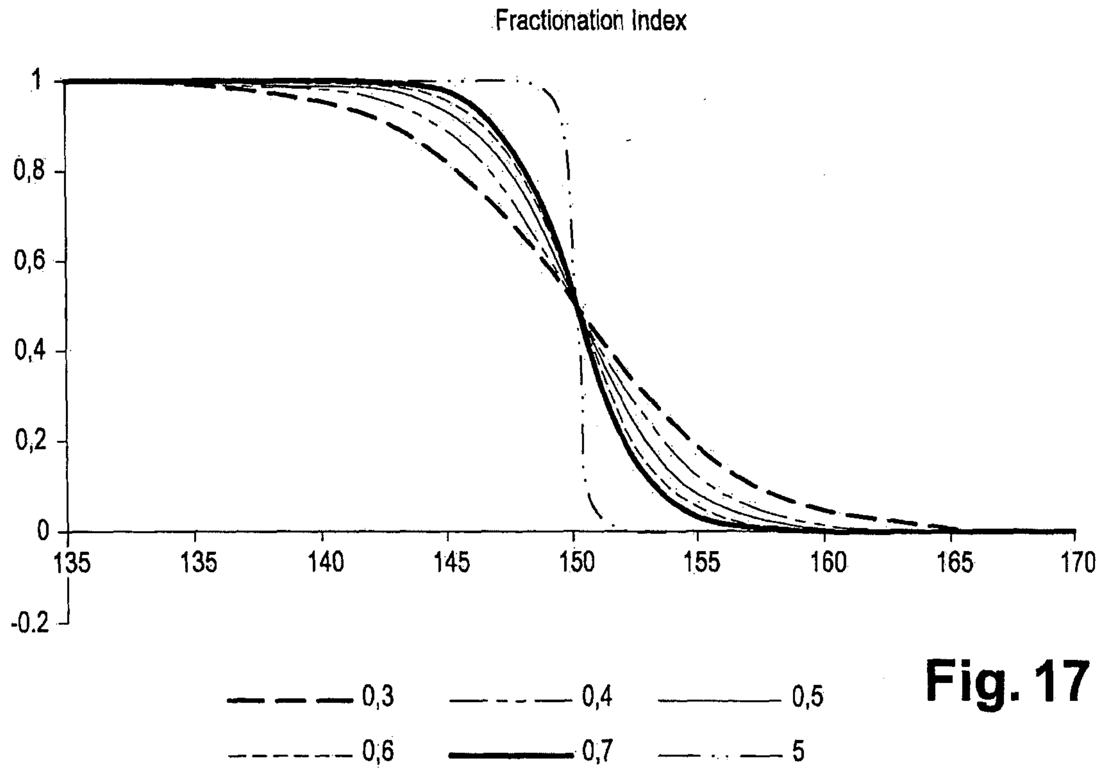
Fractionation Index
1
0,8
0,6
0,4
0,2
0
-0.2
135
135
140
145
150
155
160
165
170
0,3
0,4
0,5
0,6
0,7
5

**Fig. 17**

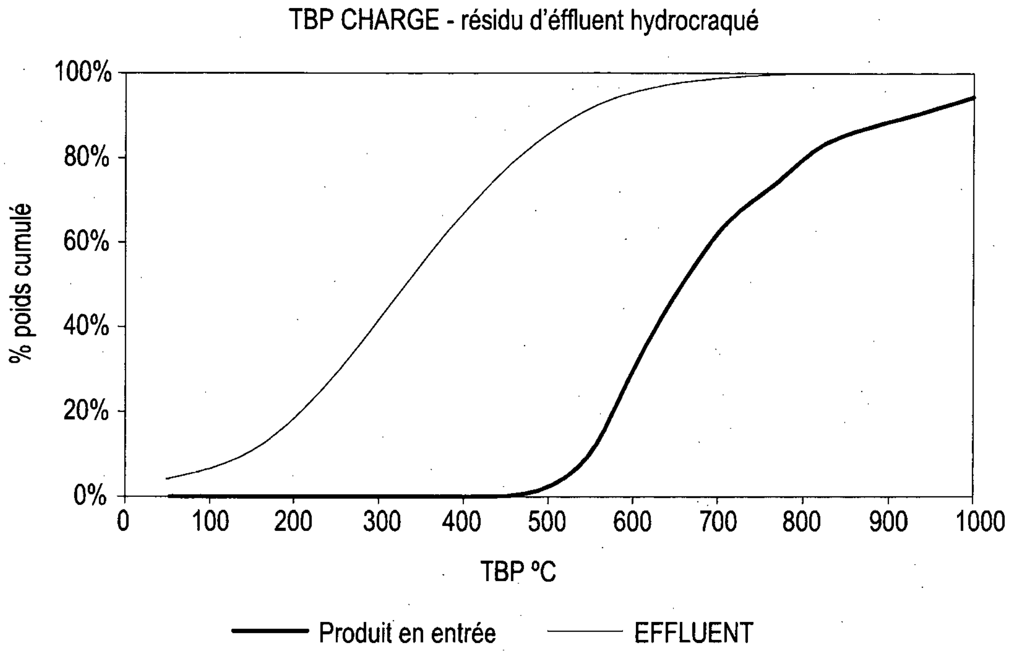
TBP CHARGE - résidu d'éffluent hydrocraqué
% poids cumulé
100%
80%
60%
40%
20%
0%
0
100
200
300
400
500
600
700
800
900
1000
TBP °C
Produit en entrée
EFFLUENT

**Fig. 18**

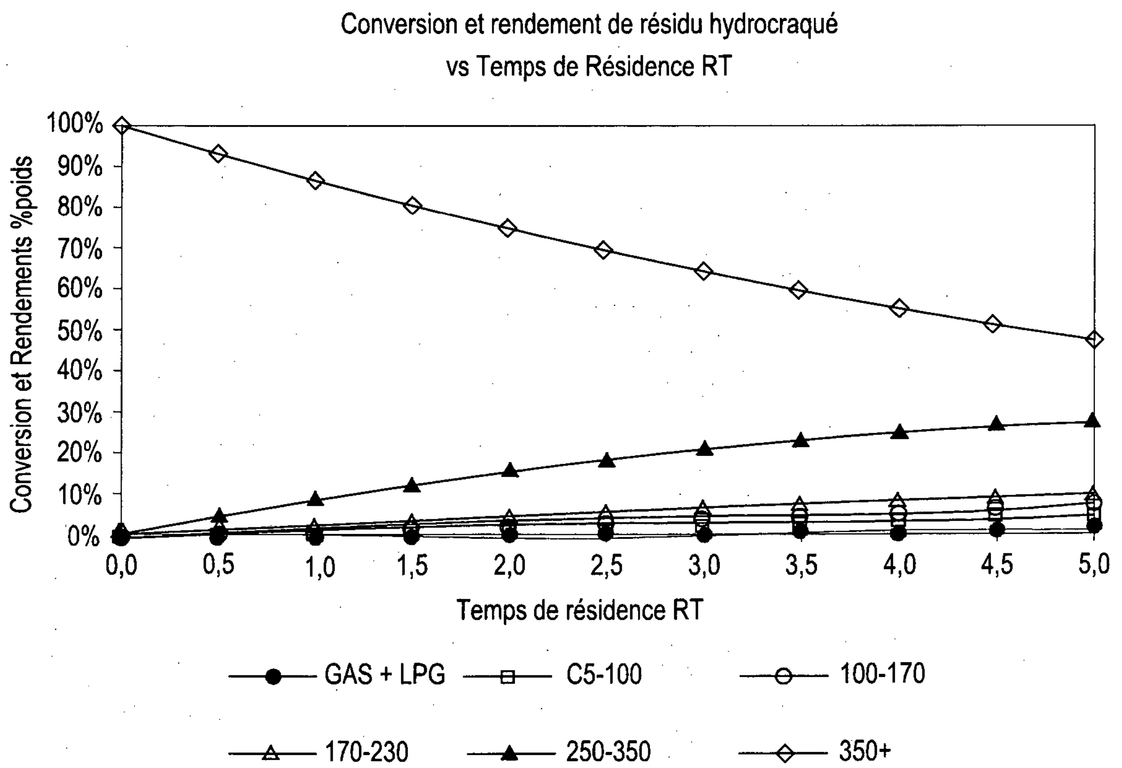
Conversion et rendement de résidu hydrocraqué
vs Temps de Résidence RT
Conversion et Rendements %poids
100%
90%
80%
70%
60%
50%
40%
30%
20%
10%
0%
0,0
0,5
1,0
1,5
2,0
2,5
3,0
3,5
4,0
4,5
5,0
Temps de résidence RT
GAS + LPG
C5-100
100-170
170-230
250-350
350+

**Fig. 19**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20090105966 A


### Littérature non-brevet citée dans la description


- **YOUNGWEN WU ; NAN ZHANG.** Molecular Characterization of Gasoline and Diesel Streams. *Ind. Eng. Chem. Res.,* 2010, vol. 49, 12773-12782